# EUROPEAN PATENT SPECIFICATION

(11) **EP 4 189 087 B1**
(45) Date of publication and mention of the grant of the patent: **15.11.2023**
(21) Application number: 21752015.4
(22) Date of filing: 27.07.2021
(51) Int. Cl.: C12N 15/11, C12Q 1/6816, C12N 15/67

(54) **RNA MOLECULE, USE THEREOF AND A PROCESS FOR DETECTING A DISEASE BY USING THEREOF**
RNA-MOLEKÜL, VERWENDUNG DAVON UND VERFAHREN ZUR DETEKTION EINER KRANKHEIT UNTER VERWENDUNG DAVON
MOLÉCULE D'ARN, SON UTILISATION ET PROCÉDÉ DE DÉTECTION D'UNE MALADIE À L'AIDE DE CELLE-CI

(30) Priority: 27.07.2020 EP 20382674; 22.09.2020 EP 20382834
(43) Date of publication of application: 07.06.2023
(73) Proprietor: Moirai Biodesign, S.L., 08028 Barcelona (ES)
(72) Inventor: DOTU RODRÍGUEZ, Ivan Javier, 08028 Barcelona (ES); MINUESA DINARÈS, Gerard, 08028 Barcelona (ES); ALSINA VERDÚ, Cristina, 08028 Barcelona (ES)
(74) Representative: Ponti & Partners, S.L.P
(86) International application number: PCT/EP2021/071007
(87) International publication number: WO 2022/023343

(56) References cited:
- WO-A1-2018/027177
- CN-A- 111 206 120
- HOANG TRUNG CHAU TIN ET AL: "Developments of Riboswitches and Toehold Switches for Molecular Detection-Biosensing and Molecular Diagnostics", INTERNATIONAL JOURNAL OF MOLECULAR SCIENCES, vol. 21, no. 9, 30 April 2020 (2020-04-30) , page 3192, XP055776867, DOI: 10.3390/ijms21093192

## Description

### Introduction

The research leading to these results has received funding from the European Union's Horizon 2020 research and innovation programme under the Marie Sk 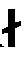 odowska-Curie grant agreement No 712949 (TECNIOspring PLUS) and from the Agency for Business Competitiveness of the Government of Catalonia.

### Field of the invention

The present invention relates to novel RNA molecules comprising a sensor part and a trigger part. Due to these two parts, these molecules are also called herein RNA Plug&Play molecules. The present invention further relates to the use of novel RNA molecules and to a process for detecting a disease in vitro using said novel RNA molecules.

### Background of the invention

Riboregulators are sequences of RNA that effect changes in cells in response to a nucleic acid sequence. These RNA-based devices, which typically regulate protein translation or trigger mRNA degradation, have been used for a number of applications in synthetic biology, including sensitive control over gene expression, shunting of metabolic flux through different metabolic pathways, and synthetic control over cell death. (see WO2014074648).

WO 2018/027177 A1 discloses SNP-sensing riboregulators which consist of two hairpins upstream of the output gene that are both capable of binding trigger RNAs.

In riboregulators that control gene expression, repression of protein translation has relied on sequestration of the normally single- stranded context of the ribosome binding site (RBS) within the loop region of a duplex RNA region (hairpin loop) that is upstream of a gene of interest (GOI). An mRNA in which the RBS is sequestered within a hairpin upstream of the GOI is thus a cis-repressed RNA (crRNA). A riboregulator based on an engineered crRNA can be constructed in which a trans-activating RNA (taRNA) binds to the crRNA and unwinds the repressing RNA duplex thereby exposing a now single-stranded RBS and activating translation of the downstream gene. In riboregulators that decrease expression of the GOI, the RBS and initiation codon of the GOI are both exposed in the absence of the trigger RNA. However, a trans-repressing RNA (trRNA), which bears anti-sense to the RBS and start codon, can bind to the riboregulator and strongly suppress translation of the downstream gene.

Over the last decade, researchers have developed a number of different riboregulator systems to control gene expression in prokaryotic cells. These previous systems have utilized a number of recurring design motifs. The vast majority of riboregulators have employed loop- linear interactions to drive the crRN A/trans -RNA hybridization reaction forward. In these interactions, a linear, single-stranded region in one of the strands binds to a loop established at the end of a duplex in the other strand. Essential in this interaction is the formation of a kissing loop structure in which the tertiary structure of the RNA sequence causes bases within the loop to flip outwards, facilitating binding with the linear RNA strand. Importantly, this kissing loop structure is only established with specific sequences inside the loop region, which can severely limit the number of possible crRNA designs.

All previous riboregulator systems have relied on sequestration of the RBS to impede translation of the GOI. This design choice has two crucial implications. First, much of the work in optimization of genetic circuits in synthetic biology relies on varying the strength of the RBS to finely tune protein levels inside the cell. Since the RBS sequence is a functional part of these riboregulators, one cannot simply replace the riboregulator RBS with variants and expect the output characteristics of the device to vary in a predictable manner following the strength of the new RBS. Furthermore, changes to the RBS will require corresponding modifications in the sequence of the trans-RNA. Second, for riboregulators that activate gene expression, riboregulators that sequester the RBS must be activated by taRNA sequences that are at least partially complementary to the crRNA RBS sequence. Consequently, unbound taRNAs can compete with de-repressed crRNA species for ribosome binding. Alternatively, RBS sequences within the taRNAs can also be sequestered within stem regions. This additional secondary structure can decrease the kinetics of binding with the crRNA and the dynamic range of the riboregulator.

WO2014074648A2 - A riboregulator or toehold switch [P1] is a riboregulator that activates gene expression by switching from a secondary structure that prevents translation to a configuration that promotes translation upon binding of a cognate *trans*-acting RNA. This trans-acting RNA is called the *trigger* and in the diagnostics setting this trigger is endogenous to the cell or organism of interest.

The idea is that the start codon AUG is sequestered within a strong double stranded region, while the Ribosome Binding Site (RBS) is accessible at a loop region of the stem. This configuration allows ribosome binding but it prevents scanning of the gene and therefore translation is compromised. Upon binding the trigger RNA, where regions a* and b* are complementary to regions a and b in the toehold, hybridization of these regions occurs which simultaneously unfolds the double stranded region around the AUG thus allowing translation of the downstream gene.

In the diagnostics setting the trigger RNA is a region of the transcriptome that can only be found in cells with a disease state. In the literature, this trigger RNA is a region of a virus (ebola, zika, etc...) that has infected the host. The gene downstream of the toehold is typically a certain fluorescence protein such as GFP or Luciferase. Therefore, when adding the toehold switch to the infected cell, the fluorescence protein will be translated and this signal will allow for the diagnostic of the disease.

The implementation of this diagnostics protocol has been carried out by using cell lysates of bacteria, which contain all the necessary translation and transcription machinery, where the toehold switch is added along with blood of the patient. If the fluorescence protein can be seen then the patient is infected.

No known approaches have so far arisen to implement this protocol in human cells or to detect other diseases than viral infections.

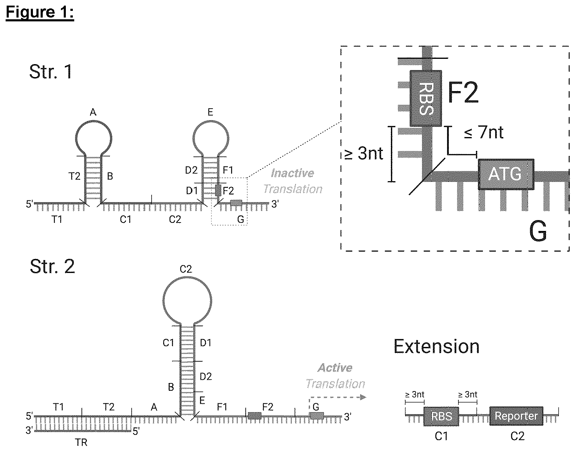

The present invention provides a new approach by developing new RNA molecules named as RNA Plug&Play (RNA P&P), useful for detecting in vitro a disease. The differences between a toehold switch and an RNA Plug&Play are twofold: first, the expression control is achieved via complete RNA duplex obfuscation of the RBS and second, the potential addition of a reporter gene to both report positive and negative biomarker detections. The present inventors have been able to develop such a structure of an RNA P&P that it can surprisingly potentially detect any RNA sequence without sequence constraints. Figure 1 depicts an RNA P&P with all of the regions and specifications.

### Summary of the invention

In a first aspect, the present invention relates to an RNA molecule as defined in the claims.

In a second aspect, the present invention relates to the use of the RNA molecule according to the first aspect of the invention in a colorimetric or fluorometric assay upon ligand binding of said RNA molecule according to the first aspect of the invention.

In a third aspect, the present invention relates to a process for detecting in vitro a disease using the RNA molecule according to the first aspect of the invention.

In a fourth aspect, the present invention relates to a kit for detecting in vitro a disease according to the third aspect of the invention.

### Brief description of the drawings

**Figure 1****. Schematics of an RNA Plug&Play molecule.** An RNA P&P along with all its regions as defined in claim 1, without (upper left region) and with (lower left region) the biomarker (RNA target). Relevant constraints are shown in the upper right region and the extension according to claim 2 is shown in the bottom right region.

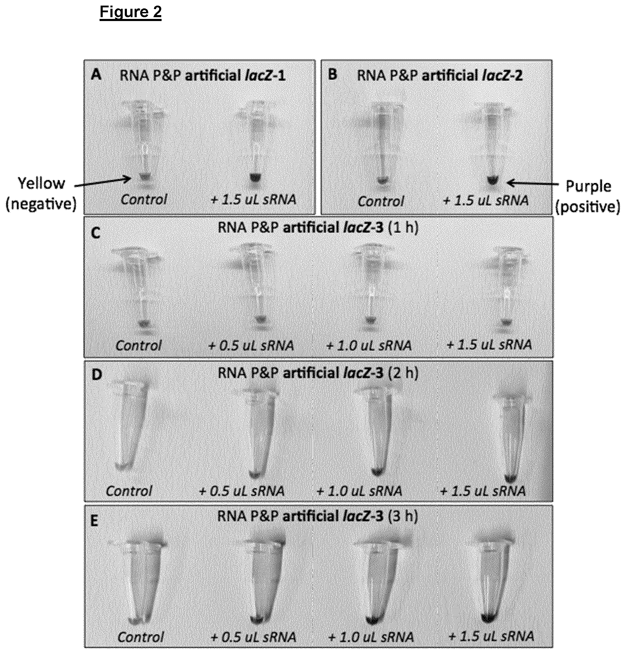

**Figure 2****. RNA P&P detection of synthetic artificial RNA with a colorimetric readout.** Depicted is the reaction of 100 ng RNA P&P artificial *lacZ*-1 and artificial *lacZ-2* in the absence (control) or presence of 2.35 µM synthetic artificial RNA (sRNA, **SEQ ID NO: 1** for RNA P&P artificial *lacZ*-1 and artificial *lacZ*-2) after 45 minutes (panels **A, B);** and 100 ng RNA P&P artificial *lacZ*-3 in the absence (control) or presence of 0.8, 1.6, 2.35 µM sRNA **(SEQ ID NO: 2)** after 1, 2 and 3 hours (panels **C, D, E respectively).** The colorimetric reaction is based on β-galactosidase enzymatic activity (yellow -negative- CPRG is transformed to purple -positive- colored chlorophenol red) only occurring when RNA P&P interacts with *E.coli* 16S rRNA fragment. RNA P&P *lacZ*-1 and *lacZ-2* show a certain level of leakiness so that the negative control shows a more orange color, while *lacZ-3* is very robust and the negative control stays yellow for at least 3 hours.

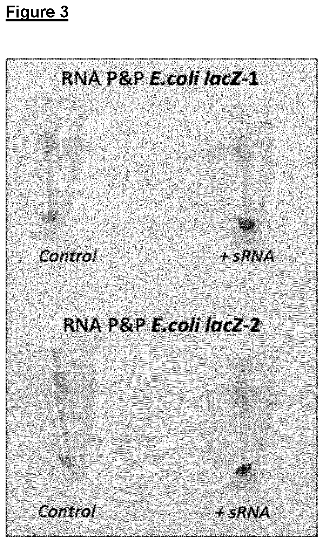

**Figure 3****. RNA P&P detection of *E.coli* 16 rRNA with a colorimetric readout.** Depicted is the reaction of 100 ng RNA P&P *E.coli* lacZ-1 and RNA P&P *E.coli* lacZ-2 in the absence or presence of synthetic RNA of *E.coli* 16S rRNA (sRNA, 1 µL of **SEQ ID NO: 18,** equivalent to 1.6 µM) after 1 hour. The colorimetric reaction is based on β-galactosidase enzymatic activity (yellow -negative- CPRG is transformed to purple -positive- colored chlorophenol red) only occurring when RNA P&P interacts with sRNA.

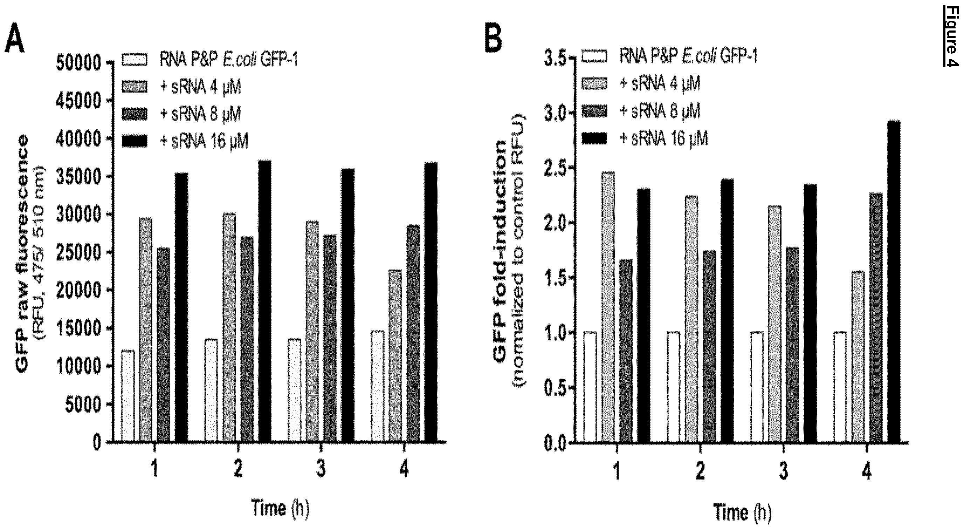

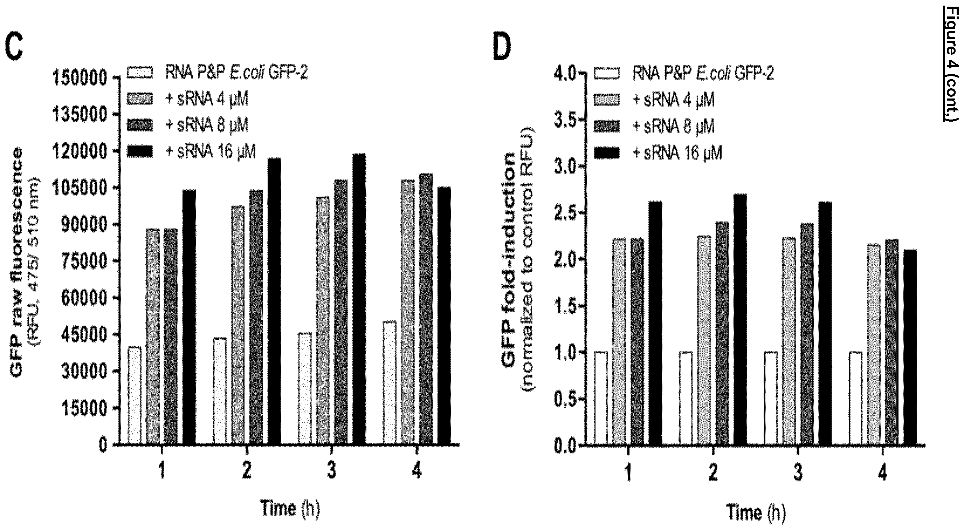

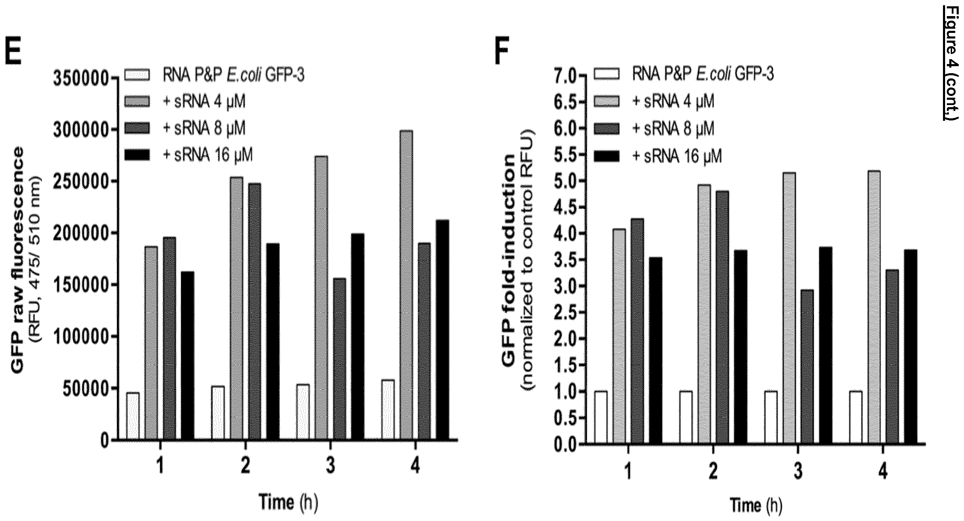

**Figure 4****. RNA P&P detection of E.coli 16S rRNA with *a* fluorescent (GFP) readout.** These graphs show the raw fluorescence GFP signal (RFU = raw fluorescence units) of 100 ng RNA P&P *E.coli* GFP-1 and 50 ng of RNA P&P *E.coli* GFP-2 and RNA P&P *E.coli* GFP-3 **(A, C, E)** and the fold-induction of GFP expression (normalized to RNA P&P *E.coli* GFP 1 and *E.coli* GFP 2 controls) **(B, D, F)** in the absence or presence of *E.coli* 16S rRNA sRNA **(SEQ ID NO: 15)** at 4, 8 and 16 µM) after 1 to 4 hours. Fluorescence signal was read at 475 and 510 nm (excitation and emission wavelengths, respectively) in black bottom low-volume 384-well plates (Corning) on a Synergy H1 Hybrid Multi-Mode Plate Reader.

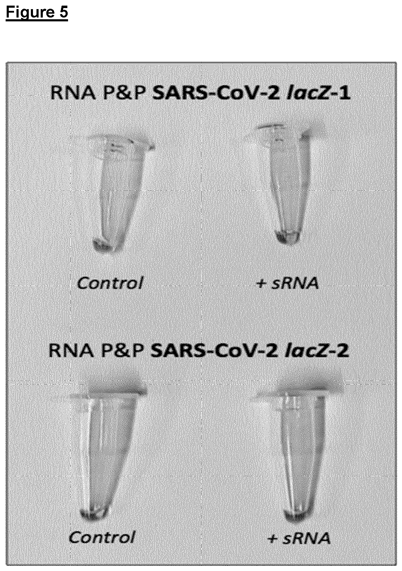

**Figure 5****. RNA P&P detection of SARS-CoV-2 *orf1ab* gene with a colorimetric readout.** Depicted is the reaction of 100 ng SARS-CoV-2 *lacZ-1* and SARS-CoV-2 *lacZ-2* in the absence or presence of sRNA **(SEQ ID NO: 38,** *orf1ab* gene region) (1.5 µL equivalent to 2.35 µM sRNA, respectively) after 2 hours. Final colorimetric reaction is based on β-galactosidase enzymatic activity (yellow -negative- CPRG is transformed to purple -positive-colored chlorophenol red) occurring when RNA P&P interacts with sRNA.

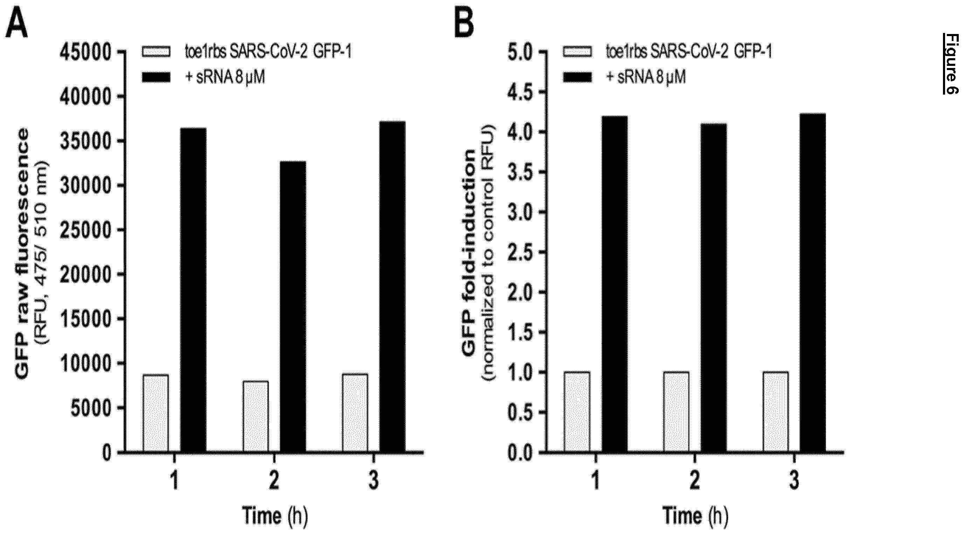

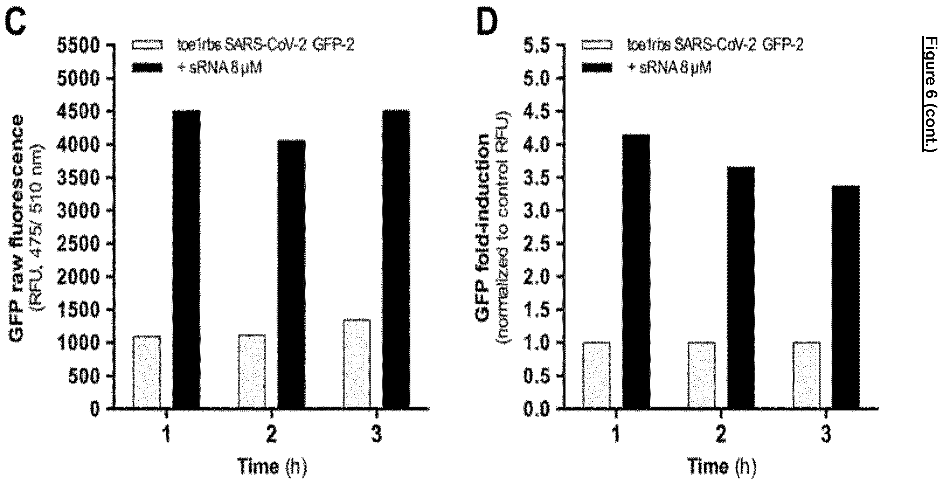

**Figure 6****. RNA P&P detection of SARS-CoV-2 with a fluorescent (GFP) readout.** These graphs show the raw fluorescence GFP signal (RFU = raw fluorescence units) of 100 ng RNA P&P SARS-CoV-2 GFP-1 and RNA P&P SARS-CoV-2 GFP-2 **(A, C)** and the fold-induction of GFP expression (normalized to RNA P&P SARS-CoV-2 GFP-1 and RNA P&P SARS-CoV-2 GFP-2 controls) **(B, D)** in the absence or presence of sRNA (8 µM), **SEQ ID NO: 46** for RNA P&P SARS-CoV-2 GFP-1 and **SEQ ID NO: 47** for RNA P&P SARS-CoV-2 GFP-2) after 1 to 3 hours. Fluorescence signal was read at 475 and 510 nm (excitation and emission wavelengths, respectively) in black bottom low-volume 384-well plates (Corning) on a Synergy H1 Hybrid Multi-Mode Plate Reader.

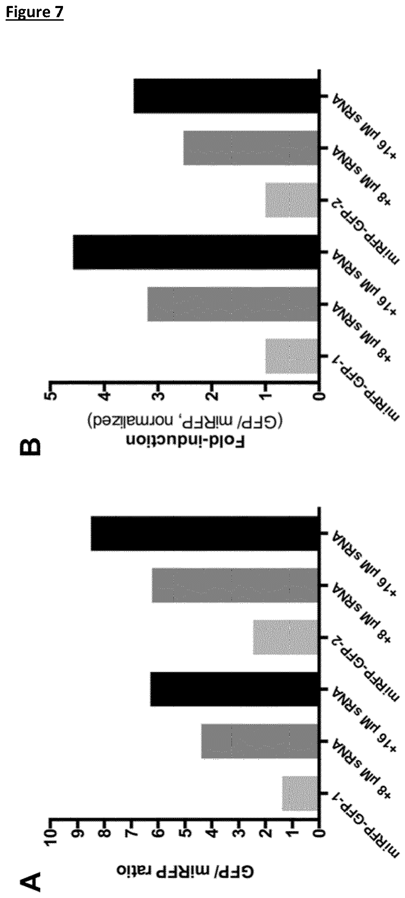

**Figure 7****. RNA P&P detection of SARS-CoV-2 with dual fluorescent (miRFP and GFP) readout.** These graphs show the GFP/ miRFP signal ratio (based on RFU = raw fluorescence units) **(A)** of 150 ng RNA P&P SARS-CoV-2 miRFP/GFP-1 **(SEQ ID NO: 65,** miRFP/GFP-1 in the graph) and RNA P&P SARS-CoV-2 miRFP/GFP-2 **(SEQ ID NO: 66,** miRFP/GFP-2 in the graph) in the absense or the presence of sRNA (8 or 16 µM), **SEQ ID NO: 46)** and **(B)** the fold-induction of GFP of RNA P&P in the presence sRNA versus control (normalizing data on miRFP RFU levels) after 1 hour. Fluorescence signal of miRFP670nano was read at 645 and 670 nm (excitation and emission wavelengths, respectively) and after 30 seconds delay, GFP was read at 475 and 510 nm (excitation and emission wavelengths, respectively) in black bottom low-volume 384-well plates (Corning) on a Synergy H1 Hybrid Multi-Mode Plate Reader.

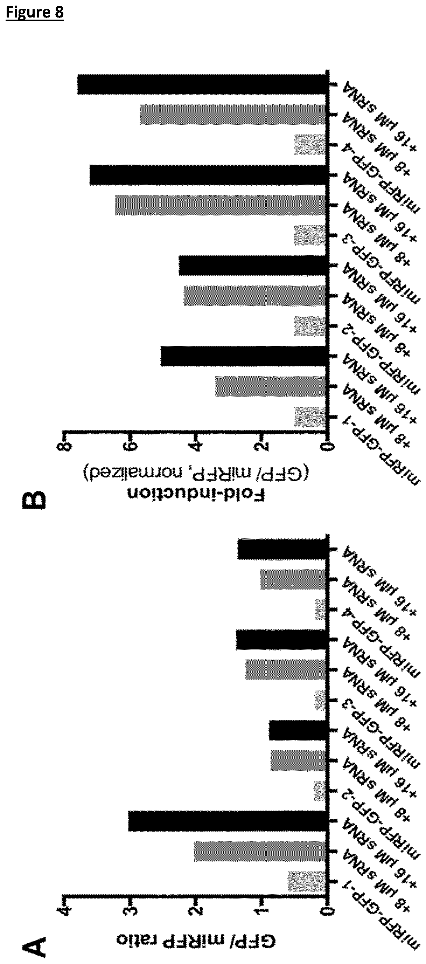

**Figure 8****. RNA P&P detection of *Ecoli* with dual fluorescent (miRFP and GFP) readout.** These graphs show the GFP/ miRFP signal ratio (based on RFU = raw fluorescence units) **(A)** of 150 ng RNA P&P *E.coli* miRFP/GFP-1 **(SEQ ID NO: 83,** miRFP/GFP-1 in the graph), RNA P&P *E.coli* miRFP/GFP-2 **(SEQ ID NO: 84,** miRFP/GFP-2 in the graph), RNA P&P *E.coli* miRFP/GFP-3 **(SEQ ID NO: 85,** miRFP/GFP-3 in the graph) and RNA P&P *E.coli* miRFP/GFP-4 **(SEQ ID NO: 86,** miRFP/GFP-4 in the graph) in the absense or the presence of sRNA (8 or 16 µM), **SEQ ID NO: 18)** and **(B)** the fold-induction of GFP of RNA P&P in the presence sRNA versus control (normalizing data on miRFP RFU levels) after 1 hour. Fluorescence signal of miRFP670nano was read at 645 and 670 nm (excitation and emission wavelengths, respectively) and after 30 seconds delay, GFP was read at 475 and 510 nm (excitation and emission wavelengths, respectively) in black bottom low-volume 384-well plates (Corning) on a Synergy H1 Hybrid Multi-Mode Plate Reader.

### Detailed description

In a fist aspect and main embodiment, the present invention relates to an RNA molecule comprising or consisting of a sensor part and a trigger part, wherein the trigger part is an mRNA coding for a protein and the sensor part comprises or consists of 2 stem loops divided in the following regions in order from 5' to 3': T (T1,T2), A, B, C (C1,C2), D (D1,D2), E, F (F1,F2) and G, wherein
- region T1 is unpaired;
- T2 forms a stem with B where A is the loop region;
- C can be paired or unpaired;
- D forms a stem with F, where E is the loop region;
- F2 must contain an RBS;
- stem loop D1 basepairs with F2 and D2 with F1;
- G contains an AUG (corresponding to the start codon) and a linker and is immediately followed by the trigger part;
- further with the following structural limitations:
   a) T has a length of >= 30 nucleotides;
   b) T has the same number of nucleotides as B;
   c) D1 has the same number of nucleotides as F2 and D2 has the same number of nucleotides as F1:
   d) RBS in F1 is at least 3 nucleotides away from its start;
   e) (B + C1) has the same number of nucleotides as (D + E);
   f) D2 + E has the same number of nucleotides as T;
   g) C1 has the same number of nucleotides as D1;
   h) RBS in F2 must be at least 3 nucleotides away from F2 3' end;

further wherein in the absence of a target RNA, the RBS in region F2 is sequestered in the DEF stem loop and thus, translation of the trigger is not possible;
further wherein in the presence of a target RNA, the sensor part hybridizes the target RNA and a conformational change is produced, in which region T is sequestered by target RNA, B and C1 form a stem loop with D and E, where C2 is the loop region, F and G are partially or totally unpaired and thus the RBS in F2 is not sequestered and translation of the trigger is possible; and wherein RBS do not form stems with regions that also hybridize to the target RNA or form a stem with regions that hybridize with the regions which hybridize the target RNA.

In a preferred embodiment, the trigger part is an mRNA coding for a bioluminescence protein, a fluorescent protein or a colorimetric reporter protein.

In a further embodiment and as a particular case, the present invention relates to an RNA molecule, wherein in the sensor part:
- C contains an RBS in C1 and a sensor reporter gene different from the trigger part,
- RBS in C1 must be at least 3 nucleotides away from C1 each end;
- ATG of the reporter gene can be either on C1 or C2 as long as the distance from the RBS is from 3 to 10, preferably 5-6 nucleotides;
- D+E must be proportional to the length of C2; and

wherein in the absence of RNA target, RBS in C1 is partially or totally unpaired, and
wherein in the presence of RNA target, C1-D1 are part of the stem BC1-DE.

Preferably, in this further embodiment, both the sensor and the trigger reporter genes are mRNAs coding for a bioluminescence protein, a fluorescent protein or a colorimetric reporter protein, as long as both reporter genes are different.

In the context of the present invention, "RBS" is understood as ribosome binding site, which is a sequence of nucleotides upstream of the start codon of an mRNA transcript that is responsible for the recruitment of a ribosome during the initiation of protein translation.

In the context of the present invention, "ATG" is understood as the codon for methionine or the translation initiation codon. Usually, protein translation can only start at a Methionine codon (although this codon may be found elsewhere within the protein sequence as well). In eukaryotic DNA, the sequence is ATG; in RNA it is AUG. Usually, the first AUG in the mRNA is the point at which translation starts, and an open reading frame follows - i.e. the nucleotides taken three at a time will code for the amino acids of the protein, and a stop codon will be found only when the protein coding region is complete.

In the context of the present invention, "stem" and "loop" regions are used as it is well known in the art. Stem-loop intramolecular base pairing is a pattern that can occur in single-stranded DNA or, more commonly, in RNA. The structure is also known as a hairpin or hairpin loop. It occurs when two regions of the same strand, usually complementary in nucleotide sequence when read in opposite directions, base-pair to form a double helix that ends in an unpaired loop. The resulting structure is a key building block of many RNA secondary structures. As an important secondary structure of RNA, it can direct RNA folding, protect structural stability for messenger RNA (mRNA), provide recognition sites for RNA binding proteins, and serve as a substrate for enzymatic reactions.

In the context of the present invention, a "linker" is an artificial RNA sequence that contains no stop codons in frame with the upstream start codon. It serves as a separation between the structurally relevant regions of the RNA P&P and the trigger. It will be translated as part of the trigger so it needs to maintain the frame of the trigger defined by the AUG upstream and it should be short (between 3 and 30 nucleotides preferably) so that it is not expected to jeopardize the activity of the trigger. Any artificial RNA sequence complying with this definition could be used as a linker in the present invention.

In the context of the present invention, "proportional" means that the longer is one region, the longer needs to be the second region, for example, longer C2 regions should imply longer D+E regions, but there is no need for a linear increase in length.

In the context of the present invention, "paired" is understood as for the bases (A, U, G, C for RNA) which stand for the chemicals adenine (A), uracil (U), guanine (G), and cytosine (C), respectively, adenine always pairs with uracil, and guanine always pairs with either cytosine or uracil. When referring to a region, pair or basepair means that all bases in the region are paired. In contrast "unpaired" means that at least one base is not paired.

In this disclosure and in the claims, terms such as "comprises," "comprising," "containing" and "having" are open-ended terms and can mean "includes," "including," and the like; while terms like "consisting of" or "consists of" refer to the mentioned elements after these terms and others which are not mentioned are excluded.

Unless otherwise explained, all technical and scientific terms used herein have the same meaning as commonly understood by one of ordinary skill in the art to which this disclosure belongs. The singular terms "a", "an", and "the" include plural referents unless context clearly indicates otherwise. Similarly, the word "or" is intended to include "and" unless the context clearly indicate otherwise.

In a preferred embodiment for any of the previous embodiments of the RNA molecule of the invention, said bioluminescence protein is selected from luciferase and aequorin, said fluorescent protein is selected from GFP (green fluorescent protein), YFP (yellow fluorescent protein) and RFP (red fluorescent protein) and said colorimetric reporter protein is LacZ (β-galactosidase).

In a preferred embodiment for the RNA molecule according to the main embodiment, there is no sensor reporter gene and the trigger is a LacZ mRNA and the sensor part corresponds to the following artificial RNA sequences selected from: SEQ ID NO: 4, SEQ ID NO: 5 and SEQ ID NO: 6 , where their respective artificial RNA targets are the following: SEQ ID NO: 1 (for SEQ ID NO: 4,5) and SEQ ID NO: 2 (for SEQ ID NO: 6)

In a preferred embodiment for any of the previous embodiments of RNA molecule of the invention, said artificial RNA target is a biomarker characterizing a disease selected from a viral infection, a bacterial infection, cancer or a genetic disease.

Preferably, according to the main embodiment, said biomarker is a region of the e.coli rRNA16s mRNA reverse complemented, the trigger is a LacZ mRNA and wherein the sensor part corresponds to the following artificial RNA sequence: SEQ ID NO: 16 and SEQ ID NO: 17 or alternatively, where the trigger is a GFP mRNA and wherein the sensor part corresponds to the following artificial RNA sequences: SEQ ID NO: 24, SEQ ID NO: 25, SEQ ID NO: 26 .or alternatively, according to the further embodiment and particular case, wherein the sensor reporter gene is RFP and the trigger is a GFP mRNA.

Also preferably, according to the main embodiment said biomarker is a region of the SARS-CoV-2 viral genome (SEQ ID NO: 38), the trigger is a LacZ mRNA and the sensor part corresponds to the following artificial RNA sequences: SEQ ID NO: 36 and SEQ ID NO: 37, or alternatively, said biomarker are regions of the SARS-CoV-2 viral genome reverse complemented (SEQ ID NO: 46, SEQ ID NO: 47), the trigger is a GFP mRNA and wherein the sensor part corresponds to the following artificial RNA sequences: SEQ ID NO: 44 and SEQ ID NO: 45, or alternatively, according to the further embodiment and particular case, said biomarker are regions of the SARS-CoV-2 viral genome reverse complemented, the sensor reporter gene is RFP and the trigger is a GFP mRNA.

In a second aspect, the present invention relates to the use of the RNA molecule according to any of the previous embodiments, in a colorimetric or fluorometric assay (where a transcription/translation assay is performed with the molecule and patient fluid and the result is read with the appropriate instrumentation) upon RNA target binding of said RNA molecule, wherein said RNA molecule is contained with a cell extract in the recipient to be used in an in-vitro diagnostic of a certain disease, such as bacterial infections, viral infections, cancer, genetic disorders, etc... In particular said disease is a bloodstream infection caused by pathogenic bacteria or COVID-19.

In a third aspect, the present invention relates to a process for detecting in vitro a disease, in particular as defined previously, comprising the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule as defined in the main embodiment of the present invention; and
f) identifying a positive result of the disease by confirming the expression of the trigger mRNA.

In the context of the present invention, "body fluids" is understood as blood, saliva, urine, etc...

In the context of the present invention, "isothermal amplification" is understood as and RNA to RNA amplification that requires no cyclic temperature changes, such as NASBA or TMA. For further information about NASBA or TMA, see https://www.neb.com/applications/dna-amplification-pcr-and-qpcr/isothermal-amplification/nucleic-acid-sequenced-based-amplification-and-transcription-mediated-amplification

In another process embodiment, the process for detecting in vitro a disease, in particular as defined previously, comprises the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule as defined in the further embodiment and particular case of the present invention; and
f) identifying a positive result of the disease by confirming the expression of the trigger mRNA.
g) identifying a negative result of the disease by confirming the expression of the sensor mRNA.
h) Null if no expression detected.

In a more preferred embodiment:
1) the isothermal amplification is selected from NASBA, TMA and SMART technologies, and/or
2) the RNA molecule as defined in the present invention is lyophilized, along with a cell extract, in a microfluidics device, and/or
3) the identification of a positive result in step f) is confirmed through a colorimetric or fluorometric assay wherein the trigger is a LacZ mRNA or GFP.
4) the identification of a negative result in step f) is confirmed through a fluorimetric assay wherein the sensor reporter is RFP.

In a further preferred embodiment, the process further comprises between steps a) and c):
b) treating said isolated sample or swab with at least a lysate buffer and/or a heating step.

In a fourth aspect, the present invention relates to a kit for detecting in vitro a disease comprising:
a) at least one receiving means for receiving said sample or swab isolated from the individual;
b) a lysate buffer for treating the sample or swab;
c) heating capacity (a thermoblock for example)
d) at least the RNA molecule as defined in the present invention for the detection of a disease in the individual;
e) instructions for using the kit.

In a more preferred embodiment for the process, the kit, or the use, as previously characterized, said disease is a bloodstream infection caused by pathogenic bacteria or COVID-19.

A number of examples will be provided below that are intended to illustrate the invention and in no way limit the scope of the invention, which is established by the attached claims.

### EXAMPLES

### Example 1: Artificial RNA Plug&Plays (IacZ reporter)

We designed three RNA P&Ps for the detection of two different synthetic artificial RNAs **(SEQ ID NO: 1** and **SEQ ID NO: 2).** These RNA P&Ps are composed of a sensor region and a trigger. The trigger region corresponds to the *lacZ* gene **(SEQ ID NO: 3),** while the sensor regions correspond to RNA P&P artificial /*acZ-*1 **(SEQ ID NO: 4),** RNA P&P artificial *lacZ-2* **(SEQ ID NO: 5)** and RNA P&P artificial *lacZ-*3 **(SEQ ID NO: 6).** All sensor regions are designed to detect a synthetic artificial RNA **(SEQ ID NO: 1** for both RNA P&P artificial *lacZ-1* and *lacZ-2,* and **SEQ ID NO: 2** for RNA P&P artificial *lacZ*-3). In order to generate the complete RNA P&P sequences we performed PCR using YEp358 vector **(SEQ ID NO: 7)** with a 3' *lacZ* terminator primer **(SEQ ID NO: 8)** and 5' primers for P&P artificial /*acZ*-1 **(SEQ ID NO: 9),** for P&P artificial *lacZ*-2 **(SEQ ID NO: 10)** and for artificial *lacZ*-3 **(SEQ ID NO: 11).** The PCR reaction results in the three complete dsDNA products containing a T7 promoter for *in vitro* transcription, the P&P with a ribosome binding site (RBS) and the trigger region *(IacZ* gene) **(SEQ ID NO: 12, 13** and **14,** respectively).

In order to test the efficiency of our RNA P&P, we used **100 ng** of purified PCR product (dsDNA) in a cell-free system that permits mRNA transcription and protein synthesis using recombinant elements (PURExpress, New England Biolabs) in the absence or presence of the corresponding artificial synthetic RNA (sRNA) **(SEQ ID NO: 1** and **SEQ ID NO: 2).** In the absence of the latter, the P&P structure of RNA (blocking access to the RBS) should prevent translation of β-galactosidase (protein product of *lacZ* gene). On the contrary, in the presence of the sRNA **(SEQ ID NO: 1** for both RNA P&P artificial /*acZ*-1 and *lacZ-2,* and **SEQ ID NO: 2** for RNA P&P artificial *lacZ-3*)*,* RNA P&P structure changes conformation upon hybridization and translation of β-galactosidase occurs. **Figure 1** depicts the result of all P&Ps with and without the addition of 2.35 µM of sRNA (1.5 uL of **SEQ ID NO: 1)** after 45 minutes (for RNA P&P artificial /*acZ*-1 and *lacZ-*2) and with and without the addition of 0.8, 1.6 and 2.35 µM of sRNA (0.5, 1.0 and 1.5 uL of **SEQ ID NO: 2,** respectively) for RNA P&P artificial *lacZ*-3 after 1, 2 and 3 hours (for RNA P&P artificial *lacZ-3*) from the start of the reaction.

In this case, using a colorimetric reaction readout, the enzyme expressed cleaves the *yellow* (negative, control) chloro phenol red-β-D-galactopyranoside (CPRG) substrate present in the mix to produce the *purple* (positive, with sRNA) chlorophenol red product.

### Example 2: RNA Plug&Play for E.coli 16S rRNA detection (IacZ reporter)

We designed two RNA P&Ps for the detection of *E.coli* 16S rRNA **(SEQ ID NO: 15).** These RNA P&Ps are composed of a sensor region and a trigger. The trigger region corresponds to the *lacZ* gene **(SEQ ID NO: 3),** while the sensor regions correspond to RNA P&P *E.coli lacZ-1* **(SEQ ID NO: 16)** and *E.coli lacZ-2* **(SEQ ID NO: 17).** Both sensor regions are designed to detect the reverse complement of a region of the 16S rRNA of *E.coli* **(SEQ ID NO: 18).** The region is reverse complemented to simulate the result of a prior step of NASBA amplification (Kao et al. Detection of Escherichia coli Using Nucleic Acid Sequence-Based Amplification and Oligonucleotide Probes for 16S Ribosomal RNA. Analytical Letters, 43, 1756-1769 (2010). In order to generate the complete RNA P&P sequences we performed PCR using YEp358 vector **(SEQ ID NO: 7)** with a 3' *lacZ* terminator primer **(SEQ ID NO: 8)** and 5' primers for RNA P&P *E.coli lacZ-1* **(SEQ ID NO: 19)** and for RNA P&P *E.coli lacZ-*2 **(SEQ ID NO: 20).** The PCR results in the two complete dsDNA products containing a T7 promoter for transcription, the P&P with a ribosome binding site (RBS) and the trigger region *(IacZ* gene) **(SEQ ID NO: 21** and **SEQ ID NO: 22,** respectively).

In order to test the efficiency of our RNA P&P, we used 100 ng of purified PCR product (dsDNA) in a cell-free system that permits mRNA transcription and protein synthesis using recombinant elements (PURExpress, New England Biolabs) in the absence or presence of the synthetic RNA target (sRNA) of *E.coli* 16S RNA **(SEQ ID NO: 18).** In the absence of the latter, the P&P structure of RNA (blocking access of RBS) should prevent translation of β-galactosidase (protein product of *lacZ*)*.* On the contrary, in the presence of the sRNA **(SEQ ID NO: 18),** RNA P&P structure changes conformation upon hybridization and translation of β-galactosidase occurs. **Figure 2** depicts the result of both P&Ps with and without the addition of 1.6 µM of sRNA after 1 hour from the start of the reaction.

In this case, using colorimetric reaction readout, the enzyme expressed cleaves the *yellow* (negative, control) chloro phenol red-β-D-galactopyranoside (CPRG) substrate present in the mix to produce the *purple* (positive, with sRNA) chlorophenol red product.

### Example 3: RNA Plug&Play for Ecoli 16S rRNA detection (GFP reporter)

We designed three RNA P&Ps for the detection of *E.coli* 16S rRNA **(SEQ ID NO: 15).** These RNA P&Ps are composed of a sensor region and a trigger. The trigger region corresponds to the *green fluorescent protein* (*gfp*) *mut3b-ASV* (*gfpmut3b-ASV*) gene **(SEQ ID NO: 23),** while the sensor regions correspond to RNA P&P *E.coli* GFP-1 **(SEQ ID NO: 24),** RNA P&P *E.coli* GFP-2 **(SEQ ID NO: 25)** and RNA P&P *E.coli* GFP-3 **(SEQ ID NO: 26).** Both sensor regions are designed to detect the reverse complement of a region of the 16S rRNA of *E.coli* **(SEQ ID NO: 18).** The region is reverse complemented to simulate the result of a prior step of NASBA amplification (Kao et al. Detection of Escherichia coli Using Nucleic Acid Sequence-Based Amplification and Oligonucleotide Probes for 16S Ribosomal RNA. Analytical Letters, 43, 1756-1769 (2010)). In order to generate the complete RNA P&P sequences we performed PCR using a *gfpmut3b-ASV* containing synthetic construct **(SEQ ID NO: 27)** with a 3' GFP terminator primer **(SEQ ID NO: 28)** and 5' primers for RNA P&P *E.coli* GFP-1 **(SEQ ID NO: 29),** for RNA P&P *E.coli* GFP-2 **(SEQ ID NO: 30)** and for RNA P&P *E.coli* GFP-3 **(SEQ ID NO: 31).** The PCR results in the complete dsDNA products containing a T7 promoter for transcription, the P&P with a ribosome binding site (RBS) and the trigger region (*gfpmut3b-ASV* gene) **(SEQ ID NO: 32, 33** and **34** for P&P *E.coli* GFP-1, -2 and -3, respectively).

In order to test the efficiency of our RNA P&P, we used 100 ng (RNA P&P *E.coli* GFP-1) and 50 ng (RNA P&P *E.coli* GFP-2 and RNA P&P *E.coli* GFP-3) of purified PCR product (dsDNA) in a cell-free system that permits mRNA transcription and protein synthesis using recombinant elements (PURExpress, New England Biolabs) in the absence or presence of the sRNA of *E.coli* 16S rRNA **(SEQ ID NO: 15).** In the absence of the latter, the P&P structure of RNA (blocking access to the RBS) should prevent translation of GFPmut3b-ASV protein. On the contrary, in the presence of the sRNA **(SEQ ID NO: 15),** RNA P&P structure changes conformation upon hybridization and translation of β-galactosidase occurs. **Figure 3** shows the result (in terms of both raw fluorescence and fold change) of both RNA P&Ps with and without the addition of 4, 8 and 16 µM of sRNA **(SEQ ID NO: 15)** after 1 to 4 hours from the start of the reaction.

In this case, the fluorescent readout of the translated GFPmut3b-ASV was used considering a ratio of 2.0 (P&P+sRNA over control) as the threshold for an effective detection. When expressed, GFPmut3b-ASV fluoresces with an excitation and emission wavelengths of 475 and 510 nm, respectively. It is an unstable GFP form with a half life of 110 min, what reduces the background (autofluorescence) to minimum levels, particularly when working in the cellular environment (Chapel et al. Computational design of small transcription activating RNAs for versatile and dynamic gene regulation. Nature Communications, 8, 1051 (2017).

### Example 4: RNA Plug&Play for Severe Acute Respiratory Syndrome Coronavirus-2 (SARS-CoV-2) detection (IacZ reporter)

We designed two RNA P&Ps for the detection of SARS-CoV-2 *orf1ab* gene **(SEQ ID NO: 35).** These RNA P&Ps are composed of a sensor region and a trigger. The trigger region corresponds to the *lacZ gene* **(SEQ ID NO: 3),** while the sensor regions correspond to RNA P&P SARS-CoV-2 *lacZ*-1 **(SEQ ID NO: 36)** and RNA P&P SARS-CoV-2 *lacZ-2* **(SEQ ID NO: 37).** Both sensor regions are designed to detect a specific region of the *orf1ab* gene of SARS-CoV-2 **(SEQ ID NO: 38).** In order to generate the complete RNA P&P sequences we performed PCR using YEp358 vector **(SEQ ID NO: 7)** with a 3' *lacZ* terminator primer **(SEQ ID NO: 8)** and 5' primers for SARS-CoV-2 *lacZ*-1 **(SEQ ID NO: 39)** and for SARS-CoV-2 *lacZ-2* **(SEQ ID NO: 40).** The PCR results in the complete dsDNA products containing a T7 promoter for transcription, the P&P with a ribosome binding site (RBS) and the trigger region *(IacZ* gene) **(SEQ ID NO: 41** and **42** for SARS-CoV-2 *lacZ*-1 and *lacZ-2,* respectively)

In order to test the efficiency of our RNA P&P, we used 100 ng of purified PCR product (dsDNA) in a recombinant protein transcription/ translation system that permits mRNA transcription and protein synthesis using recombinant elements (PURExpress, New England Biolabs) in the absence or presence of the SARS-CoV-2 sRNA of the *orf1ab* gene region of interest **(SEQ ID NO: 38).** In the absence of the latter, the P&P structure of RNA (blocking access to the RBS) should prevent translation of β-galactosidase (product of *lacZ*)*.* On the contrary, in the presence of the sRNA **(SEQ ID NO: 38),** RNA P&P structure changes conformation upon binding and translation of β-galactosidase occurs. **Figure 4** depicts the result of both P&Ps with and without the addition of 2.35 µM of RNA target after 2 hours from the start of the reaction.

In this case, using colorimetric reaction readout, the enzyme expressed cleaves the *yellow* (negative, control) chloro phenol red-β-D-galactopyranoside (CPRG) substrate present in the mix to produce the *purple* (positive, with sRNA) chlorophenol red product.

### Example 5: RNA Plug&Play for SARS-CoV-2 detection (GFP reporter)

We designed two RNA P&Ps for the detection of SARS-CoV-2 *orf1ab* gene **(SEQ ID NO: 35)** and *orf8* gene **(SEQ ID NO: 43).** These RNA P&Ps are composed of a sensor region and a trigger. The trigger region corresponds to the *green fluorescent protein (gfp) mut3b-ASV (gfpmut3b-ASV)* gene **(SEQ ID NO: 23),** while the sensor regions correspond to RNA P&P SARS-CoV-2 GFP-1 **(SEQ ID NO: 44)** and RNA P&P SARS-CoV-2 GFP-2 **(SEQ ID NO: 45).** Both sensor regions are designed to detect the reverse complement of a region of the SARS-CoV-2 *orf1ab* gene **(SEQ ID NO: 46** for RNA P&P SARS-CoV-2 GFP-1) and *orf8* gene **(SEQ ID NO: 47** for RNA P&P SARS-CoV-2 GFP-2). The region is reverse complemented to simulate the result of a prior step of NASBA amplification. In order to generate the complete RNA P&P sequences we performed PCR using a *gfpmut3b-ASV* containing synthetic construct **(SEQ ID NO: 27)** with a 3' GFP terminator primer **(SEQ ID NO: 28)** and 5' primers for RNA P&P SARS-CoV-2 GFP-1 **(SEQ ID NO: 48)** and for RNA P&P SARS-CoV-2 GFP-2 **(SEQ ID NO: 49).** The PCR results in the complete dsDNA products containing a T7 promoter for transcription, the P&P with a ribosome binding site (RBS) and the trigger region *(gfpmut3b-ASV* gene) **(SEQ ID NO: 50** and **51** for P&P SARS-CoV-2 GFP-1 and GFP-2, respectively).

In order to test the efficiency of our RNA P&P, we used 100 ng of purified PCR product (dsDNA) in a cell-free system that permits mRNA transcription and protein synthesis using recombinant elements (PURExpress, New England Biolabs) in the absence or presence of the SARS-CoV-2 sRNA **(SEQ ID NO: 46** for RNA P&P SARS-CoV-2 GFP-1 and **SEQ ID NO: 47** for RNA P&P SARS-CoV-2 GFP-2). In the absence of the sRNA, the P&P structure of RNA (blocking access to the RBS) should prevent translation of GFPmut3b-ASV protein. On the contrary, in the presence of the sRNA **(SEQ ID NO: 46** and **SEQ ID NO: 47),** RNA P&P structure changes conformation upon hybridization and translation of GFPmut3b-ASV occurs. ++**Figure 5** shows the result of both RNA P&Ps (in terms of both raw fluorescence and fold change) with and without the addition of 8 µM of sRNA after **1 to 3 hours** from the start of the reaction.

In this case, the fluorescent readout of the translated GFPmut3b-ASV was used considering a ratio of 2.0 (P&P+sRNA over control) as positive. When expressed, GFPmut3b-ASV fluoresces with an excitation and emission wavelengths of 475 and 510 nm, respectively. It is an unstable GFP form with a half life of ~110 min, what reduces the background (autofluorescence) to minimum levels, specially when working in the cellular environment (Chapell et al. Computational design of small transcription activating RNAs for versatile and dynamic gene regulation. Nature Communications, 8, 1051 (2017)).

### Example 6: RNA Plug&Play for SARS-CoV-2 detection (miRFP/GFP reporter)

We designed two RNA P&Ps RFP/GFP for the detection of SARS-CoV-2 *orf1ab* gene **(SEQ ID NO: 35).** These RNA P&Ps miRFP/GFP are composed of a sensor region, a sensor reporter gene and a trigger region (claim 2). The sensor reporter corresponds to the *miRFP670nano* gene **(SEQ ID NO: 52),** while the trigger corresponds to *gfpmut3b-ASV* gene **(SEQ ID NO: 23).** The sensor regions correspond to RNA P&P SARS-CoV-2 RFP/GFP-1 and -2 **(SEQ ID NO: 53** and **SEQ ID NO: 54),** which include the sensor reporter gene. These sensor regions are designed to detect the reverse complement of a region of the *orf1ab* gene **(SEQ ID NO: 46).**

In order to generate the complete RNA P&P sequences we performed two initial PCR reactions (PCR#1 and PCR#2) and an Overlap Extension PCR (OE-PCR). The first PCR (PCR#1) used the dsDNA *miRFP670nano* synthetic gene **(SEQ ID NO: 52)** as template, a 5' primer #1 containing a T7 for RNA P&P SARS-CoV-2 miRFP/GFP-1 and -2 **(SEQ ID NO: 55** and **SEQ ID NO: 56)** and a common 3' primer (with 32 overlapping nucleotides with PCR#2 5' region) **(SEQ ID NO: 57)** resulting in the PCR product #1 for RNA P&P SARS-CoV-2 miRFP/GFP-1 and -2 **(SEQ ID NO: 58** and **SEQ ID NO: 59,** respectively). The second PCR (PCR#2) reaction used the the *gfpmut3b-ASV* synthetic gene **(SEQ ID NO: 27)** as template, a common 5' primer #2 for RNA P&P SARS-CoV-2 RFP/GFP-1 and -2 **(SEQ ID NO: 60)** (containing the end of *miRFP670nano* gene, RBS and upstream region of GFP start codon) and a common 3' GFP terminator primer **(SEQ ID NO: 28)** resulting in the PCR product #2 for RNA P&P SARS-CoV-2 miRFP/GFP-1 and -2 **(SEQ ID NO: 61** and **SEQ ID NO: 62,** respectively). With the two PCR products for each P&P miRFP/GFP, we performed an OE-PCR by running 15 cycles (without primers addition) to anneal the 32-nt overlapping region (marked in grey in **SEQ ID Nos: 57 to 62)** and then adding primers 5' T7 **(SEQ ID NO: 63)** and 3' GFP term **(SEQ ID NO: 64)** to perform 30 cycles to finish the reaction. The products of this OE-PCR were purified and constituted the final constructs to be assayed **(SEQ ID NO: 65** and **66).**

In order to test the efficiency of our RNA P&P, we used 150 ng of the final PCR products **(SEQ ID NO: 65** and **66)** in a cell-free system that permits mRNA transcription and protein synthesis using recombinant elements (PURExpress, New England Biolabs) in the absence or presence of the SARS-CoV-2 *orf1ab* gene sRNA **(SEQ ID NO: 46).** In the absence of the sRNA, the RNA P&P structure of the sensor allows the translation of miRFP670nano protein while, at the same time, prevents the translation of GFPmut3b-ASV protein. On the contrary, in the presence of the sRNA and upon hybridization, RNA P&P structure of the sensor changes conformation and translation of GFPmut3b-ASV occurs whereas miRFP670nano translation is blocked. **Figure 7** shows the result of both RNA P&Ps with and without the addition of 8 and 16 µM of the SARS-CoV-2 sRNA **(SEQ ID NO: 46)** after 1 hour from the start of the reaction.

In this case, the fluorescent readout of the translated GFPmut3b-ASV is read at an excitation and emission wavelengths of 475 and 510 nm, respectively. miRFP670nano fluorescence in the presence of biliverdin is read at an excitation and emission wavelengths of 645 and 675 nm, respectively. Ratios of GFP/miRFP > 2.5 (or fold-induction over 2.0) indicate a positive outcome (presence of sRNA) and ratios of GFP/miRFP ≤ 2.5 (or fold-induction below 2.0) indicate a negative outcome (sRNA is not present or null/poor hybridization).

### Example 7: RNA Plug&Play for Ecoli 16S rRNA detection (miRFP/GFP reporter)

We designed two RNA P&Ps miRFP/GFP for the detection of *E.coli* 16S rRNA **(SEQ ID NO: 15).** These RNA P&Ps miRFP/GFP are composed of a sensor region, a sensor reporter gene and a trigger region (claim 2). The sensor reporter corresponds to the *miRFP670nano* gene **(SEQ ID NO: 52),** while the trigger corresponds to *gfpmut3b-ASV*gene **(SEQ ID NO: 23).** The sensor regions correspond to RNA P&P *E.coli* miRFP/GFP-1, -2, -3 and -4 **(SEQ ID NOs: 67 to 70),** which include the sensor reporter gene. These sensor regions are designed to detect the reverse complement of a region of the 16S rRNA of *E.coli* **(SEQ ID NO: 18).** The region is reverse complemented to simulate the result of a prior step of NASBA amplification (Kao et al. Anal Letters, 2010).

In order to generate the complete RNA P&P sequences we performed two initial PCR reactions (PCR#1 and PCR#2) and an Overlap Extension PCR (OE-PCR). The first PCR (PCR#1) used the dsDNA *miRFP670nano* synthetic gene **(SEQ ID NO: 52)** as template, a 5' primer #1 containing a T7 for RNA P&P *E.coli* miRFP/GFP-1, -2, -3 and -4 **(SEQ ID Nos: 71 to 74)** and a common 3' primer (with 32 overlapping nucleotides with PCR#2 5' region) **(SEQ ID NO: 57)** resulting in the PCR product #1 for RNA P&P *E.coli* miRFP/GFP-1, -2, -3 and -4 **(SEQ ID Nos: 75 to 78).** The second PCR (PCR#2) reaction used the the *gfpmut3b-ASV* synthetic gene **(SEQ ID NO: 27)** as template, a 5' primer #2 **(SEQ ID NO: 79** for RNA P&P *E.coli* miRFP/GFP-1 and **SEQ ID NO: 80** for *E.coli* miRFP/GFP-2, -3 and -4) containing the end of *miRFP670nano* gene, RBS and upstream region of GFP start codon and a common 3' GFP terminator primer **(SEQ ID NO: 28)** resulting in the PCR products #2 for RNA P&P *E.coli* miRFP/GFP-1 **(SEQ ID NO: 81)** and RNA P&P *E.coli* miRFP/GFP-2, -3 and -4 **(SEQ ID NO: 82).** With the two PCR products for each P&P miRFP/GFP, we performed an OE-PCR by running 15 cycles (without primers addition) to anneal the 32-nt overlapping region (marked in grey in **SEQ ID NOs: 57, 75 to 82)** and then adding primers 5' T7 **(SEQ ID NO: 63)** and 3' GFP term **(SEQ ID NO: 64)** to perform 30 cycles to finish the reaction. The products of this OE-PCR were purified and constituted the final constructs to be assayed **(SEQ ID NOs: 83 to 86).**

In order to test the efficiency of our RNA P&P, we used 150 ng of the final PCR products **(SEQ ID NOs: 83 to 86)** in a cell-free system that permits mRNA transcription and protein synthesis using recombinant elements (PURExpress, New England Biolabs) in the absence or presence of the 16S rRNA *E.coli* sRNA **(SEQ ID NO: 18).** In the absence of the sRNA, the RNA P&P structure of the sensor allows the translation of miRFP670nano protein while, at the same time, prevents the translation of GFPmut3b-ASV protein. On the contrary, in the presence of the sRNA and upon hybridization, RNA P&P structure of the sensor changes conformation and translation of GFPmut3b-ASV occurs whereas miRFP670nano translation is blocked. **Figure 8** shows the result of both RNA P&Ps with and without the addition of 8 and 16 µM of the *E.coli 16S* sRNA **(SEQ ID NO: 18)** after 1 hour from the start of the reaction.

In this case, the fluorescent readout of the translated GFPmut3b-ASV is read at an excitation and emission wavelengths of 475 and 510 nm, respectively. miRFP670nano fluorescence in the presence of 10 µM biliverdin (chromophore) is read at an excitation and emission wavelengths of 645 and 675 nm, respectively. Ratios of GFP/miRFP > 1.0 indicate a positive outcome (presence of sRNA) and ratios of GFP/miRFP ≤ 1.0, indicate a negative outcome (sRNA is not present or null/poor hybridization).

The list of sequences mentioned herein is as follows:
Sequence #1:
   > **Synthetic RNA target artificial-1/2**
   GUCCAACAGCGCCAGAGAACAACUAUGAUG
Sequence #2:
   > **Synthetic RNA target artificial-3**
   CGCCGCUGGCCCGGGUUGGAUAAUAUUUAG
Sequence #3
   *>* ***lacZ* gene** from NC 000913.3:c366305-363231 *Escherichia coli* str. K-12 substr. *MG1655,* complete genome 
ATGCACGGTTACGATGCGCCCATCTACACCAACGTGACCTATCCCATTACGGTCAAT
CCGCCGTTTGTTCCCACGGAGAATCCGACGGGTTGTTACTCGCTCACATTTAATGTT
GATGAAAGCTGGCTACAGGAAGGCCAGACGCGAATTATTTTTGATGGCGTTAACTCG
GCGTTTCATCTGTGGTGCAACGGGCGCTGGGTCGGTTACGGCCAGGACAGTCGTTT
GCCGTCTGAATTTGACCTGAGCGCATTTTTACGCGCCGGAGAAAACCGCCTCGCGG
 
TGATGGTGCTGCGCTGGAGTGACGGCAGTTATCTGGAAGATCAGGATATGTGGCGG
ATGAGCGGCATTTTCCGTGACGTCTCGTTGCTGCATAAACCGACTACACAAATCAGC
GATTTCCATGTTGCCACTCGCTTTAATGATGATTTCAGCCGCGCTGTACTGGAGGCT
GAAGTTCAGATGTGCGGCGAGTTGCGTGACTACCTACGGGTAACAGTTTCTTTATGG
CAGGGTGAAACGCAGGTCGCCAGCGGCACCGCGCCTTTCGGCGGTGAAATTATCGA
TGAGCGTGGTGGTTATGCCGATCGCGTCACACTACGTCTGAACGTCGAAAACCCGA
AACTGTGGAGCGCCGAAATCCCGAATCTCTATCGTGCGGTGGTTGAACTGCACACC
GCCGACGGCACGCTGATTGAAGCAGAAGCCTGCGATGTCGGTTTCCGCGAGGTGC
GGATTGAAAATGGTCTGCTGCTGCTGAACGGCAAGCCGTTGCTGATTCGAGGCGTTA
ACCGTCACGAGCATCATCCTCTGCATGGTCAGGTCATGGATGAGCAGACGATGGTG
CAGGATATCCTGCTGATGAAGCAGAACAACTTTAACGCCGTGCGCTGTTCGCATTAT
CCGAACCATCCGCTGTGGTACACGCTGTGCGACCGCTACGGCCTGTATGTGGTGGA
TGAAGCCAATATTGAAACCCACGGCATGGTGCCAATGAATCGTCTGACCGATGATCC
GCGCTGGCTACCGGCGATGAGCGAACGCGTAACGCGAATGGTGCAGCGCGATCGT
AATCACCCGAGTGTGATCATCTGGTCGCTGGGGAATGAATCAGGCCACGGCGCTAA
TCACGACGCGCTGTATCGCTGGATCAAATCTGTCGATCCTTCCCGCCCGGTGCAGTA
TGAAGGCGGCGGAGCCGACACCACGGCCACCGATATTATTTGCCCGATGTACGCGC
GCGTGGATGAAGACCAGCCCTTCCCGGCTGTGCCGAAATGGTCCATCAAAAAATGG
CTTTCGCTACCTGGAGAGACGCGCCCGCTGATCCTTTGCGAATACGCCCACGCGAT
GGGTAACAGTCTTGGCGGTTTCGCTAAATACTGGCAGGCGTTTCGTCAGTATCCCCG
TTTACAGGGCGGCTTCGTCTGGGACTGGGTGGATCAGTCGCTGATTAAATATGATGA
AAACGGCAACCCGTGGTCGGCTTACGGCGGTGATTTTGGCGATACGCCGAACGATC
GCCAGTTCTGTATGAACGGTCTGGTCTTTGCCGACCGCACGCCGCATCCAGCGCTG
ACGGAAGCAAAACACCAGCAGCAGTTTTTCCAGTTCCGTTTATCCGGGCAAACCATC
GAAGTGACCAGCGAATACCTGTTCCGTCATAGCGATAACGAGCTCCTGCACTGGATG
GTGGCGCTGGATGGTAAGCCGCTGGCAAGCGGTGAAGTGCCTCTGGATGTCGCTCC
ACAAGGTAAACAGTTGATTGAACTGCCTGAACTACCGCAGCCGGAGAGCGCCGGGC
AACTCTGGCTCACAGTACGCGTAGTGCAACCGAACGCGACCGCATGGTCAGAAGCC
GGGCACATCAGCGCCTGGCAGCAGTGGCGTCTGGCGGAAAACCTCAGTGTGACGC
TCCCCGCCGCGTCCCACGCCATCCCGCATCTGACCACCAGCGAAATGGATTTTTGC
ATCGAGCTGGGTAATAAGCGTTGGCAATTTAACCGCCAGTCAGGCTTTCTTTCACAG
ATGTGGATTGGCGATAAAAAACAACTGCTGACGCCGCTGCGCGATCAGTTCACCCGT
GCACCGCTGGATAACGACATTGGCGTAAGTGAAGCGACCCGCATTGACCCTAACGC
CTGGGTCGAACGCTGGAAGGCGGCGGGCCATTACCAGGCCGAAGCAGCGTTGTTG
CAGTGCACGGCAGATACACTTGCTGATGCGGTGCTGATTACGACCGCTCACGCGTG
GCAGCATCAGGGGAAAACCTTATTTATCAGCCGGAAAACCTACCGGATTGATGGTAG
TGGTCAAATGGCGATTACCGTTGATGTTGAAGTGGCGAGCGATACACCGCATCCGG
 
CGCGGATTGGCCTGAACTGCCAGCTGGCGCAGGTAGCAGAGCGGGTAAACTGGCT
CGGATTAGGGCCGCAAGAAAACTATCCCGACCGCCTTACTGCCGCCTGTTTTGACC
GCTGGGATCTGCCATTGTCAGACATGTATACCCCGTACGTCTTCCCGAGCGAAAACG
GTCTGCGCTGCGGGACGCGCGAATTGAATTATGGCCCACACCAGTGGCGCGGCGA
CTTCCAGTTCAACATCAGCCGCTACAGTCAACAGCAACTGATGGAAACCAGCCATCG
CCATCTGCTGCACGCGGAAGAAGGCACATGGCTGAATATCGACGGTTTCCATATGG
GGATTGGTGGCGACGACTCCTGGAGCCCGTCAGTATCGGCGGAATTCCAGCTGAGC
GCCGGTCGCTACCATTACCAGTTGGTCTGGTGTCAAAAATAA
Sequence #4
   > **RNA P&P artificial lacZ-1** 
GGCCAUCAUAGUUGUUCUCUGGCGCUGUUGGACGAAAACAGUCCAGCAGCGCCG
GGAAGGAGGGGCGAGGCAUAAGUUCCUCCUUCUUGGCGUUGUUGGACCUAAGAA
GGAGGAAUUUAUGUCAUAUCUCACACAACGGGAGGUCGUUUUACAACGUCGU
Sequence #5
   > **RNA P&P artificial lacZ-2** 
GGCCAUCAUAGUUGUUCUCUGGCGCUGUUGGACGAAAACAGUCCAGCAGCGCCG
GGAAGGAGGGGCGAGAGAUAAGUUCCUCCUUCUUGGCGUUGUUGGACCUAAGAA
GGAGGAAUUUAUGUCAUAUCUCACACAACGGGAGGUCGUUUUACAACGUCGU
Sequence #6
   > **RNA P&P artificial lacZ-3** 
GGCCUAAAUAUUAUCCAACCCGGGCCAGCGGCGGAUAAAAUGUCGUUGGUCCGG
GAGAGGGAGGCGUGAAAAAAUUGUCUUUCUUUUUCGGAUUAAUGAUACGAAAAAG
GAGGAUAUCAUGUACUACAGCUUCGAUCCCGCCGUCGUUUUACAACGUCGU
Sequence #7
   > **YEp358 vector** *http:llgenome-www.stanford.edu*/*vectordb*/*vector descrip*/*COMPLETE*/*YEP358.SEQ.html* 
GAATTCGAGCTCGGTACCCGGGGATCCTCTAGAGTCGACCTGCAGGCATGCAAGCT
TCGATCCCGCCGTCGTTTTACAACGTCGTGACTGGGAAAACCCTGGCGTTACCCAAC
TTAATCGCCTTGCAGCACATCCCCCTTTCGCCAGCTGGCGTAATAGCGAAGAGGCCC
GCACCGATCGCCCTTCCCAACAGTTGCGCAGCCTGAATGGCGAATGGCGCTTTGCC
 
TGGTTTCCGGCACCAGAAGCGGTGCCGGAAAGCTGGCTGGAGTGCGATCTTCCTGA
GGCCGATACTGTCGTCGTCCCCTCAAACTGGCAGATGCACGGTTACGATGCGCCCA
TCTACACCAACGTAACCTATCCCATTACGGTCAATCCGCCGTTTGTTCCCACGGAGA
ATCCGACGGGTTGTTACTCGCTCACATTTAATGTTGATGAAAGCTGGCTACAGGAAG
GCCAGACGCGAATTATTTTTGATGGCGTTAACTCGGCGTTTCATCTGTGGTGCAACG
GGCGCTGGGTCGGTTACGGCCAGGACAGTCGTTTGCCGTCTGAATTTGACCTGAGC
GCATTTTTACGCGCCGGAGAAAACCGCCTCGCGGTGATGGTGCTGCGTTGGAGTGA
CGGCAGTTATCTGGAAGATCAGGATATGTGGCGGATGAGCGGCATTTTCCGTGACG
TCTCGTTGCTGCATAAACCGACTACACAAATCAGCGATTTCCATGTTGCCACTCGCTT
TAATGATGATTTCAGCCGCGCTGTACTGGAGGCTGAAGTTCAGATGTGCGGCGAGTT
GCGTGACTACCTACGGGTAACAGTTTCTTTATGGCAGGGTGAAACGCAGGTCGCCA
GCGGCACCGCGCCTTTCGGCGGTGAAATTATCGATGAGCGTGGTGGTTATGCCGAT
CGCGTCACACTACGTCTGAACGTCGAAAACCCGAAACTGTGGAGCGCCGAAATCCC
GAATCTCTATCGTGCGGTGGTTGAACTGCACACCGCCGACGGCACGCTGATTGAAG
CAGAAGCCTGCGATGTCGGTTTCCGCGAGGTGCGGATTGAAAATGGTCTGCTGCTG
CTGAACGGCAAGCCGTTGCTGATTCGAGGCGTTAACCGTCACGAGCATCATCCTCTG
CATGGTCAGGTCATGGATGAGCAGACGATGGTGCAGGATATCCTGCTGATGAAGCA
GAACAACTTTAACGCCGTGCGCTGTTCGCATTATCCGAACCATCCGCTGTGGTACAC
GCTGTGCGACCGCTACGGCCTGTATGTGGTGGATGAAGCCAATATTGAAACCCACG
GCATGGTGCCAATGAATCGTCTGACCGATGATCCGCGCTGGCTACCGGCGATGAGC
GAACGCGTAACGCGAATGGTGCAGCGCGATCGTAATCACCCGAGTGTGATCATCTG
GTCGCTGGGGAATGAATCAGGCCACGGCGCTAATCACGACGCGCTGTATCGCTGGA
TCAAATCTGTCGATCCTTCCCGCCCGGTGCAGTATGAAGGCGGCGGAGCCGACACC
ACGGCCACCGATATTATTTGCCCGATGTACGCGCGCGTGGATGAAGACCAGCCCTT
CCCGGCTGTGCCGAAATGGTCCATCAAAAAATGGCTTTCGCTACCTGGAGAGACGC
GCCCGCTGATCCTTTGCGAATACGCCCACGCGATGGGTAACAGTCTTGGCGGTTTC
GCTAAATACTGGCAGGCGTTTCGTCAGTATCCCCGTTTACAGGGCGGCTTCGTCTGG
GACTGGGTGGATCAGTCGCTGATTAAATATGATGAAAACGGCAACCCGTGGTCGGCT
TACGGCGGTGATTTTGGCGATACGCCGAACGATCGCCAGTTCTGTATGAACGGTCTG
GTCTTTGCCGACCGCACGCCGCATCCAGCGCTGACGGAAGCAAAACACCAGCAGCA
GTTTTTCCAGTTCCGTTTATCCGGGCAAACCATCGAAGTGACCAGCGAATACCTGTT
CCGTCATAGCGATAACGAGCTCCTGCACTGGATGGTGGCGCTGGATGGTAAGCCGC
TGGCAAGCGGTGAAGTGCCTCTGGATGTCGCTCCACAAGGTAAACAGTTGATTGAAC
TGCCTGAACTACCGCAGCCGGAGAGCGCCGGGCAACTCTGGCTCACAGTACGCGTA
GTGCAACCGAACGCGACCGCATGGTCAGAAGCCGGGCACATCAGCGCCTGGCAGC
AGTGGCGTCTGGCGGAAAACCTCAGTGTGACGCTCCCCGCCGCGTCCCACGCCATC
CCGCATCTGACCACCAGCGAAATGGATTTTTGCATCGAGCTGGGTAATAAGCGTTGG
 
CAATTTAACCGCCAGTCAGGCTTTCTTTCACAGATGTGGATTGGCGATAAAAAACAAC
TGCTGACGCCGCTGCGCGATCAGTTCACCCGTGCACCGCTGGATAACGACATTGGC
GTAAGTGAAGCGACCCGCATTGACCCTAACGCCTGGGTCGAACGCTGGAAGGCGGC
GGGCCATTACCAGGCCGAAGCAGCGTTGTTGCAGTGCACGGCAGATACACTTGCTG
ATGCGGTGCTGATTACGACCGCTCACGCGTGGCAGCATCAGGGGAAAACCTTATTTA
TCAGCCGGAAAACCTACCGGATTGATGGTAGTGGTCAAATGGCGATTACCGTTGATG
TTGAAGTGGCGAGCGATACACCGCATCCGGCGCGGATTGGCCTGAACTGCCAGCTG
GCGCAGGTAGCAGAGCGGGTAAACTGGCTCGGATTAGGGCCGCAAGAAAACTATCC
CGACCGCCTTACTGCCGCCTGTTTTGACCGCTGGGATCTGCCATTGTCAGACATGTA
TACCCCGTACGTCTTCCCGAGCGAAAACGGTCTGCGCTGCGGGACGCGCGAATTGA
ATTATGGCCCACACCAGTGGCGCGGCGACTTCCAGTTCAACATCAGCCGCTACAGT
CAACAGCAACTGATGGAAACCAGCCATCGCCATCTGCTGCACGCGGAAGAAGGCAC
ATGGCTGAATATCGACGGTTTCCATATGGGGATTGGTGGCGACGACTCCTGGAGCC
CGTCAGTATCGGCGGAATTACAGCTGAGCGCCGGTCGCTACCATTACCAGTTGGTCT
GGTGTCAAAAATAATAATAACCGGGCAGGCCATGTCTGCCCGTATTTCGCGTAAGGA
AATCCATTATGTACTATTTCGCCTGATGCGGTATTTTCTCCTTACGCATCTGTGCGGT
ATTTCACACCGCATAGGGTAATAACTGATATAATTAAATTGAAGCTCTAATTTGTGAGT
TTAGTATACATGCATTTACTTATAATACAGTTTTTTAGTTTTGCTGGCCGCATCTTCTC
AAATATGCTTCCCAGCCTGCTTTTCTGTAACGTTCACCCTCTACCTTAGCATCCCTTC
CCTTTGCAAATAGTCCTCTTCCAACAATAATAATGTCAGATCCTGTAGAGACCACATC
ATCCACGGTTCTATACTGTTGACCCAATGCGTCTCCCTTGTCATCTAAACCCACACCG
GGTGTCATAATCAACCAATCGTAACCTTCATCTCTTCCACCCATGTCTCTTTGAGCAA
TAAAGCCGATAACAAAATCTTTGTCGCTCTTCGCAATGTCAACAGTACCCTTAGTATA
TTCTCCAGTAGATAGGGAGCCCTTGCATGACAATTCTGCTAACATCAAAAGGCCTCTA
GGTTCCTTTGTTACTTCTTCTGCCGCCTGCTTCAAACCGCTAACAATACCTGGGCCCA
CCACACCGTGTGCATTCGTAATGTCTGCCCATTCTGCTATTCTGTATACACCCGCAGA
GTACTGCAATTTGACTGTATTACCAATGTCAGCAAATTTTCTGTCTTCGAAGAGTAAAA
AATTGTACTTGGCGGATAATGCCTTTAGCGGCTTAACTGTGCCCTCCATGGAAAAATC
AGTCAAGATATCCACATGTGTTTTTAGTAAACAAATTTTGGGACCTAATGCTTCAACTA
ACTCCAGTAATTCCTTGGTGGTACGAACATCCAATGAAGCACACAAGTTTGTTTGCTT
TTCGTGCATGATATTAAATAGCTTGGCAGCAACAGGACTAGGATGAGTAGCAGCACG
TTCCTTATATGTAGCTTTCGACATGATTTATCTTCGTTTTTTGTTCTGTGCAGTTGGGT
TAAGAATACTGGGCAATTTCATGTTTCTTCAACACTACATATGCGTATATATACCAATC
TAAGTCTGTGCTCCTTCCTTCGTTCTTCCTTCTGTTCGGAGATTACCGAATCAAAAAA
ATTTCAAAGAAACCGAAATCAAAAAAAAGAATAAAAAAAAAATGATGAATTGAATTGAA
AAGCTACTTGTTACCCATCATTGAATTTTGAACATCCGAACCTGGGAGTTTTCCCTGA
AACAGATAGTATATTTGAACCTGTATAATAATATATAGTCTAGCGCTTTACGGAAGACA
 
ATGTATGTATTTCGGTTCCTGGAGAAACTATTGCATCTATTGCATAGGTAATCTTGCA
CGTCGCATCCCCGGTTCATTTTCTGCGTTTCCATCTTGCACTTCAATAGCATATCTTT
GTTAACGAAGCATCTGTGCTTCATTTTGTAGAACAAAAATGCAACGCGAGAGCGCTA
ATTTTTCAAACAAAGAATCTGAGCTGCATTTTTACAGAACAGAAATGCAACGCGAAAG
CGCTATTTTACCAACGAAGAATCTGTGCTTCATTTTTGTAAAACAAAAATGCAACGCG
AGAGCGCTAATTTTTCAAACAAAGAATCTGAGCTGCATTTTTACAGAACAGAAATGCA
ACGCGAGAGCGCTATTTTACCAACAAAGAATCTATACTTCTTTTTTGTTCTACAAAAAT
GCATCCCGAGAGCGCTATTTTTCTAACAAAGCATCTTAGATTACTTTTTTTCTCCTTTG
TGCGCTCTATAATGCAGTCTCTTGATAACTTTTTGCACTGTAGGTCCGTTAAGGTTAG
AAGAAGGCTACTTTGGTGTCTATTTTCTCTTCCATAAAAAAAGCCTGACTCCACTTCC
CGCGTTTACTGATTACTAGCGAAGCTGCGGGTGCATTTTTTCAAGATAAAGGCATCC
CCGATTATATTCTATACCGATGTGGATTGCGCATACTTTGTGAACAGAAAGTGATAGC
GTTGATGATTCTTCATTGGTCAGAAAATTATGAACGGTTTCTTCTATTTTGTCTCTATA
TACTACGTATAGGAAATGTTTACATTTTCGTATTGTTTTCGATTCACTCTATGAATAGT
TCTTACTACAATTTTTTTGTCTAAAGAGTAATACTAGAGATAAACATAAAAAATGTAGA
GGTCGAGTTTAGATGCAAGTTCAAGGAGCGAAAGGTGGATGGGTAGGTTATATAGG
GATATAGCACAGAGATATATAGCAAAGAGATACTTTTGAGCAATGTTTGTGGAAGCG
GTATTCGCAATATTTTAGTAGCTCGTTACAGTCCGGTGCGTTTTTGGTTTTTTGAAAGT
GCGTCTTCAGAGCGCTTTTGGTTTTCAAAAGCGCTCTGAAGTTCCTATACTTTCTAGC
TAGAGAATAGGAACTTCGGAATAGGAACTTCAAAGCGTTTCCGAAAACGAGCGCTTC
CGAAAATGCAACGCGAGCTGCGCACATACAGCTCACTGTTCACGTCGCACCTATATC
TGCGTGTTGCCTGTATATATATATACATGAGAAGAACGGCATAGTGCGTGTTTATGCT
TAAATGCGTTATGGTGCACTCTCAGTACAATCTGCTCTGATGCCGCATAGTTAAGCCA
GCCCCGACACCCGCCAACACCCGCTGACGCGCCCTGACGGGCTTGTCTGCTCCCG
GCATCCGCTTACAGACAAGCTGTGACCGTCTCCGGGAGCTGCATGTGTCAGAGGTT
TTCACCGTCATCACCGAAACGCGCGAGACGAAAGGGCCTCGTGATACGCCTATTTTT
ATAGGTTAATGTCATGATAATAATGGTTTCTTAGACGTCAGGTGGCACTTTTCGGGGA
AATGTGCGCGGAACCCCTATTTGTTTATTTTTCTAAATACATTCAAATATGTATCCGCT
CATGAGACAATAACCCTGATAAATGCTTCAATAATATTGAAAAAGGAAGAGTATGAGT
ATTCAACATTTCCGTGTCGCCCTTATTCCCTTTTTTGCGGCATTTTGCCTTCCTGTTTT
TGCTCACCCAGAAACGCTGGTGAAAGTAAAAGATGCTGAAGATCAGTTGGGTGCACG
AGTGGGTTACATCGAACTGGATCTCAACAGCGGTAAGATCCTTGAGAGTTTTCGCCC
CGAAGAACGTTTTCCAATGATGAGCACTTTTAAAGTTCTGCTATGTGGCGCGGTATTA
TCCCGTATTGACGCCGGGCAAGAGCAACTCGGTCGCCGCATACACTATTCTCAGAAT
GACTTGGTTGAGTACTCACCAGTCACAGAAAAGCATCTTACGGATGGCATGACAGTA
AGAGAATTATGCAGTGCTGCCATAACCATGAGTGATAACACTGCGGCCAACTTACTT
CTGACAACGATCGGAGGACCGAAGGAGCTAACCGCTTTTTTGCACAACATGGGGGA
 
TCATGTAACTCGCCTTGATCGTTGGGAACCGGAGCTGAATGAAGCCATACCAAACGA
CGAGCGTGACACCACGATGCCTGTAGCAATGGCAACAACGTTGCGCAAACTATTAAC
TGGCGAACTACTTACTCTAGCTTCCCGGCAACAATTAATAGACTGGATGGAGGCGGA
TAAAGTTGCAGGACCACTTCTGCGCTCGGCCCTTCCGGCTGGCTGGTTTATTGCTGA
TAAATCTGGAGCCGGTGAGCGTGGGTCTCGCGGTATCATTGCAGCACTGGGGCCAG
ATGGTAAGCCCTCCCGTATCGTAGTTATCTACACGACGGGGAGTCAGGCAACTATGG
ATGAACGAAATAGACAGATCGCTGAGATAGGTGCCTCACTGATTAAGCATTGGTAAC
TGTCAGACCAAGTTTACTCATATATACTTTAGATTGATTTAAAACTTCATTTTTAATTTA
AAAGGATCTAGGTGAAGATCCTTTTTGATAATCTCATGACCAAAATCCCTTAACGTGA
GTTTTCGTTCCACTGAGCGTCAGACCCCGTAGAAAAGATCAAAGGATCTTCTTGAGA
TCCTTTTTTTCTGCGCGTAATCTGCTGCTTGCAAACAAAAAAACCACCGCTACCAGCG
GTGGTTTGTTTGCCGGATCAAGAGCTACCAACTCTTTTTCCGAAGGTAACTGGCTTCA
GCAGAGCGCAGATACCAAATACTGTCCTTCTAGTGTAGCCGTAGTTAGGCCACCACT
TCAAGAACTCTGTAGCACCGCCTACATACCTCGCTCTGCTAATCCTGTTACCAGTGG
CTGCTGCCAGTGGCGATAAGTCGTGTCTTACCGGGTTGGACTCAAGACGATAGTTAC
CGGATAAGGCGCAGCGGTCGGGCTGAACGGGGGGTTCGTGCACACAGCCCAGCTT
GGAGCGAACGACCTACACCGAACTGAGATACCTACAGCGTGAGCATTGAGAAAGCG
CCACGCTTCCCGAAGGGAGAAAGGCGGACAGGTATCCGGTAAGCGGCAGGGTCGG
AACAGGAGAGCGCACGAGGGAGCTTCCAGGGGGAAACGCCTGGTATCTTTATAGTC
CTGTCGGGTTTCGCCACCTCTGACTTGAGCGTCGATTTTTGTGATGCTCGTCAGGGG
GGCGGAGCCTATGGAAAAACGCCAGCAACGCGGCCTTTTTACGGTTCCTGGCCTTTT
GCTGGCCTTTTGCTCACATGTTCTTTCCTGCGTTATCCCCTGATTCTGTGGATAACCG
TATTACCGCCTTTGAGTGAGCTGATACCGCTCGCCGCAGCCGAACGACCGAGCGCA
GCGAGTCAGTGAGCGAGGAAGCGGAAGAGCGCCCAATACGCAAACCGCCTCTCCC
CGCGCGTTGGCCGATTCATTCCCG
Sequence #8
   > **3' *lacZ* terminator** (reverse DNA primer)
   AAACCCCTCCGTTTAGAGAGGGGTTATGCTAGTTATTTTTGACACCAGACCAACTG
Sequence #9
   > **5' primer of RNA P&P artificial *lacZ-1***
   T7 promoter (in *italics)* 
TCATAAGCTTTAATACGACTCACTATAGGCCATCATAGTTGTTCTCTGGCGCTGTTGG
ACGAAAACAGTCCAGCAGCGCCGGGAAGGAGGGGCGAGGCATAAGTTCCTCCTTCT
TGGCGTTGTTGGACCTAAGAAGGAGGAATTTATGTCATATCTCACACAACGGGAGGT
CGTTTTACAACGTCGT
Sequence #10
   **> 5' primer of RNA P&P artificial *lacZ-2***
   T7 promoter (in *italics)* 
TCATAAGCTTTAATACGACTCACTATAGGCCATCATAGTTGTTCTCTGGCGCTGTTGG
ACGAAAACAGTCCAGCAGCGCCGGGAAGGAGGGGCGAGAGATAAGTTCCTCCTTCT
TGGCGTTGTTGGACCTAAGAAGGAGGAATTTATGTCATATCTCACACAACGGGAGGT
CGTTTTACAACGTCGT
Sequence #11
   > **5' primer of RNA P&P artificial *lacZ-3***
   T7 promoter (in *italics)* 
TCATAAGCTTTAATACGACTCACTATAGGCCTAAATATTATCCAACCCGGGCCAGCGG
CGGATAAAATGTCGTTGGTCCGGGAGAGGGAGGCGTGAAAAAATTGTCTTTCTTTTT
CGGATTAATGATACGAAAAAGGAGGATATCATGTACTACAGCTTCGATCCCGCCGTC
GTTTTACAACGTCGT
Sequence #12
   > **PCR product of RNA P&P artificial *lacZ-1***
   T7 promoter (in *italics),* YEp358 vector underlined, *lacZ* CDS in grey 
TCATAAGCTTTAATACGACTCACTATAGGCCATCATAGTTGTTCTCTGGCGCTGTTGG
ACGAAAACAGTCCAGCAGCGCCGGGAAGGAGGGGCGAGGCATAAGTTCCTCCTTCT
TGGCGTTGTTGGACCTAAGAAGGAGGAATTTATGTCATATCTCACACAACGGGAGGT
CGTTTTACAACGTCGTGACTGGGAAAACCCTGGCGTTACCCAACTTAATCGCCTTGC
AGCACATCCCCCTTTCGCCAGCTGGCGTAATAGCGAAGAGGCCCGCACCGATCGCC
CTTCCCAACAGTTGCGCAGCCTGAATGGCGAATGGCGCTTTGCCTGGTTTCCGGCA
CCAGAAGCGGTGCCGGAAAGCTGGCTGGAGTGCGATCTTCCTGAGGCCGATACTGT
CGTCGTCCCCTCAAACTGGCAGATGCACGGTTACGATGCGCCCATCTACACCAACG
TGACCTATCCCATTACGGTCAATCCGCCGTTTGTTCCCACGGAGAATCCGACGGGTT
GTTACTCGCTCACATTTAATGTTGATGAAAGCTGGCTACAGGAAGGCCAGACGCGAA
TTATTTTTGATGGCGTTAACTCGGCGTTTCATCTGTGGTGCAACGGGCGCTGGGTCG
GTTACGGCCAGGACAGTCGTTTGCCGTCTGAATTTGACCTGAGCGCATTTTTACGCG
CCGGAGAAAACCGCCTCGCGGTGATGGTGCTGCGCTGGAGTGACGGCAGTTATCTG
GAAGATCAGGATATGTGGCGGATGAGCGGCATTTTCCGTGACGTCTCGTTGCTGCAT
AAACCGACTACACAAATCAGCGATTTCCATGTTGCCACTCGCTTTAATGATGATTTCA
GCCGCGCTGTACTGGAGGCTGAAGTTCAGATGTGCGGCGAGTTGCGTGACTACCTA
CGGGTAACAGTTTCTTTATGGCAGGGTGAAACGCAGGTCGCCAGCGGCACCGCGCC
 
TTTCGGCGGTGAAATTATCGATGAGCGTGGTGGTTATGCCGATCGCGTCACACTACG
TCTGAACGTCGAAAACCCGAAACTGTGGAGCGCCGAAATCCCGAATCTCTATCGTGC
GGTGGTTGAACTGCACACCGCCGACGGCACGCTGATTGAAGCAGAAGCCTGCGATG
TCGGTTTCCGCGAGGTGCGGATTGAAAATGGTCTGCTGCTGCTGAACGGCAAGCCG
TTGCTGATTCGAGGCGTTAACCGTCACGAGCATCATCCTCTGCATGGTCAGGTCATG
GATGAGCAGACGATGGTGCAGGATATCCTGCTGATGAAGCAGAACAACTTTAACGCC
GTGCGCTGTTCGCATTATCCGAACCATCCGCTGTGGTACACGCTGTGCGACCGCTA
CGGCCTGTATGTGGTGGATGAAGCCAATATTGAAACCCACGGCATGGTGCCAATGAA
TCGTCTGACCGATGATCCGCGCTGGCTACCGGCGATGAGCGAACGCGTAACGCGAA
TGGTGCAGCGCGATCGTAATCACCCGAGTGTGATCATCTGGTCGCTGGGGAATGAA
TCAGGCCACGGCGCTAATCACGACGCGCTGTATCGCTGGATCAAATCTGTCGATCCT
TCCCGCCCGGTGCAGTATGAAGGCGGCGGAGCCGACACCACGGCCACCGATATTAT
TTGCCCGATGTACGCGCGCGTGGATGAAGACCAGCCCTTCCCGGCTGTGCCGAAAT
GGTCCATCAAAAAATGGCTTTCGCTACCTGGAGAGACGCGCCCGCTGATCCTTTGCG
AATACGCCCACGCGATGGGTAACAGTCTTGGCGGTTTCGCTAAATACTGGCAGGCG
TTTCGTCAGTATCCCCGTTTACAGGGCGGCTTCGTCTGGGACTGGGTGGATCAGTC
GCTGATTAAATATGATGAAAACGGCAACCCGTGGTCGGCTTACGGCGGTGATTTTGG
CGATACGCCGAACGATCGCCAGTTCTGTATGAACGGTCTGGTCTTTGCCGACCGCA
CGCCGCATCCAGCGCTGACGGAAGCAAAACACCAGCAGCAGTTTTTCCAGTTCCGTT
TATCCGGGCAAACCATCGAAGTGACCAGCGAATACCTGTTCCGTCATAGCGATAACG
AGCTCCTGCACTGGATGGTGGCGCTGGATGGTAAGCCGCTGGCAAGCGGTGAAGT
GCCTCTGGATGTCGCTCCACAAGGTAAACAGTTGATTGAACTGCCTGAACTACCGCA
GCCGGAGAGCGCCGGGCAACTCTGGCTCACAGTACGCGTAGTGCAACCGAACGCG
ACCGCATGGTCAGAAGCCGGGCACATCAGCGCCTGGCAGCAGTGGCGTCTGGCGG
AAAACCTCAGTGTGACGCTCCCCGCCGCGTCCCACGCCATCCCGCATCTGACCACC
AGCGAAATGGATTTTTGCATCGAGCTGGGTAATAAGCGTTGGCAATTTAACCGCCAG
TCAGGCTTTCTTTCACAGATGTGGATTGGCGATAAAAAACAACTGCTGACGCCGCTG
CGCGATCAGTTCACCCGTGCACCGCTGGATAACGACATTGGCGTAAGTGAAGCGAC
CCGCATTGACCCTAACGCCTGGGTCGAACGCTGGAAGGCGGCGGGCCATTACCAG
GCCGAAGCAGCGTTGTTGCAGTGCACGGCAGATACACTTGCTGATGCGGTGCTGAT
TACGACCGCTCACGCGTGGCAGCATCAGGGGAAAACCTTATTTATCAGCCGGAAAAC
CTACCGGATTGATGGTAGTGGTCAAATGGCGATTACCGTTGATGTTGAAGTGGCGAG
CGATACACCGCATCCGGCGCGGATTGGCCTGAACTGCCAGCTGGCGCAGGTAGCA
GAGCGGGTAAACTGGCTCGGATTAGGGCCGCAAGAAAACTATCCCGACCGCCTTAC
TGCCGCCTGTTTTGACCGCTGGGATCTGCCATTGTCAGACATGTATACCCCGTACGT
CTTCCCGAGCGAAAACGGTCTGCGCTGCGGGACGCGCGAATTGAATTATGGCCCAC
ACCAGTGGCGCGGCGACTTCCAGTTCAACATCAGCCGCTACAGTCAACAGCAACTG
 
ATGGAAACCAGCCATCGCCATCTGCTGCACGCGGAAGAAGGCACATGGCTGAATAT
CGACGGTTTCCATATGGGGATTGGTGGCGACGACTCCTGGAGCCCGTCAGTATCGG
CGGAATTCCAGCTGAGCGCCGGTCGCTACCATTACCAGTTGGTCTGGTGTCAAAAAT
AACTAGCATAACCCCTCTCTAAACGGAGGGGTTT
Sequence #13
   > **PCR product of RNA P&P artificial *lacZ-2***
   T7 promoter (in *italics),* YEp358 vector underlined, *lacZ* CDS in grey 
TCATAAGCTTTAATACGACTCACTATAGGCCATCATAGTTGTTCTCTGGCGCTGTTGG
ACGAAAACAGTCCAGCAGCGCCGGGAAGGAGGGGCGAGAGATAAGTTCCTCCTTCT
TGGCGTTGTTGGACCTAAGAAGGAGGAATTTATGTCATATCTCACACAACGGGAGGT
CGTTTTACAACGTCGTGACTGGGAAAACCCTGGCGTTACCCAACTTAATCGCCTTGC
AGCACATCCCCCTTTCGCCAGCTGGCGTAATAGCGAAGAGGCCCGCACCGATCGCC
CTTCCCAACAGTTGCGCAGCCTGAATGGCGAATGGCGCTTTGCCTGGTTTCCGGCA
CCAGAAGCGGTGCCGGAAAGCTGGCTGGAGTGCGATCTTCCTGAGGCCGATACTGT
CGTCGTCCCCTCAAACTGGCAGATGCACGGTTACGATGCGCCCATCTACACCAACG
TGACCTATCCCATTACGGTCAATCCGCCGTTTGTTCCCACGGAGAATCCGACGGGTT
GTTACTCGCTCACATTTAATGTTGATGAAAGCTGGCTACAGGAAGGCCAGACGCGAA
TTATTTTTGATGGCGTTAACTCGGCGTTTCATCTGTGGTGCAACGGGCGCTGGGTCG
GTTACGGCCAGGACAGTCGTTTGCCGTCTGAATTTGACCTGAGCGCATTTTTACGCG
CCGGAGAAAACCGCCTCGCGGTGATGGTGCTGCGCTGGAGTGACGGCAGTTATCTG
GAAGATCAGGATATGTGGCGGATGAGCGGCATTTTCCGTGACGTCTCGTTGCTGCAT
AAACCGACTACACAAATCAGCGATTTCCATGTTGCCACTCGCTTTAATGATGATTTCA
GCCGCGCTGTACTGGAGGCTGAAGTTCAGATGTGCGGCGAGTTGCGTGACTACCTA
CGGGTAACAGTTTCTTTATGGCAGGGTGAAACGCAGGTCGCCAGCGGCACCGCGCC
TTTCGGCGGTGAAATTATCGATGAGCGTGGTGGTTATGCCGATCGCGTCACACTACG
TCTGAACGTCGAAAACCCGAAACTGTGGAGCGCCGAAATCCCGAATCTCTATCGTGC
GGTGGTTGAACTGCACACCGCCGACGGCACGCTGATTGAAGCAGAAGCCTGCGATG
TCGGTTTCCGCGAGGTGCGGATTGAAAATGGTCTGCTGCTGCTGAACGGCAAGCCG
TTGCTGATTCGAGGCGTTAACCGTCACGAGCATCATCCTCTGCATGGTCAGGTCATG
GATGAGCAGACGATGGTGCAGGATATCCTGCTGATGAAGCAGAACAACTTTAACGCC
GTGCGCTGTTCGCATTATCCGAACCATCCGCTGTGGTACACGCTGTGCGACCGCTA
CGGCCTGTATGTGGTGGATGAAGCCAATATTGAAACCCACGGCATGGTGCCAATGAA
TCGTCTGACCGATGATCCGCGCTGGCTACCGGCGATGAGCGAACGCGTAACGCGAA
TGGTGCAGCGCGATCGTAATCACCCGAGTGTGATCATCTGGTCGCTGGGGAATGAA
TCAGGCCACGGCGCTAATCACGACGCGCTGTATCGCTGGATCAAATCTGTCGATCCT
TCCCGCCCGGTGCAGTATGAAGGCGGCGGAGCCGACACCACGGCCACCGATATTAT
 
TTGCCCGATGTACGCGCGCGTGGATGAAGACCAGCCCTTCCCGGCTGTGCCGAAAT
GGTCCATCAAAAAATGGCTTTCGCTACCTGGAGAGACGCGCCCGCTGATCCTTTGCG
AATACGCCCACGCGATGGGTAACAGTCTTGGCGGTTTCGCTAAATACTGGCAGGCG
TTTCGTCAGTATCCCCGTTTACAGGGCGGCTTCGTCTGGGACTGGGTGGATCAGTC
GCTGATTAAATATGATGAAAACGGCAACCCGTGGTCGGCTTACGGCGGTGATTTTGG
CGATACGCCGAACGATCGCCAGTTCTGTATGAACGGTCTGGTCTTTGCCGACCGCA
CGCCGCATCCAGCGCTGACGGAAGCAAAACACCAGCAGCAGTTTTTCCAGTTCCGTT
TATCCGGGCAAACCATCGAAGTGACCAGCGAATACCTGTTCCGTCATAGCGATAACG
AGCTCCTGCACTGGATGGTGGCGCTGGATGGTAAGCCGCTGGCAAGCGGTGAAGT
GCCTCTGGATGTCGCTCCACAAGGTAAACAGTTGATTGAACTGCCTGAACTACCGCA
GCCGGAGAGCGCCGGGCAACTCTGGCTCACAGTACGCGTAGTGCAACCGAACGCG
ACCGCATGGTCAGAAGCCGGGCACATCAGCGCCTGGCAGCAGTGGCGTCTGGCGG
AAAACCTCAGTGTGACGCTCCCCGCCGCGTCCCACGCCATCCCGCATCTGACCACC
AGCGAAATGGATTTTTGCATCGAGCTGGGTAATAAGCGTTGGCAATTTAACCGCCAG
TCAGGCTTTCTTTCACAGATGTGGATTGGCGATAAAAAACAACTGCTGACGCCGCTG
CGCGATCAGTTCACCCGTGCACCGCTGGATAACGACATTGGCGTAAGTGAAGCGAC
CCGCATTGACCCTAACGCCTGGGTCGAACGCTGGAAGGCGGCGGGCCATTACCAG
GCCGAAGCAGCGTTGTTGCAGTGCACGGCAGATACACTTGCTGATGCGGTGCTGAT
TACGACCGCTCACGCGTGGCAGCATCAGGGGAAAACCTTATTTATCAGCCGGAAAAC
CTACCGGATTGATGGTAGTGGTCAAATGGCGATTACCGTTGATGTTGAAGTGGCGAG
CGATACACCGCATCCGGCGCGGATTGGCCTGAACTGCCAGCTGGCGCAGGTAGCA
GAGCGGGTAAACTGGCTCGGATTAGGGCCGCAAGAAAACTATCCCGACCGCCTTAC
TGCCGCCTGTTTTGACCGCTGGGATCTGCCATTGTCAGACATGTATACCCCGTACGT
CTTCCCGAGCGAAAACGGTCTGCGCTGCGGGACGCGCGAATTGAATTATGGCCCAC
ACCAGTGGCGCGGCGACTTCCAGTTCAACATCAGCCGCTACAGTCAACAGCAACTG
ATGGAAACCAGCCATCGCCATCTGCTGCACGCGGAAGAAGGCACATGGCTGAATAT
CGACGGTTTCCATATGGGGATTGGTGGCGACGACTCCTGGAGCCCGTCAGTATCGG
CGGAATTCCAGCTGAGCGCCGGTCGCTACCATTACCAGTTGGTCTGGTGTCAAAAAT
AACTAGCATAACCCCTCTCTAAACGGAGGGGTTT
Sequence #14
   > **PCR product of RNA P&P artificial *lacZ-3***
   T7 promoter (in *italics),* YEp358 vector underlined, *lacZ* CDS in grey 
TCATAAGCTTTAATACGACTCACTATAGGCCTAAATATTATCCAACCCGGGCCAGCGG
CGGATAAAATGTCGTTGGTCCGGGAGAGGGAGGCGTGAAAAAATTGTCTTTCTTTTT
CGGATTAATGATACGAAAAAGGAGGATATCATGTACTACAGCTTCGATCCCGCCGTC
GTTTTACAACGTCGTGACTGGGAAAACCCTGGCGTTACCCAACTTAATCGCCTTGCA
 
GCACATCCCCCTTTCGCCAGCTGGCGTAATAGCGAAGAGGCCCGCACCGATCGCCC
TTCCCAACAGTTGCGCAGCCTGAATGGCGAATGGCGCTTTGCCTGGTTTCCGGCAC
CAGAAGCGGTGCCGGAAAGCTGGCTGGAGTGCGATCTTCCTGAGGCCGATACTGTC
GTCGTCCCCTCAAACTGGCAGATGCACGGTTACGATGCGCCCATCTACACCAACGT
GACCTATCCCATTACGGTCAATCCGCCGTTTGTTCCCACGGAGAATCCGACGGGTTG
TTACTCGCTCACATTTAATGTTGATGAAAGCTGGCTACAGGAAGGCCAGACGCGAAT
TATTTTTGATGGCGTTAACTCGGCGTTTCATCTGTGGTGCAACGGGCGCTGGGTCGG
TTACGGCCAGGACAGTCGTTTGCCGTCTGAATTTGACCTGAGCGCATTTTTACGCGC
CGGAGAAAACCGCCTCGCGGTGATGGTGCTGCGCTGGAGTGACGGCAGTTATCTGG
AAGATCAGGATATGTGGCGGATGAGCGGCATTTTCCGTGACGTCTCGTTGCTGCATA
AACCGACTACACAAATCAGCGATTTCCATGTTGCCACTCGCTTTAATGATGATTTCAG
CCGCGCTGTACTGGAGGCTGAAGTTCAGATGTGCGGCGAGTTGCGTGACTACCTAC
GGGTAACAGTTTCTTTATGGCAGGGTGAAACGCAGGTCGCCAGCGGCACCGCGCCT
TTCGGCGGTGAAATTATCGATGAGCGTGGTGGTTATGCCGATCGCGTCACACTACGT
CTGAACGTCGAAAACCCGAAACTGTGGAGCGCCGAAATCCCGAATCTCTATCGTGC
GGTGGTTGAACTGCACACCGCCGACGGCACGCTGATTGAAGCAGAAGCCTGCGATG
TCGGTTTCCGCGAGGTGCGGATTGAAAATGGTCTGCTGCTGCTGAACGGCAAGCCG
TTGCTGATTCGAGGCGTTAACCGTCACGAGCATCATCCTCTGCATGGTCAGGTCATG
GATGAGCAGACGATGGTGCAGGATATCCTGCTGATGAAGCAGAACAACTTTAACGCC
GTGCGCTGTTCGCATTATCCGAACCATCCGCTGTGGTACACGCTGTGCGACCGCTA
CGGCCTGTATGTGGTGGATGAAGCCAATATTGAAACCCACGGCATGGTGCCAATGAA
TCGTCTGACCGATGATCCGCGCTGGCTACCGGCGATGAGCGAACGCGTAACGCGAA
TGGTGCAGCGCGATCGTAATCACCCGAGTGTGATCATCTGGTCGCTGGGGAATGAA
TCAGGCCACGGCGCTAATCACGACGCGCTGTATCGCTGGATCAAATCTGTCGATCCT
TCCCGCCCGGTGCAGTATGAAGGCGGCGGAGCCGACACCACGGCCACCGATATTAT
TTGCCCGATGTACGCGCGCGTGGATGAAGACCAGCCCTTCCCGGCTGTGCCGAAAT
GGTCCATCAAAAAATGGCTTTCGCTACCTGGAGAGACGCGCCCGCTGATCCTTTGCG
AATACGCCCACGCGATGGGTAACAGTCTTGGCGGTTTCGCTAAATACTGGCAGGCG
TTTCGTCAGTATCCCCGTTTACAGGGCGGCTTCGTCTGGGACTGGGTGGATCAGTC
GCTGATTAAATATGATGAAAACGGCAACCCGTGGTCGGCTTACGGCGGTGATTTTGG
CGATACGCCGAACGATCGCCAGTTCTGTATGAACGGTCTGGTCTTTGCCGACCGCA
CGCCGCATCCAGCGCTGACGGAAGCAAAACACCAGCAGCAGTTTTTCCAGTTCCGTT
TATCCGGGCAAACCATCGAAGTGACCAGCGAATACCTGTTCCGTCATAGCGATAACG
AGCTCCTGCACTGGATGGTGGCGCTGGATGGTAAGCCGCTGGCAAGCGGTGAAGT
GCCTCTGGATGTCGCTCCACAAGGTAAACAGTTGATTGAACTGCCTGAACTACCGCA
GCCGGAGAGCGCCGGGCAACTCTGGCTCACAGTACGCGTAGTGCAACCGAACGCG
ACCGCATGGTCAGAAGCCGGGCACATCAGCGCCTGGCAGCAGTGGCGTCTGGCGG
 
AAAACCTCAGTGTGACGCTCCCCGCCGCGTCCCACGCCATCCCGCATCTGACCACC
AGCGAAATGGATTTTTGCATCGAGCTGGGTAATAAGCGTTGGCAATTTAACCGCCAG
TCAGGCTTTCTTTCACAGATGTGGATTGGCGATAAAAAACAACTGCTGACGCCGCTG
CGCGATCAGTTCACCCGTGCACCGCTGGATAACGACATTGGCGTAAGTGAAGCGAC
CCGCATTGACCCTAACGCCTGGGTCGAACGCTGGAAGGCGGCGGGCCATTACCAG
GCCGAAGCAGCGTTGTTGCAGTGCACGGCAGATACACTTGCTGATGCGGTGCTGAT
TACGACCGCTCACGCGTGGCAGCATCAGGGGAAAACCTTATTTATCAGCCGGAAAAC
CTACCGGATTGATGGTAGTGGTCAAATGGCGATTACCGTTGATGTTGAAGTGGCGAG
CGATACACCGCATCCGGCGCGGATTGGCCTGAACTGCCAGCTGGCGCAGGTAGCA
GAGCGGGTAAACTGGCTCGGATTAGGGCCGCAAGAAAACTATCCCGACCGCCTTAC
TGCCGCCTGTTTTGACCGCTGGGATCTGCCATTGTCAGACATGTATACCCCGTACGT
CTTCCCGAGCGAAAACGGTCTGCGCTGCGGGACGCGCGAATTGAATTATGGCCCAC
ACCAGTGGCGCGGCGACTTCCAGTTCAACATCAGCCGCTACAGTCAACAGCAACTG
ATGGAAACCAGCCATCGCCATCTGCTGCACGCGGAAGAAGGCACATGGCTGAATAT
CGACGGTTTCCATATGGGGATTGGTGGCGACGACTCCTGGAGCCCGTCAGTATCGG
CGGAATTCCAGCTGAGCGCCGGTCGCTACCATTACCAGTTGGTCTGGTGTCAAAAAT
AACTAGCATAACCCCTCTCTAAACGGAGGGGTTT
Sequence #15
   **> NR 024570.1 *Escherichia coli* strain U 5/41 16S ribosomal RNA,** partial sequence 
AGTTTGATCATGGCTCAGATTGAACGCTGGCGGCAGGCCTAACACATGCAAGTCGAA
CGGTAACAGGAAGCAGCTTGCTGCTTTGCTGACGAGTGGCGGACGGGTGAGTAATG
TCTGGGAAACTGCCTGATGGAGGGGGATAACTACTGGAAACGGTAGCTAATACCGC
ATAACGTCGCAAGCACAAAGAGGGGGACCTTAGGGCCTCTTGCCATCGGATGTGCC
CAGATGGGATTAGCTAGTAGGTGGGGTAACGGCTCACCTAGGCGACGATCCCTAGC
TGGTCTGAGAGGATGACCAGCAACACTGGAACTGAGACACGGTCCAGACTCCTACG
GGAGGCAGCAGTGGGGAATATTGCACAATGGGCGCAAGCCTGATGCAGCCATGCC
GCGTGTATGAAGAAGGCCTTCGGGTTGTAAAGTACTTTCAGCGGGGAGGAAGGGAG
TAAAGTTAATACCTTTGCTCATTGACGTTACCCGCAGAAGAAGCACCGGCTAACTCC
GTGCCAGCAGCCGCGGTAATACGGAGGGTGCAAGCGTTAATCGGAATTACTGGGCG
TAAAGCGCACGCAGGCGGTTTGTTAAGTCAGATGTGAAATCCCCGGGCTCAACCTG
GGAACTGCATCTGATACTGGCAAGCTTGAGTCTCGTAGAGGGGGGTAGAATTCCAG
GTGTAGCGGTGAAATGCGTAGAGATCTGGAGGAATACCGGTGGCGAAGGCGGCCC
CCTGGACGAAGACTGACGCTCAGGTGCGAAAGCGTGGGGAGCAAACAGGATTAGAT
ACCCTGGTAGTCCACGCCGTAAACGATGTCGACTTGGAGGTTGTGCCCTTGAGGCG
TGGCTTCCGGAGCTAACGCGTTAAGTCGACCGCCTGGGGAGTACGGCCGCAAGGTT
AAAACTCAAATGAATTGACGGGGGCCGCACAAGCGGTGGAGCATGTGGTTTAATTCG
 
ATGCAACGCGAAGAACCTTACCTGGTCTTGACATCCACGGAAGTTTTCAGAGATGAG
AATGTGCCTTCGGGAACCGTGAGACAGGTGCTGCATGGCT
GTCGTCAGCTCGTGTTGTGAAATGTTGGGTTAAGTCCCGCAACGAGCGCAACCCTTA
TCCTTTGTTGCCAGCGGTCCGGCCGGGAACTCAAAGGAGACTGCCAGTGATAAACT
GGAGGAAGGTGGGGATGACGTCAAGTCATCATGGCCCTTACGACCAGGGCTACACA
CGTGCTACAATGGCGCATACAAAGAGAAGCGACCTCGCGAGAGCAAGCGGACCTCA
TAAAGTGCGTCGTAGTCCGGATTGGAGTCTGCAACTCGACTCCATGAAGTCGGAATC
GCTAGTAATCGTGGATCAGAATGCCACGGTGAATACGTTCCCGGGCCTTGTACACAC
CGCCCGTCACACCATGGGAGTGGGTTGCAAAAGAAGTAGGTAGCTTAACTTCGGGA
GGGCG
Sequence #16:
   **> RNA P&P *E.coli lacZ*-1** 
GGCGUGUAGUUGUGCUGCGUGUGUGAGGAGGGCUGAAAGAGCUCUUUUCGUAUA
UAAAGGAGGGUACCUAUAAUCUGUCCUCCUUUAUAUACGAAAAGAGUAUAUAAAGG
AGGACAAUAUGCACAUACAAAGCUUCGAUCCCGCCGUCGUUUUACAACG
Sequence #17:
   > **RNA P&P *E.coli lacZ-2*** 
GGCGUGUAGUUGUGCUGCGUGUGAGGAGGGCUGAGAGCUCUUUUCGUAUA
UAAAGGAGGGUACCUAUAAUCUGUCCUCCUUUAUAUACGAAAAGAGUAUAUAAAGG
AGGACAAUAUGCACAUACAAAGCUUCGAUCCCGCCGUCGUUUACAACG
Sequence #18:
   > **Synthetic RNA target of 16S ribosomal RNA** (nt 357-432, reverse complement) 
UGAAAGUACUUUACAACCCGAAGGCCUUCUUCAUACACGCGGCAUGGCUGCAUCA
GGCUUGUUGCGCCCAUUCUGCAA
Sequence #19:
   > **5' primer of P&P *E.coli lacZ-1***
   T7 promoter (in *italics)* 
TCATAAGCTTTAATACGACTCACTATAGGCGTGTAGTTGTGCTGCGTGTGTGAGGAG
GGCTGAAAGAGCTCTTTTCGTATATAAAGGAGGGTACCTATAATCTGTCCTCCTTTAT
ATACGAAAAGAGTATATAAAGGAGGACAATATGCACATACAAAGCTTCGATCCCGCC
GTCGTTTTACAACG
Sequence #20:
   > **5' primer of RNA P&P *E.coli lacZ-2***
   T7 promoter (in *italics)* 
TCATAAGCTTTAATACGACTCACTATAGGCGTGTAGTTGTGCTGCGTGTGTGAGGAG
GGCTGAGAGAGCTCTTTTCGTATATAAAGGAGGGTACCTATAATCTGTCCTCCTTTAT
ATACGAAAAGAGTATATAAAGGAGGACAATATGCACATACAAAGCTTCGATCCCGCC
GTCGTTTTACAACG
Sequence #21:
   **> PCR product of RNA P&P *E.coli lacZ-1***
   T7 promoter (in *italics),* YEp358 vector underlined, *lacZ* CDS in grey 
TCATAAGCTTTAATACGACTCACTATAGGCGTGTAGTTGTGCTGCGTGTGTGAGGAG
GGCTGAAAGAGCTCTTTTCGTATATAAAGGAGGGTACCTATAATCTGTCCTCCTTTAT
ATACGAAAAGAGTATATAAAGGAGGACAATATGCACATACAAAGCTTCGATCCCGCC
GTCGTTTTACAACGTCGTGACTGGGAAAACCCTGGCGTTACCCAACTTAATCGCCTT
GCAGCACATCCCCCTTTCGCCAGCTGGCGTAATAGCGAAGAGGCCCGCACCGATCG
CCCTTCCCAACAGTTGCGCAGCCTGAATGGCGAATGGCGCTTTGCCTGGTTTCCGG
CACCAGAAGCGGTGCCGGAAAGCTGGCTGGAGTGCGATCTTCCTGAGGCCGATACT
GTCGTCGTCCCCTCAAACTGGCAGATGCACGGTTACGATGCGCCCATCTACACCAA
CGTGACCTATCCCATTACGGTCAATCCGCCGTTTGTTCCCACGGAGAATCCGACGGG
TTGTTACTCGCTCACATTTAATGTTGATGAAAGCTGGCTACAGGAAGGCCAGACGCG
AATTATTTTTGATGGCGTTAACTCGGCGTTTCATCTGTGGTGCAACGGGCGCTGGGT
CGGTTACGGCCAGGACAGTCGTTTGCCGTCTGAATTTGACCTGAGCGCATTTTTACG
CGCCGGAGAAAACCGCCTCGCGGTGATGGTGCTGCGCTGGAGTGACGGCAGTTAT
CTGGAAGATCAGGATATGTGGCGGATGAGCGGCATTTTCCGTGACGTCTCGTTGCT
GCATAAACCGACTACACAAATCAGCGATTTCCATGTTGCCACTCGCTTTAATGATGAT
TTCAGCCGCGCTGTACTGGAGGCTGAAGTTCAGATGTGCGGCGAGTTGCGTGACTA
CCTACGGGTAACAGTTTCTTTATGGCAGGGTGAAACGCAGGTCGCCAGCGGCACCG
CGCCTTTCGGCGGTGAAATTATCGATGAGCGTGGTGGTTATGCCGATCGCGTCACA
CTACGTCTGAACGTCGAAAACCCGAAACTGTGGAGCGCCGAAATCCCGAATCTCTAT
CGTGCGGTGGTTGAACTGCACACCGCCGACGGCACGCTGATTGAAGCAGAAGCCTG
CGATGTCGGTTTCCGCGAGGTGCGGATTGAAAATGGTCTGCTGCTGCTGAACGGCA
AGCCGTTGCTGATTCGAGGCGTTAACCGTCACGAGCATCATCCTCTGCATGGTCAGG
TCATGGATGAGCAGACGATGGTGCAGGATATCCTGCTGATGAAGCAGAACAACTTTA
ACGCCGTGCGCTGTTCGCATTATCCGAACCATCCGCTGTGGTACACGCTGTGCGAC
CGCTACGGCCTGTATGTGGTGGATGAAGCCAATATTGAAACCCACGGCATGGTGCC
AATGAATCGTCTGACCGATGATCCGCGCTGGCTACCGGCGATGAGCGAACGCGTAA
CGCGAATGGTGCAGCGCGATCGTAATCACCCGAGTGTGATCATCTGGTCGCTGGGG
 
AATGAATCAGGCCACGGCGCTAATCACGACGCGCTGTATCGCTGGATCAAATCTGTC
GATCCTTCCCGCCCGGTGCAGTATGAAGGCGGCGGAGCCGACACCACGGCCACCG
ATATTATTTGCCCGATGTACGCGCGCGTGGATGAAGACCAGCCCTTCCCGGCTGTG
CCGAAATGGTCCATCAAAAAATGGCTTTCGCTACCTGGAGAGACGCGCCCGCTGATC
CTTTGCGAATACGCCCACGCGATGGGTAACAGTCTTGGCGGTTTCGCTAAATACTGG
CAGGCGTTTCGTCAGTATCCCCGTTTACAGGGCGGCTTCGTCTGGGACTGGGTGGA
TCAGTCGCTGATTAAATATGATGAAAACGGCAACCCGTGGTCGGCTTACGGCGGTGA
TTTTGGCGATACGCCGAACGATCGCCAGTTCTGTATGAACGGTCTGGTCTTTGCCGA
CCGCACGCCGCATCCAGCGCTGACGGAAGCAAAACACCAGCAGCAGTTTTTCCAGT
TCCGTTTATCCGGGCAAACCATCGAAGTGACCAGCGAATACCTGTTCCGTCATAGCG
ATAACGAGCTCCTGCACTGGATGGTGGCGCTGGATGGTAAGCCGCTGGCAAGCGGT
GAAGTGCCTCTGGATGTCGCTCCACAAGGTAAACAGTTGATTGAACTGCCTGAACTA
CCGCAGCCGGAGAGCGCCGGGCAACTCTGGCTCACAGTACGCGTAGTGCAACCGA
ACGCGACCGCATGGTCAGAAGCCGGGCACATCAGCGCCTGGCAGCAGTGGCGTCT
GGCGGAAAACCTCAGTGTGACGCTCCCCGCCGCGTCCCACGCCATCCCGCATCTGA
CCACCAGCGAAATGGATTTTTGCATCGAGCTGGGTAATAAGCGTTGGCAATTTAACC
GCCAGTCAGGCTTTCTTTCACAGATGTGGATTGGCGATAAAAAACAACTGCTGACGC
CGCTGCGCGATCAGTTCACCCGTGCACCGCTGGATAACGACATTGGCGTAAGTGAA
GCGACCCGCATTGACCCTAACGCCTGGGTCGAACGCTGGAAGGCGGCGGGCCATT
ACCAGGCCGAAGCAGCGTTGTTGCAGTGCACGGCAGATACACTTGCTGATGCGGTG
CTGATTACGACCGCTCACGCGTGGCAGCATCAGGGGAAAACCTTATTTATCAGCCGG
AAAACCTACCGGATTGATGGTAGTGGTCAAATGGCGATTACCGTTGATGTTGAAGTG
GCGAGCGATACACCGCATCCGGCGCGGATTGGCCTGAACTGCCAGCTGGCGCAGG
TAGCAGAGCGGGTAAACTGGCTCGGATTAGGGCCGCAAGAAAACTATCCCGACCGC
CTTACTGCCGCCTGTTTTGACCGCTGGGATCTGCCATTGTCAGACATGTATACCCCG
TACGTCTTCCCGAGCGAAAACGGTCTGCGCTGCGGGACGCGCGAATTGAATTATGG
CCCACACCAGTGGCGCGGCGACTTCCAGTTCAACATCAGCCGCTACAGTCAACAGC
AACTGATGGAAACCAGCCATCGCCATCTGCTGCACGCGGAAGAAGGCACATGGCTG
AATATCGACGGTTTCCATATGGGGATTGGTGGCGACGACTCCTGGAGCCCGTCAGT
ATCGGCGGAATTCCAGCTGAGCGCCGGTCGCTACCATTACCAGTTGGTCTGGTGTC
AAAAATAACTAGCATAACCCCTCTCTAAACGGAGGGGTTT
Sequence #22:
   > **PCR product of RNA P&P *E.coli lacZ-2***
   T7 promoter (in *italics),* YEp358 vector underlined, *lacZ* CDS in grey 
TCATAAGCTTTAATACGACTCACTATAGGCGTGTAGTTGTGCTGCGTGTGTGAGGAG
GGCTGAGAGAGCTCTTTTCGTATATAAAGGAGGGTACCTATAATCTGTCCTCCTTTAT
 
ATACGAAAAGAGTATATAAAGGAGGACAATATGCACATACAAAGCTTCGATCCCGCC
GTCGTTTTACAACGTCGTGACTGGGAAAACCCTGGCGTTACCCAACTTAATCGCCTT
GCAGCACATCCCCCTTTCGCCAGCTGGCGTAATAGCGAAGAGGCCCGCACCGATCG
CCCTTCCCAACAGTTGCGCAGCCTGAATGGCGAATGGCGCTTTGCCTGGTTTCCGG
CACCAGAAGCGGTGCCGGAAAGCTGGCTGGAGTGCGATCTTCCTGAGGCCGATACT
GTCGTCGTCCCCTCAAACTGGCAGATGCACGGTTACGATGCGCCCATCTACACCAA
CGTGACCTATCCCATTACGGTCAATCCGCCGTTTGTTCCCACGGAGAATCCGACGGG
TTGTTACTCGCTCACATTTAATGTTGATGAAAGCTGGCTACAGGAAGGCCAGACGCG
AATTATTTTTGATGGCGTTAACTCGGCGTTTCATCTGTGGTGCAACGGGCGCTGGGT
CGGTTACGGCCAGGACAGTCGTTTGCCGTCTGAATTTGACCTGAGCGCATTTTTACG
CGCCGGAGAAAACCGCCTCGCGGTGATGGTGCTGCGCTGGAGTGACGGCAGTTAT
CTGGAAGATCAGGATATGTGGCGGATGAGCGGCATTTTCCGTGACGTCTCGTTGCT
GCATAAACCGACTACACAAATCAGCGATTTCCATGTTGCCACTCGCTTTAATGATGAT
TTCAGCCGCGCTGTACTGGAGGCTGAAGTTCAGATGTGCGGCGAGTTGCGTGACTA
CCTACGGGTAACAGTTTCTTTATGGCAGGGTGAAACGCAGGTCGCCAGCGGCACCG
CGCCTTTCGGCGGTGAAATTATCGATGAGCGTGGTGGTTATGCCGATCGCGTCACA
CTACGTCTGAACGTCGAAAACCCGAAACTGTGGAGCGCCGAAATCCCGAATCTCTAT
CGTGCGGTGGTTGAACTGCACACCGCCGACGGCACGCTGATTGAAGCAGAAGCCTG
CGATGTCGGTTTCCGCGAGGTGCGGATTGAAAATGGTCTGCTGCTGCTGAACGGCA
AGCCGTTGCTGATTCGAGGCGTTAACCGTCACGAGCATCATCCTCTGCATGGTCAGG
TCATGGATGAGCAGACGATGGTGCAGGATATCCTGCTGATGAAGCAGAACAACTTTA
ACGCCGTGCGCTGTTCGCATTATCCGAACCATCCGCTGTGGTACACGCTGTGCGAC
CGCTACGGCCTGTATGTGGTGGATGAAGCCAATATTGAAACCCACGGCATGGTGCC
AATGAATCGTCTGACCGATGATCCGCGCTGGCTACCGGCGATGAGCGAACGCGTAA
CGCGAATGGTGCAGCGCGATCGTAATCACCCGAGTGTGATCATCTGGTCGCTGGGG
AATGAATCAGGCCACGGCGCTAATCACGACGCGCTGTATCGCTGGATCAAATCTGTC
GATCCTTCCCGCCCGGTGCAGTATGAAGGCGGCGGAGCCGACACCACGGCCACCG
ATATTATTTGCCCGATGTACGCGCGCGTGGATGAAGACCAGCCCTTCCCGGCTGTG
CCGAAATGGTCCATCAAAAAATGGCTTTCGCTACCTGGAGAGACGCGCCCGCTGATC
CTTTGCGAATACGCCCACGCGATGGGTAACAGTCTTGGCGGTTTCGCTAAATACTGG
CAGGCGTTTCGTCAGTATCCCCGTTTACAGGGCGGCTTCGTCTGGGACTGGGTGGA
TCAGTCGCTGATTAAATATGATGAAAACGGCAACCCGTGGTCGGCTTACGGCGGTGA
TTTTGGCGATACGCCGAACGATCGCCAGTTCTGTATGAACGGTCTGGTCTTTGCCGA
CCGCACGCCGCATCCAGCGCTGACGGAAGCAAAACACCAGCAGCAGTTTTTCCAGT
TCCGTTTATCCGGGCAAACCATCGAAGTGACCAGCGAATACCTGTTCCGTCATAGCG
ATAACGAGCTCCTGCACTGGATGGTGGCGCTGGATGGTAAGCCGCTGGCAAGCGGT
GAAGTGCCTCTGGATGTCGCTCCACAAGGTAAACAGTTGATTGAACTGCCTGAACTA
 
CCGCAGCCGGAGAGCGCCGGGCAACTCTGGCTCACAGTACGCGTAGTGCAACCGA
ACGCGACCGCATGGTCAGAAGCCGGGCACATCAGCGCCTGGCAGCAGTGGCGTCT
GGCGGAAAACCTCAGTGTGACGCTCCCCGCCGCGTCCCACGCCATCCCGCATCTGA
CCACCAGCGAAATGGATTTTTGCATCGAGCTGGGTAATAAGCGTTGGCAATTTAACC
GCCAGTCAGGCTTTCTTTCACAGATGTGGATTGGCGATAAAAAACAACTGCTGACGC
CGCTGCGCGATCAGTTCACCCGTGCACCGCTGGATAACGACATTGGCGTAAGTGAA
GCGACCCGCATTGACCCTAACGCCTGGGTCGAACGCTGGAAGGCGGCGGGCCATT
ACCAGGCCGAAGCAGCGTTGTTGCAGTGCACGGCAGATACACTTGCTGATGCGGTG
CTGATTACGACCGCTCACGCGTGGCAGCATCAGGGGAAAACCTTATTTATCAGCCGG
AAAACCTACCGGATTGATGGTAGTGGTCAAATGGCGATTACCGTTGATGTTGAAGTG
GCGAGCGATACACCGCATCCGGCGCGGATTGGCCTGAACTGCCAGCTGGCGCAGG
TAGCAGAGCGGGTAAACTGGCTCGGATTAGGGCCGCAAGAAAACTATCCCGACCGC
CTTACTGCCGCCTGTTTTGACCGCTGGGATCTGCCATTGTCAGACATGTATACCCCG
TACGTCTTCCCGAGCGAAAACGGTCTGCGCTGCGGGACGCGCGAATTGAATTATGG
CCCACACCAGTGGCGCGGCGACTTCCAGTTCAACATCAGCCGCTACAGTCAACAGC
AACTGATGGAAACCAGCCATCGCCATCTGCTGCACGCGGAAGAAGGCACATGGCTG
AATATCGACGGTTTCCATATGGGGATTGGTGGCGACGACTCCTGGAGCCCGTCAGT
ATCGGCGGAATTCCAGCTGAGCGCCGGTCGCTACCATTACCAGTTGGTCTGGTGTC
AAAAATAACTAGCATAACCCCTCTCTAAACGGAGGGGTTT
Sequence #23:
   *>* ***gfpmut3B-ASV* gene** (CDS) 
ATGCGTAAAGGAGAAGAACTTTTCACTGGAGTTGTCCCAATTCTTGTTGAATTAGATG
GTGATGTTAATGGGCACAAATTTTCTGTCAGTGGAGAGGGTGAAGGTGATGCAACAT
ACGGAAAACTTACCCTTAAATTTATTTGCACTACTGGAAAACTACCTGTTCCGTGGCC
AACACTTGTCACTACTTTCGGTTATGGTGTTCAATGCTTTGCGAGATACCCAGATCAC
ATGAAACAGCATGACTTTTTCAAGAGTGCCATGCCCGAAGGTTACGTACAGGAAAGA
ACTATATTTTTCAAAGATGACGGGAACTACAAGACACGTGCTGAAGTCAAGTTTGAAG
GTGATACCCTTGTTAATAGAATCGAGTTAAAAGGTATTGATTTTAAAGAAGATGGAAA
CATTCTTGGACACAAATTGGAATACAACTATAACTCACACAATGTATACATCATGGCA
GACAAACAAAAGAATGGAATCAAAGTTAACTTCAAAATTAGACACAACATTGAAGATG
GAAGCGTTCAACTAGCAGACCATTATCAACAAAATACTCCGATTGGCGATGGCCCTG
TCCTTTTACCAGACAACCATTACCTGTCCACACAATCTGCCCTTTCGAAAGATCCCAA
CGAAAAGAGAGACCACATGGTCCTTCTTGAGTTTGTAACCGCTGCTGGGATTACACA
TGGCATGGATGAACTATACAAAAGGCCTGCAGCAAACGACGAAAACTACGCTGCATC
AGTTTAATAA
Sequence #24:
   > **RNA P&P *E.coli* GFP-1** 
GGCGUGGCGAGUUUGGUGCGGUCGUGCUGUGUGGUAAAUACGUAUAGUACGAUC
GGAGGGAGGGAGAACAAUCGGUUUUCUCCUUUUGAUCGUAUUAUACGAUCAAAAG
GAGGAGAUAAUGUACAUUUGCAACCUGGCGGCAGCGCAAAAGAUGCGUAAAGGAG
AAG
Sequence #25:
   > **RNA P&P *E.coli* GFP-2** 
GGCGUGUAGCUAUGUUGCGUGUAUGGGGAGGGCGAAAAUCGCUUUUCUCGUAUA
UAAAGGAGGGAGCACUUAAUACUUCCUCCUUUAUAUACGAGAAGGGUAUAUAAAG
GAGGAAGCAAUGGAAGUUGGCAACCUGGCGGCAGCGCAAAAGAUGCGUAAAGGAG
AAG
Sequence #26:
   > **RNA P&P *E.coli* GFP-3** 
GGCGUGUGGAGGAGGUUUUCGGGUUGUAAAGUAAUACAUUUAUUUUGCGAUUCG
AUAGGGAGGAAGUACACAUAUCUUCCUCCUUAUUGGAUCGCGAGAUAUCAAUAAG
GAGGAAGAAAUGCAAAUUGCGAACCUGGCGGCAGCGCAAAAGAUGCGUAAAGGAG
AAG
Sequence #27:
   > **Synthetic construct containing *gfpmut3b-ASV* gene**
   proD promoter (in *italics), gfpmut3b-ASV* CDS in grey 
GGCAACAGATCTCACAGCTAACACCACGTCGTCCCTATCTGCTGCCCTAGGTCTATG
AGTGGTTGCTGGATAACTTTACGGGCATGCATAAGGCTCGTATAATATATTCAAGTAA
TACTGGAAGCAATGTGAGCAATGTCGTGCTGTTATAGTTATGAGACAAGAACAGAGG
AGACATAACATGAACAGCACGAACGCTGTTAACCTGGCGGCAGCGCAAAAGATGCG
TAAAGGAGAAGAACTTTTCACTGGAGTTGTCCCAATTCTTGTTGAATTAGATGGTGAT
GTTAATGGGCACAAATTTTCTGTCAGTGGAGAGGGTGAAGGTGATGCAACATACGGA
AAACTTACCCTTAAATTTATTTGCACTACTGGAAAACTACCTGTTCCGTGGCCAACAC
TTGTCACTACTTTCGGTTATGGTGTTCAATGCTTTGCGAGATACCCAGATCACATGAA
ACAGCATGACTTTTTCAAGAGTGCCATGCCCGAAGGTTACGTACAGGAAAGAACTAT
ATTTTTCAAAGATGACGGGAACTACAAGACACGTGCTGAAGTCAAGTTTGAAGGTGA
TACCCTTGTTAATAGAATCGAGTTAAAAGGTATTGATTTTAAAGAAGATGGAAACATTC
TTGGACACAAATTGGAATACAACTATAACTCACACAATGTATACATCATGGCAGACAA
 
ACAAAAGAATGGAATCAAAGTTAACTTCAAAATTAGACACAACATTGAAGATGGAAGC
GTTCAACTAGCAGACCATTATCAACAAAATACTCCGATTGGCGATGGCCCTGTCCTTT
TACCAGACAACCATTACCTGTCCACACAATCTGCCCTTTCGAAAGATCCCAACGAAAA
GAGAGACCACATGGTCCTTCTTGAGTTTGTAACCGCTGCTGGGATTACACATGGCAT
GGATGAACTATACAAAAGGCCTGCAGCAAACGACGAAAACTACGCTGCATCAGTTTA
ATAAGATAAACCAGAGCGGCACGGCAAGCAGAGTATACGAGATTCGGTAGCCACCG
CTGAGCAATAACTAGCATAACCCCTTGGGGCCTCTAAACGGGTCTTGAGGGGTTTTT
TGCATATGCAACGG
Sequence #28:
   > **3' *GFPmut3b-ASVterminator*** (reverse DNA primer)
   CCGTTGCATATGCAAAAAACCCCTCAAGACCCGTTTAGAGGCC
Sequence #29:
   > **5' primer of RNA P&P *Ecoli* GFP-1**
   T7 promoter (in *italics)* 
TCATAAGCTTTAATACGACTCACTATAGGCGTGGCGAGTTTGGTGCGGTCGTGCTGT
GTGGTAAATACGTATAGTACGATCGGAGGGAGGGAGAACAATCGGTTTTCTCCTTTT
GATCGTATTATACGATCAAAAGGAGGAGATAATGTACATTTGCAACCTGGCGGCAGC
GCAAAAGATGCGTAAAGGAGAAG
Sequence #30:
   > **5' primer of RNA P&P *Ecoli* GFP-2**
   T7 promoter (in *italics)* 
TCATAAGCTTTAATACGACTCACTATAGGCGTGTAGCTATGTTGCGTGTATGGGGAG
GGCGAAAATCGCTTTTCTCGTATATAAAGGAGGGAGCACTTAATACTTCCTCCTTTAT
ATACGAGAAGGGTATATAAAGGAGGAAGCAATGGAAGTTGGCAACCTGGCGGCAGC
GCAAAAGATGCGTAAAGGAGAAG
Sequence #31:
   > **5' primer of RNA P&P *Ecoli* GFP-3**
   T7 promoter (in *italics)* 
TCATAAGCTTTAATACGACTCACTATAGGCGTGTGGAGGAGGTTTTCGGGTTGTAAA
GTAATACATTTATTTTGCGATTCGATAGGGAGGAAGTACACATATCTTCCTCCTTATTG
GATCGCGAGATATCAATAAGGAGGAAGAAATGCAAATTGCGAACCTGGCGGCAGCG
CAAAAGATGCGTAAAGGAGAAG
Sequence #32:
   > **PCR product of RNA P&P *Ecoli* GFP-1**
   T7 promoter (in *italics), gfpmut3b-ASV* CDS in grey 
TCATAAGCTTTAATACGACTCACTATAGGCGTGGCGAGTTTGGTGCGGTCGTGCTGT
GTGGTAAATACGTATAGTACGATCGGAGGGAGGGAGAACAATCGGTTTTCTCCTTTT
GATCGTATTATACGATCAAAAGGAGGAGATAATGTACATTTGCAACCTGGCGGCAGC
GCAAAAGATGCGTAAAGGAGAAGAACTTTTCACTGGAGTTGTCCCAATTCTTGTTGAA
TTAGATGGTGATGTTAATGGGCACAAATTTTCTGTCAGTGGAGAGGGTGAAGGTGAT
GCAACATACGGAAAACTTACCCTTAAATTTATTTGCACTACTGGAAAACTACCTGTTC
CGTGGCCAACACTTGTCACTACTTTCGGTTATGGTGTTCAATGCTTTGCGAGATACCC
AGATCACATGAAACAGCATGACTTTTTCAAGAGTGCCATGCCCGAAGGTTACGTACA
GGAAAGAACTATATTTTTCAAAGATGACGGGAACTACAAGACACGTGCTGAAGTCAA
GTTTGAAGGTGATACCCTTGTTAATAGAATCGAGTTAAAAGGTATTGATTTTAAAGAA
GATGGAAACATTCTTGGACACAAATTGGAATACAACTATAACTCACACAATGTATACA
TCATGGCAGACAAACAAAAGAATGGAATCAAAGTTAACTTCAAAATTAGACACAACAT
TGAAGATGGAAGCGTTCAACTAGCAGACCATTATCAACAAAATACTCCGATTGGCGA
TGGCCCTGTCCTTTTACCAGACAACCATTACCTGTCCACACAATCTGCCCTTTCGAAA
GATCCCAACGAAAAGAGAGACCACATGGTCCTTCTTGAGTTTGTAACCGCTGCTGGG
ATTACACATGGCATGGATGAACTATACAAAAGGCCTGCAGCAAACGACGAAAACTAC
GCTGCATCAGTTTAATAATAAGATAAACCAGAGCGGCACGGCAAGCAGAGTATACGA
GATTCGGTAGCCACCGCTGAGCAATAACTAGCATAACCCCTTGGGGCCTCTAAACGG
GTCTTGAGGGGTTTTTTGCATATGCAACGG
Sequence #33:
   > **PCR product of RNA P&P *Ecoli* GFP-2**
   T7 promoter (in *italics), gfpmut3b-ASV* CDS in grey 
TCATAAGCTTTAATACGACTCACTATAGGCGTGTAGCTATGTTGCGTGTATGGGGAG
GGCGAAAATCGCTTTTCTCGTATATAAAGGAGGGAGCACTTAATACTTCCTCCTTTAT
ATACGAGAAGGGTATATAAAGGAGGAAGCAATGGAAGTTGGCAACCTGGCGGCAGC
GCAAAAGATGCGTAAAGGAGAAGAACTTTTCACTGGAGTTGTCCCAATTCTTGTTGAA
TTAGATGGTGATGTTAATGGGCACAAATTTTCTGTCAGTGGAGAGGGTGAAGGTGAT
GCAACATACGGAAAACTTACCCTTAAATTTATTTGCACTACTGGAAAACTACCTGTTC
CGTGGCCAACACTTGTCACTACTTTCGGTTATGGTGTTCAATGCTTTGCGAGATACCC
AGATCACATGAAACAGCATGACTTTTTCAAGAGTGCCATGCCCGAAGGTTACGTACA
GGAAAGAACTATATTTTTCAAAGATGACGGGAACTACAAGACACGTGCTGAAGTCAA
GTTTGAAGGTGATACCCTTGTTAATAGAATCGAGTTAAAAGGTATTGATTTTAAAGAA
GATGGAAACATTCTTGGACACAAATTGGAATACAACTATAACTCACACAATGTATACA
 
TCATGGCAGACAAACAAAAGAATGGAATCAAAGTTAACTTCAAAATTAGACACAACAT
TGAAGATGGAAGCGTTCAACTAGCAGACCATTATCAACAAAATACTCCGATTGGCGA
TGGCCCTGTCCTTTTACCAGACAACCATTACCTGTCCACACAATCTGCCCTTTCGAAA
GATCCCAACGAAAAGAGAGACCACATGGTCCTTCTTGAGTTTGTAACCGCTGCTGGG
ATTACACATGGCATGGATGAACTATACAAAAGGCCTGCAGCAAACGACGAAAACTAC
GCTGCATCAGTTTAATAATAAGATAAACCAGAGCGGCACGGCAAGCAGAGTATACGA
GATTCGGTAGCCACCGCTGAGCAATAACTAGCATAACCCCTTGGGGCCTCTAAACGG
GTCTTGAGGGGTTTTTTGCATATGCAACGG
Sequence #34:
   > **PCR product of RNA P&P *Ecoli* GFP-3**
   T7 promoter (in *italics), gfpmut3b-ASV* CDS in grey 
TCATAAGCTTTAATACGACTCACTATAGGCGTGTGGAGGAGGTTTTCGGGTTGTAAA
GTAATACATTTATTTTGCGATTCGATAGGGAGGAAGTACACATATCTTCCTCCTTATTG
GATCGCGAGATATCAATAAGGAGGAAGAAATGCAAATTGCGAACCTGGCGGCAGCG
CAAAAGATGCGTAAAGGAGAAGAACTTTTCACTGGAGTTGTCCCAATTCTTGTTGAAT
TAGATGGTGATGTTAATGGGCACAAATTTTCTGTCAGTGGAGAGGGTGAAGGTGATG
CAACATACGGAAAACTTACCCTTAAATTTATTTGCACTACTGGAAAACTACCTGTTCC
GTGGCCAACACTTGTCACTACTTTCGGTTATGGTGTTCAATGCTTTGCGAGATACCCA
GATCACATGAAACAGCATGACTTTTTCAAGAGTGCCATGCCCGAAGGTTACGTACAG
GAAAGAACTATATTTTTCAAAGATGACGGGAACTACAAGACACGTGCTGAAGTCAAGT
TTGAAGGTGATACCCTTGTTAATAGAATCGAGTTAAAAGGTATTGATTTTAAAGAAGAT
GGAAACATTCTTGGACACAAATTGGAATACAACTATAACTCACACAATGTATACATCA
TGGCAGACAAACAAAAGAATGGAATCAAAGTTAACTTCAAAATTAGACACAACATTGA
AGATGGAAGCGTTCAACTAGCAGACCATTATCAACAAAATACTCCGATTGGCGATGG
CCCTGTCCTTTTACCAGACAACCATTACCTGTCCACACAATCTGCCCTTTCGAAAGAT
CCCAACGAAAAGAGAGACCACATGGTCCTTCTTGAGTTTGTAACCGCTGCTGGGATT
ACACATGGCATGGATGAACTATACAAAAGGCCTGCAGCAAACGACGAAAACTACGCT
GCATCAGTTTAATAATAAGATAAACCAGAGCGGCACGGCAAGCAGAGTATACGAGAT
TCGGTAGCCACCGCTGAGCAATAACTAGCATAACCCCTTGGGGCCTCTAAACGGGT
CTTGAGGGGTTTTTTGCATATGCAACGG
Sequence #35:
   *>* ***orf1ab* gene** (CDS); MN908947.3:266-21555 Severe acute respiratory syndrome coronavirus 2 isolate Wuhan-Hu-1 (SARS-CoV-2), complete genome 
ATGGAGAGCCTTGTCCCTGGTTTCAACGAGAAAACACACGTCCAACTCAGTTTGCCT
GTTTTACAGGTTCGCGACGTGCTCGTACGTGGCTTTGGAGACTCCGTGGAGGAGGT
 
CTTATCAGAGGCACGTCAACATCTTAAAGATGGCACTTGTGGCTTAGTAGAAGTTGAA
AAAGGCGTTTTGCCTCAACTTGAACAGCCCTATGTGTTCATCAAACGTTCGGATGCTC
GAACTGCACCTCATGGTCATGTTATGGTTGAGCTGGTAGCAGAACTCGAAGGCATTC
AGTACGGTCGTAGTGGTGAGACACTTGGTGTCCTTGTCCCTCATGTGGGCGAAATAC
CAGTGGCTTACCGCAAGGTTCTTCTTCGTAAGAACGGTAATAAAGGAGCTGGTGGCC
ATAGTTACGGCGCCGATCTAAAGTCATTTGACTTAGGCGACGAGCTTGGCACTGATC
CTTATGAAGATTTTCAAGAAAACTGGAACACTAAACATAGCAGTGGTGTTACCCGTGA
ACTCATGCGTGAGCTTAACGGAGGGGCATACACTCGCTATGTCGATAACAACTTCTG
TGGCCCTGATGGCTACCCTCTTGAGTGCATTAAAGACCTTCTAGCACGTGCTGGTAA
AGCTTCATGCACTTTGTCCGAACAACTGGACTTTATTGACACTAAGAGGGGTGTATAC
TGCTGCCGTGAACATGAGCATGAAATTGCTTGGTACACGGAACGTTCTGAAAAGAGC
TATGAATTGCAGACACCTTTTGAAATTAAATTGGCAAAGAAATTTGACACCTTCAATG
GGGAATGTCCAAATTTTGTATTTCCCTTAAATTCCATAATCAAGACTATTCAACCAAGG
GTTGAAAAGAAAAAGCTTGATGGCTTTATGGGTAGAATTCGATCTGTCTATCCAGTTG
CGTCACCAAATGAATGCAACCAAATGTGCCTTTCAACTCTCATGAAGTGTGATCATTG
TGGTGAAACTTCATGGCAGACGGGCGATTTTGTTAAAGCCACTTGCGAATTTTGTGG
CACTGAGAATTTGACTAAAGAAGGTGCCACTACTTGTGGTTACTTACCCCAAAATGCT
GTTGTTAAAATTTATTGTCCAGCATGTCACAATTCAGAAGTAGGACCTGAGCATAGTC
TTGCCGAATACCATAATGAATCTGGCTTGAAAACCATTCTTCGTAAGGGTGGTCGCA
CTATTGCCTTTGGAGGCTGTGTGTTCTCTTATGTTGGTTGCCATAACAAGTGTGCCTA
TTGGGTTCCACGTGCTAGCGCTAACATAGGTTGTAACCATACAGGTGTTGTTGGAGA
AGGTTCCGAAGGTCTTAATGACAACCTTCTTGAAATACTCCAAAAAGAGAAAGTCAAC
ATCAATATTGTTGGTGACTTTAAACTTAATGAAGAGATCGCCATTATTTTGGCATCTTT
TTCTGCTTCCACAAGTGCTTTTGTGGAAACTGTGAAAGGTTTGGATTATAAAGCATTC
AAACAAATTGTTGAATCCTGTGGTAATTTTAAAGTTACAAAAGGAAAAGCTAAAAAAG
GTGCCTGGAATATTGGTGAACAGAAATCAATACTGAGTCCTCTTTATGCATTTGCATC
AGAGGCTGCTCGTGTTGTACGATCAATTTTCTCCCGCACTCTTGAAACTGCTCAAAAT
TCTGTGCGTGTTTTACAGAAGGCCGCTATAACAATACTAGATGGAATTTCACAGTATT
CACTGAGACTCATTGATGCTATGATGTTCACATCTGATTTGGCTACTAACAATCTAGT
TGTAATGGCCTACATTACAGGTGGTGTTGTTCAGTTGACTTCGCAGTGGCTAACTAAC
ATCTTTGGCACTGTTTATGAAAAACTCAAACCCGTCCTTGATTGGCTTGAAGAGAAGT
TTAAGGAAGGTGTAGAGTTTCTTAGAGACGGTTGGGAAATTGTTAAATTTATCTCAAC
CTGTGCTTGTGAAATTGTCGGTGGACAAATTGTCACCTGTGCAAAGGAAATTAAGGA
GAGTGTTCAGACATTCTTTAAGCTTGTAAATAAATTTTTGGCTTTGTGTGCTGACTCTA
TCATTATTGGTGGAGCTAAACTTAAAGCCTTGAATTTAGGTGAAACATTTGTCACGCA
CTCAAAGGGATTGTACAGAAAGTGTGTTAAATCCAGAGAAGAAACTGGCCTACTCAT
GCCTCTAAAAGCCCCAAAAGAAATTATCTTCTTAGAGGGAGAAACACTTCCCACAGAA
 
GTGTTAACAGAGGAAGTTGTCTTGAAAACTGGTGATTTACAACCATTAGAACAACCTA
CTAGTGAAGCTGTTGAAGCTCCATTGGTTGGTACACCAGTTTGTATTAACGGGCTTAT
GTTGCTCGAAATCAAAGACACAGAAAAGTACTGTGCCCTTGCACCTAATATGATGGTA
ACAAACAATACCTTCACACTCAAAGGCGGTGCACCAACAAAGGTTACTTTTGGTGAT
GACACTGTGATAGAAGTGCAAGGTTACAAGAGTGTGAATATCACTTTTGAACTTGATG
AAAGGATTGATAAAGTACTTAATGAGAAGTGCTCTGCCTATACAGTTGAACTCGGTAC
AGAAGTAAATGAGTTCGCCTGTGTTGTGGCAGATGCTGTCATAAAAACTTTGCAACCA
GTATCTGAATTACTTACACCACTGGGCATTGATTTAGATGAGTGGAGTATGGCTACAT
ACTACTTATTTGATGAGTCTGGTGAGTTTAAATTGGCTTCACATATGTATTGTTCTTTC
TACCCTCCAGATGAGGATGAAGAAGAAGGTGATTGTGAAGAAGAAGAGTTTGAGCCA
TCAACTCAATATGAGTATGGTACTGAAGATGATTACCAAGGTAAACCTTTGGAATTTG
GTGCCACTTCTGCTGCTCTTCAACCTGAAGAAGAGCAAGAAGAAGATTGGTTAGATG
ATGATAGTCAACAAACTGTTGGTCAACAAGACGGCAGTGAGGACAATCAGACAACTA
CTATTCAAACAATTGTTGAGGTTCAACCTCAATTAGAGATGGAACTTACACCAGTTGT
TCAGACTATTGAAGTGAATAGTTTTAGTGGTTATTTAAAACTTACTGACAATGTATACA
TTAAAAATGCAGACATTGTGGAAGAAGCTAAAAAGGTAAAACCAACAGTGGTTGTTAA
TGCAGCCAATGTTTACCTTAAACATGGAGGAGGTGTTGCAGGAGCCTTAAATAAGGC
TACTAACAATGCCATGCAAGTTGAATCTGATGATTACATAGCTACTAATGGACCACTT
AAAGTGGGTGGTAGTTGTGTTTTAAGCGGACACAATCTTGCTAAACACTGTCTTCATG
TTGTCGGCCCAAATGTTAACAAAGGTGAAGACATTCAACTTCTTAAGAGTGCTTATGA
AAATTTTAATCAGCACGAAGTTCTACTTGCACCATTATTATCAGCTGGTATTTTTGGTG
CTGACCCTATACATTCTTTAAGAGTTTGTGTAGATACTGTTCGCACAAATGTCTACTTA
GCTGTCTTTGATAAAAATCTCTATGACAAACTTGTTTCAAGCTTTTTGGAAATGAAGAG
TGAAAAGCAAGTTGAACAAAAGATCGCTGAGATTCCTAAAGAGGAAGTTAAGCCATTT
ATAACTGAAAGTAAACCTTCAGTTGAACAGAGAAAACAAGATGATAAGAAAATCAAAG
CTTGTGTTGAAGAAGTTACAACAACTCTGGAAGAAACTAAGTTCCTCACAGAAAACTT
GTTACTTTATATTGACATTAATGGCAATCTTCATCCAGATTCTGCCACTCTTGTTAGTG
ACATTGACATCACTTTCTTAAAGAAAGATGCTCCATATATAGTGGGTGATGTTGTTCA
AGAGGGTGTTTTAACTGCTGTGGTTATACCTACTAAAAAGGCTGGTGGCACTACTGA
AATGCTAGCGAAAGCTTTGAGAAAAGTGCCAACAGACAATTATATAACCACTTACCCG
GGTCAGGGTTTAAATGGTTACACTGTAGAGGAGGCAAAGACAGTGCTTAAAAAGTGT
AAAAGTGCCTTTTACATTCTACCATCTATTATCTCTAATGAGAAGCAAGAAATTCTTGG
AACTGTTTCTTGGAATTTGCGAGAAATGCTTGCACATGCAGAAGAAACACGCAAATTA
ATGCCTGTCTGTGTGGAAACTAAAGCCATAGTTTCAACTATACAGCGTAAATATAAGG
GTATTAAAATACAAGAGGGTGTGGTTGATTATGGTGCTAGATTTTACTTTTACACCAG
TAAAACAACTGTAGCGTCACTTATCAACACACTTAACGATCTAAATGAAACTCTTGTTA
CAATGCCACTTGGCTATGTAACACATGGCTTAAATTTGGAAGAAGCTGCTCGGTATAT
 
GAGATCTCTCAAAGTGCCAGCTACAGTTTCTGTTTCTTCACCTGATGCTGTTACAGCG
TATAATGGTTATCTTACTTCTTCTTCTAAAACACCTGAAGAACATTTTATTGAAACCAT
CTCACTTGCTGGTTCCTATAAAGATTGGTCCTATTCTGGACAATCTACACAACTAGGT
ATAGAATTTCTTAAGAGAGGTGATAAAAGTGTATATTACACTAGTAATCCTACCACATT
CCACCTAGATGGTGAAGTTATCACCTTTGACAATCTTAAGACACTTCTTTCTTTGAGA
GAAGTGAGGACTATTAAGGTGTTTACAACAGTAGACAACATTAACCTCCACACGCAA
GTTGTGGACATGTCAATGACATATGGACAACAGTTTGGTCCAACTTATTTGGATGGAG
CTGATGTTACTAAAATAAAACCTCATAATTCACATGAAGGTAAAACATTTTATGTTTTA
CCTAATGATGACACTCTACGTGTTGAGGCTTTTGAGTACTACCACACAACTGATCCTA
GTTTTCTGGGTAGGTACATGTCAGCATTAAATCACACTAAAAAGTGGAAATACCCACA
AGTTAATGGTTTAACTTCTATTAAATGGGCAGATAACAACTGTTATCTTGCCACTGCAT
TGTTAACACTCCAACAAATAGAGTTGAAGTTTAATCCACCTGCTCTACAAGATGCTTA
TTACAGAGCAAGGGCTGGTGAAGCTGCTAACTTTTGTGCACTTATCTTAGCCTACTGT
AATAAGACAGTAGGTGAGTTAGGTGATGTTAGAGAAACAATGAGTTACTTGTTTCAAC
ATGCCAATTTAGATTCTTGCAAAAGAGTCTTGAACGTGGTGTGTAAAACTTGTGGACA
ACAGCAGACAACCCTTAAGGGTGTAGAAGCTGTTATGTACATGGGCACACTTTCTTAT
GAACAATTTAAGAAAGGTGTTCAGATACCTTGTACGTGTGGTAAACAAGCTACAAAAT
ATCTAGTACAACAGGAGTCACCTTTTGTTATGATGTCAGCACCACCTGCTCAGTATGA
ACTTAAGCATGGTACATTTACTTGTGCTAGTGAGTACACTGGTAATTACCAGTGTGGT
CACTATAAACATATAACTTCTAAAGAAACTTTGTATTGCATAGACGGTGCTTTACTTAC
AAAGTCCTCAGAATACAAAGGTCCTATTACGGATGTTTTCTACAAAGAAAACAGTTAC
ACAACAACCATAAAACCAGTTACTTATAAATTGGATGGTGTTGTTTGTACAGAAATTGA
CCCTAAGTTGGACAATTATTATAAGAAAGACAATTCTTATTTCACAGAGCAACCAATTG
ATCTTGTACCAAACCAACCATATCCAAACGCAAGCTTCGATAATTTTAAGTTTGTATGT
GATAATATCAAATTTGCTGATGATTTAAACCAGTTAACTGGTTATAAGAAACCTGCTTC
AAGAGAGCTTAAAGTTACATTTTTCCCTGACTTAAATGGTGATGTGGTGGCTATTGAT
TATAAACACTACACACCCTCTTTTAAGAAAGGAGCTAAATTGTTACATAAACCTATTGT
TTGGCATGTTAACAATGCAACTAATAAAGCCACGTATAAACCAAATACCTGGTGTATA
CGTTGTCTTTGGAGCACAAAACCAGTTGAAACATCAAATTCGTTTGATGTACTGAAGT
CAGAGGACGCGCAGGGAATGGATAATCTTGCCTGCGAAGATCTAAAACCAGTCTCTG
AAGAAGTAGTGGAAAATCCTACCATACAGAAAGACGTTCTTGAGTGTAATGTGAAAAC
TACCGAAGTTGTAGGAGACATTATACTTAAACCAGCAAATAATAGTTTAAAAATTACAG
AAGAGGTTGGCCACACAGATCTAATGGCTGCTTATGTAGACAATTCTAGTCTTACTAT
TAAGAAACCTAATGAATTATCTAGAGTATTAGGTTTGAAAACCCTTGCTACTCATGGTT
TAGCTGCTGTTAATAGTGTCCCTTGGGATACTATAGCTAATTATGCTAAGCCTTTTCTT
AACAAAGTTGTTAGTACAACTACTAACATAGTTACACGGTGTTTAAACCGTGTTTGTA
CTAATTATATGCCTTATTTCTTTACTTTATTGCTACAATTGTGTACTTTTACTAGAAGTA
 
CAAATTCTAGAATTAAAGCATCTATGCCGACTACTATAGCAAAGAATACTGTTAAGAG
TGTCGGTAAATTTTGTCTAGAGGCTTCATTTAATTATTTGAAGTCACCTAATTTTTCTA
AACTGATAAATATTATAATTTGGTTTTTACTATTAAGTGTTTGCCTAGGTTCTTTAATCT
ACTCAACCGCTGCTTTAGGTGTTTTAATGTCTAATTTAGGCATGCCTTCTTACTGTACT
GGTTACAGAGAAGGCTATTTGAACTCTACTAATGTCACTATTGCAACCTACTGTACTG
GTTCTATACCTTGTAGTGTTTGTCTTAGTGGTTTAGATTCTTTAGACACCTATCCTTCT
TTAGAAACTATACAAATTACCATTTCATCTTTTAAATGGGATTTAACTGCTTTTGGCTTA
GTTGCAGAGTGGTTTTTGGCATATATTCTTTTCACTAGGTTTTTCTATGTACTTGGATT
GGCTGCAATCATGCAATTGTTTTTCAGCTATTTTGCAGTACATTTTATTAGTAATTCTT
GGCTTATGTGGTTAATAATTAATCTTGTACAAATGGCCCCGATTTCAGCTATGGTTAG
AATGTACATCTTCTTTGCATCATTTTATTATGTATGGAAAAGTTATGTGCATGTTGTAG
ACGGTTGTAATTCATCAACTTGTATGATGTGTTACAAACGTAATAGAGCAACAAGAGT
CGAATGTACAACTATTGTTAATGGTGTTAGAAGGTCCTTTTATGTCTATGCTAATGGA
GGTAAAGGCTTTTGCAAACTACACAATTGGAATTGTGTTAATTGTGATACATTCTGTG
CTGGTAGTACATTTATTAGTGATGAAGTTGCGAGAGACTTGTCACTACAGTTTAAAAG
ACCAATAAATCCTACTGACCAGTCTTCTTACATCGTTGATAGTGTTACAGTGAAGAAT
GGTTCCATCCATCTTTACTTTGATAAAGCTGGTCAAAAGACTTATGAAAGACATTCTCT
CTCTCATTTTGTTAACTTAGACAACCTGAGAGCTAATAACACTAAAGGTTCATTGCCTA
TTAATGTTATAGTTTTTGATGGTAAATCAAAATGTGAAGAATCATCTGCAAAATCAGCG
TCTGTTTACTACAGTCAGCTTATGTGTCAACCTATACTGTTACTAGATCAGGCATTAGT
GTCTGATGTTGGTGATAGTGCGGAAGTTGCAGTTAAAATGTTTGATGCTTACGTTAAT
ACGTTTTCATCAACTTTTAACGTACCAATGGAAAAACTCAAAACACTAGTTGCAACTG
CAGAAGCTGAACTTGCAAAGAATGTGTCCTTAGACAATGTCTTATCTACTTTTATTTCA
GCAGCTCGGCAAGGGTTTGTTGATTCAGATGTAGAAACTAAAGATGTTGTTGAATGT
CTTAAATTGTCACATCAATCTGACATAGAAGTTACTGGCGATAGTTGTAATAACTATAT
GCTCACCTATAACAAAGTTGAAAACATGACACCCCGTGACCTTGGTGCTTGTATTGAC
TGTAGTGCGCGTCATATTAATGCGCAGGTAGCAAAAAGTCACAACATTGCTTTGATAT
GGAACGTTAAAGATTTCATGTCATTGTCTGAACAACTACGAAAACAAATACGTAGTGC
TGCTAAAAAGAATAACTTACCTTTTAAGTTGACATGTGCAACTACTAGACAAGTTGTTA
ATGTTGTAACAACAAAGATAGCACTTAAGGGTGGTAAAATTGTTAATAATTGGTTGAA
GCAGTTAATTAAAGTTACACTTGTGTTCCTTTTTGTTGCTGCTATTTTCTATTTAATAAC
ACCTGTTCATGTCATGTCTAAACATACTGACTTTTCAAGTGAAATCATAGGATACAAG
GCTATTGATGGTGGTGTCACTCGTGACATAGCATCTACAGATACTTGTTTTGCTAACA
AACATGCTGATTTTGACACATGGTTTAGCCAGCGTGGTGGTAGTTATACTAATGACAA
AGCTTGCCCATTGATTGCTGCAGTCATAACAAGAGAAGTGGGTTTTGTCGTGCCTGG
TTTGCCTGGCACGATATTACGCACAACTAATGGTGACTTTTTGCATTTCTTACCTAGA
GTTTTTAGTGCAGTTGGTAACATCTGTTACACACCATCAAAACTTATAGAGTACACTG
 
ACTTTGCAACATCAGCTTGTGTTTTGGCTGCTGAATGTACAATTTTTAAAGATGCTTCT
GGTAAGCCAGTACCATATTGTTATGATACCAATGTACTAGAAGGTTCTGTTGCTTATG
AAAGTTTACGCCCTGACACACGTTATGTGCTCATGGATGGCTCTATTATTCAATTTCC
TAACACCTACCTTGAAGGTTCTGTTAGAGTGGTAACAACTTTTGATTCTGAGTACTGT
AGGCACGGCACTTGTGAAAGATCAGAAGCTGGTGTTTGTGTATCTACTAGTGGTAGA
TGGGTACTTAACAATGATTATTACAGATCTTTACCAGGAGTTTTCTGTGGTGTAGATG
CTGTAAATTTACTTACTAATATGTTTACACCACTAATTCAACCTATTGGTGCTTTGGAC
ATATCAGCATCTATAGTAGCTGGTGGTATTGTAGCTATCGTAGTAACATGCCTTGCCT
ACTATTTTATGAGGTTTAGAAGAGCTTTTGGTGAATACAGTCATGTAGTTGCCTTTAAT
ACTTTACTATTCCTTATGTCATTCACTGTACTCTGTTTAACACCAGTTTACTCATTCTTA
CCTGGTGTTTATTCTGTTATTTACTTGTACTTGACATTTTATCTTACTAATGATGTTTCT
TTTTTAGCACATATTCAGTGGATGGTTATGTTCACACCTTTAGTACCTTTCTGGATAAC
AATTGCTTATATCATTTGTATTTCCACAAAGCATTTCTATTGGTTCTTTAGTAATTACCT
AAAGAGACGTGTAGTCTTTAATGGTGTTTCCTTTAGTACTTTTGAAGAAGCTGCGCTG
TGCACCTTTTTGTTAAATAAAGAAATGTATCTAAAGTTGCGTAGTGATGTGCTATTACC
TCTTACGCAATATAATAGATACTTAGCTCTTTATAATAAGTACAAGTATTTTAGTGGAG
CAATGGATACAACTAGCTACAGAGAAGCTGCTTGTTGTCATCTCGCAAAGGCTCTCA
ATGACTTCAGTAACTCAGGTTCTGATGTTCTTTACCAACCACCACAAACCTCTATCAC
CTCAGCTGTTTTGCAGAGTGGTTTTAGAAAAATGGCATTCCCATCTGGTAAAGTTGAG
GGTTGTATGGTACAAGTAACTTGTGGTACAACTACACTTAACGGTCTTTGGCTTGATG
ACGTAGTTTACTGTCCAAGACATGTGATCTGCACCTCTGAAGACATGCTTAACCCTAA
TTATGAAGATTTACTCATTCGTAAGTCTAATCATAATTTCTTGGTACAGGCTGGTAATG
TTCAACTCAGGGTTATTGGACATTCTATGCAAAATTGTGTACTTAAGCTTAAGGTTGAT
ACAGCCAATCCTAAGACACCTAAGTATAAGTTTGTTCGCATTCAACCAGGACAGACTT
TTTCAGTGTTAGCTTGTTACAATGGTTCACCATCTGGTGTTTACCAATGTGCTATGAG
GCCCAATTTCACTATTAAGGGTTCATTCCTTAATGGTTCATGTGGTAGTGTTGGTTTTA
ACATAGATTATGACTGTGTCTCTTTTTGTTACATGCACCATATGGAATTACCAACTGGA
GTTCATGCTGGCACAGACTTAGAAGGTAACTTTTATGGACCTTTTGTTGACAGGCAAA
CAGCACAAGCAGCTGGTACGGACACAACTATTACAGTTAATGTTTTAGCTTGGTTGTA
CGCTGCTGTTATAAATGGAGACAGGTGGTTTCTCAATCGATTTACCACAACTCTTAAT
GACTTTAACCTTGTGGCTATGAAGTACAATTATGAACCTCTAACACAAGACCATGTTG
ACATACTAGGACCTCTTTCTGCTCAAACTGGAATTGCCGTTTTAGATATGTGTGCTTC
ATTAAAAGAATTACTGCAAAATGGTATGAATGGACGTACCATATTGGGTAGTGCTTTA
TTAGAAGATGAATTTACACCTTTTGATGTTGTTAGACAATGCTCAGGTGTTACTTTCCA
AAGTGCAGTGAAAAGAACAATCAAGGGTACACACCACTGGTTGTTACTCACAATTTTG
ACTTCACTTTTAGTTTTAGTCCAGAGTACTCAATGGTCTTTGTTCTTTTTTTTGTATGAA
AATGCCTTTTTACCTTTTGCTATGGGTATTATTGCTATGTCTGCTTTTGCAATGATGTT
 
TGTCAAACATAAGCATGCATTTCTCTGTTTGTTTTTGTTACCTTCTCTTGCCACTGTAG
CTTATTTTAATATGGTCTATATGCCTGCTAGTTGGGTGATGCGTATTATGACATGGTT
GGATATGGTTGATACTAGTTTGTCTGGTTTTAAGCTAAAAGACTGTGTTATGTATGCA
TCAGCTGTAGTGTTACTAATCCTTATGACAGCAAGAACTGTGTATGATGATGGTGCTA
GGAGAGTGTGGACACTTATGAATGTCTTGACACTCGTTTATAAAGTTTATTATGGTAA
TGCTTTAGATCAAGCCATTTCCATGTGGGCTCTTATAATCTCTGTTACTTCTAACTACT
CAGGTGTAGTTACAACTGTCATGTTTTTGGCCAGAGGTATTGTTTTTATGTGTGTTGA
GTATTGCCCTATTTTCTTCATAACTGGTAATACACTTCAGTGTATAATGCTAGTTTATT
GTTTCTTAGGCTATTTTTGTACTTGTTACTTTGGCCTCTTTTGTTTACTCAACCGCTAC
TTTAGACTGACTCTTGGTGTTTATGATTACTTAGTTTCTACACAGGAGTTTAGATATAT
GAATTCACAGGGACTACTCCCACCCAAGAATAGCATAGATGCCTTCAAACTCAACATT
AAATTGTTGGGTGTTGGTGGCAAACCTTGTATCAAAGTAGCCACTGTACAGTCTAAAA
TGTCAGATGTAAAGTGCACATCAGTAGTCTTACTCTCAGTTTTGCAACAACTCAGAGT
AGAATCATCATCTAAATTGTGGGCTCAATGTGTCCAGTTACACAATGACATTCTCTTA
GCTAAAGATACTACTGAAGCCTTTGAAAAAATGGTTTCACTACTTTCTGTTTTGCTTTC
CATGCAGGGTGCTGTAGACATAAACAAGCTTTGTGAAGAAATGCTGGACAACAGGGC
AACCTTACAAGCTATAGCCTCAGAGTTTAGTTCCCTTCCATCATATGCAGCTTTTGCT
ACTGCTCAAGAAGCTTATGAGCAGGCTGTTGCTAATGGTGATTCTGAAGTTGTTCTTA
AAAAGTTGAAGAAGTCTTTGAATGTGGCTAAATCTGAATTTGACCGTGATGCAGCCAT
GCAACGTAAGTTGGAAAAGATGGCTGATCAAGCTATGACCCAAATGTATAAACAGGC
TAGATCTGAGGACAAGAGGGCAAAAGTTACTAGTGCTATGCAGACAATGCTTTTCAC
TATGCTTAGAAAGTTGGATAATGATGCACTCAACAACATTATCAACAATGCAAGAGAT
GGTTGTGTTCCCTTGAACATAATACCTCTTACAACAGCAGCCAAACTAATGGTTGTCA
TACCAGACTATAACACATATAAAAATACGTGTGATGGTACAACATTTACTTATGCATCA
GCATTGTGGGAAATCCAACAGGTTGTAGATGCAGATAGTAAAATTGTTCAACTTAGTG
AAATTAGTATGGACAATTCACCTAATTTAGCATGGCCTCTTATTGTAACAGCTTTAAGG
GCCAATTCTGCTGTCAAATTACAGAATAATGAGCTTAGTCCTGTTGCACTACGACAGA
TGTCTTGTGCTGCCGGTACTACACAAACTGCTTGCACTGATGACAATGCGTTAGCTTA
CTACAACACAACAAAGGGAGGTAGGTTTGTACTTGCACTGTTATCCGATTTACAGGAT
TTGAAATGGGCTAGATTCCCTAAGAGTGATGGAACTGGTACTATCTATACAGAACTG
GAACCACCTTGTAGGTTTGTTACAGACACACCTAAAGGTCCTAAAGTGAAGTATTTAT
ACTTTATTAAAGGATTAAACAACCTAAATAGAGGTATGGTACTTGGTAGTTTAGCTGC
CACAGTACGTCTACAAGCTGGTAATGCAACAGAAGTGCCTGCCAATTCAACTGTATTA
TCTTTCTGTGCTTTTGCTGTAGATGCTGCTAAAGCTTACAAAGATTATCTAGCTAGTG
GGGGACAACCAATCACTAATTGTGTTAAGATGTTGTGTACACACACTGGTACTGGTC
AGGCAATAACAGTTACACCGGAAGCCAATATGGATCAAGAATCCTTTGGTGGTGCAT
CGTGTTGTCTGTACTGCCGTTGCCACATAGATCATCCAAATCCTAAAGGATTTTGTGA
 
CTTAAAAGGTAAGTATGTACAAATACCTACAACTTGTGCTAATGACCCTGTGGGTTTT
ACACTTAAAAACACAGTCTGTACCGTCTGCGGTATGTGGAAAGGTTATGGCTGTAGT
TGTGATCAACTCCGCGAACCCATGCTTCAGTCAGCTGATGCACAATCGTTTTTAAACG
GGTTTGCGGTGTAAGTGCAGCCCGTCTTACACCGTGCGGCACAGGCACTAGTACTG
ATGTCGTATACAGGGCTTTTGACATCTACAATGATAAAGTAGCTGGTTTTGCTAAATT
CCTAAAAACTAATTGTTGTCGCTTCCAAGAAAAGGACGAAGATGACAATTTAATTGAT
TCTTACTTTGTAGTTAAGAGACACACTTTCTCTAACTACCAACATGAAGAAACAATTTA
TAATTTACTTAAGGATTGTCCAGCTGTTGCTAAACATGACTTCTTTAAGTTTAGAATAG
ACGGTGACATGGTACCACATATATCACGTCAACGTCTTACTAAATACACAATGGCAGA
CCTCGTCTATGCTTTAAGGCATTTTGATGAAGGTAATTGTGACACATTAAAAGAAATA
CTTGTCACATACAATTGTTGTGATGATGATTATTTCAATAAAAAGGACTGGTATGATTT
TGTAGAAAACCCAGATATATTACGCGTATACGCCAACTTAGGTGAACGTGTACGCCA
AGCTTTGTTAAAAACAGTACAATTCTGTGATGCCATGCGAAATGCTGGTATTGTTGGT
GTACTGACATTAGATAATCAAGATCTCAATGGTAACTGGTATGATTTCGGTGATTTCA
TACAAACCACGCCAGGTAGTGGAGTTCCTGTTGTAGATTCTTATTATTCATTGTTAAT
GCCTATATTAACCTTGACCAGGGCTTTAACTGCAGAGTCACATGTTGACACTGACTTA
ACAAAGCCTTACATTAAGTGGGATTTGTTAAAATATGACTTCACGGAAGAGAGGTTAA
AACTCTTTGACCGTTATTTTAAATATTGGGATCAGACATACCACCCAAATTGTGTTAAC
TGTTTGGATGACAGATGCATTCTGCATTGTGCAAACTTTAATGTTTTATTCTCTACAGT
GTTCCCACCTACAAGTTTTGGACCACTAGTGAGAAAAATATTTGTTGATGGTGTTCCA
TTTGTAGTTTCAACTGGATACCACTTCAGAGAGCTAGGTGTTGTACATAATCAGGATG
TAAACTTACATAGCTCTAGACTTAGTTTTAAGGAATTACTTGTGTATGCTGCTGACCCT
GCTATGCACGCTGCTTCTGGTAATCTATTACTAGATAAACGCACTACGTGCTTTTCAG
TAGCTGCACTTACTAACAATGTTGCTTTTCAAACTGTCAAACCCGGTAATTTTAACAAA
GACTTCTATGACTTTGCTGTGTCTAAGGGTTTCTTTAAGGAAGGAAGTTCTGTTGAAT
TAAAACACTTCTTCTTTGCTCAGGATGGTAATGCTGCTATCAGCGATTATGACTACTA
TCGTTATAATCTACCAACAATGTGTGATATCAGACAACTACTATTTGTAGTTGAAGTTG
TTGATAAGTACTTTGATTGTTACGATGGTGGCTGTATTAATGCTAACCAAGTCATCGT
CAACAACCTAGACAAATCAGCTGGTTTTCCATTTAATAAATGGGGTAAGGCTAGACTT
TATTATGATTCAATGAGTTATGAGGATCAAGATGCACTTTTCGCATATACAAAACGTAA
TGTCATCCCTACTATAACTCAAATGAATCTTAAGTATGCCATTAGTGCAAAGAATAGA
GCTCGCACCGTAGCTGGTGTCTCTATCTGTAGTACTATGACCAATAGACAGTTTCATC
AAAAATTATTGAAATCAATAGCCGCCACTAGAGGAGCTACTGTAGTAATTGGAACAAG
CAAATTCTATGGTGGTTGGCACAACATGTTAAAAACTGTTTATAGTGATGTAGAAAAC
CCTCACCTTATGGGTTGGGATTATCCTAAATGTGATAGAGCCATGCCTAACATGCTTA
GAATTATGGCCTCACTTGTTCTTGCTCGCAAACATACAACGTGTTGTAGCTTGTCACA
CCGTTTCTATAGATTAGCTAATGAGTGTGCTCAAGTATTGAGTGAAATGGTCATGTGT
 
GGCGGTTCACTATATGTTAAACCAGGTGGAACCTCATCAGGAGATGCCACAACTGCT
TATGCTAATAGTGTTTTTAACATTTGTCAAGCTGTCACGGCCAATGTTAATGCACTTTT
ATCTACTGATGGTAACAAAATTGCCGATAAGTATGTCCGCAATTTACAACACAGACTT
TATGAGTGTCTCTATAGAAATAGAGATGTTGACACAGACTTTGTGAATGAGTTTTACG
CATATTTGCGTAAACATTTCTCAATGATGATACTCTCTGACGATGCTGTTGTGTGTTTC
AATAGCACTTATGCATCTCAAGGTCTAGTGGCTAGCATAAAGAACTTTAAGTCAGTTC
TTTATTATCAAAACAATGTTTTTATGTCTGAAGCAAAATGTTGGACTGAGACTGACCTT
ACTAAAGGACCTCATGAATTTTGCTCTCAACATACAATGCTAGTTAAACAGGGTGATG
ATTATGTGTACCTTCCTTACCCAGATCCATCAAGAATCCTAGGGGCCGGCTGTTTTGT
AGATGATATCGTAAAAACAGATGGTACACTTATGATTGAACGGTTCGTGTCTTTAGCT
ATAGATGCTTACCCACTTACTAAACATCCTAATCAGGAGTATGCTGATGTCTTTCATTT
GTACTTACAATACATAAGAAAGCTACATGATGAGTTAACAGGACACATGTTAGACATG
TATTCTGTTATGCTTACTAATGATAACACTTCAAGGTATTGGGAACCTGAGTTTTATGA
GGCTATGTACACACCGCATACAGTCTTACAGGCTGTTGGGGCTTGTGTTCTTTGCAA
TTCACAGACTTCATTAAGATGTGGTGCTTGCATACGTAGACCATTCTTATGTTGTAAAT
GCTGTTACGACCATGTCATATCAACATCACATAAATTAGTCTTGTCTGTTAATCCGTAT
GTTTGCAATGCTCCAGGTTGTGATGTCACAGATGTGACTCAACTTTACTTAGGAGGTA
TGAGCTATTATTGTAAATCACATAAACCACCCATTAGTTTTCCATTGTGTGCTAATGGA
CAAGTTTTTGGTTTATATAAAAATACATGTGTTGGTAGCGATAATGTTACTGACTTTAA
TGCAATTGCAACATGTGACTGGACAAATGCTGGTGATTACATTTTAGCTAACACCTGT
ACTGAAAGACTCAAGCTTTTTGCAGCAGAAACGCTCAAAGCTACTGAGGAGACATTT
AAACTGTCTTATGGTATTGCTACTGTACGTGAAGTGCTGTCTGACAGAGAATTACATC
TTTCATGGGAAGTTGGTAAACCTAGACCACCACTTAACCGAAATTATGTCTTTACTGG
TTATCGTGTAACTAAAAACAGTAAAGTACAAATAGGAGAGTACACCTTTGAAAAAGGT
GACTATGGTGATGCTGTTGTTTACCGAGGTACAACAACTTACAAATTAAATGTTGGTG
ATTATTTTGTGCTGACATCACATACAGTAATGCCATTAAGTGCACCTACACTAGTGCC
ACAAGAGCACTATGTTAGAATTACTGGCTTATACCCAACACTCAATATCTCAGATGAG
TTTTCTAGCAATGTTGCAAATTATCAAAAGGTTGGTATGCAAAAGTATTCTACACTCCA
GGGACCACCTGGTACTGGTAAGAGTCATTTTGCTATTGGCCTAGCTCTCTACTACCC
TTCTGCTCGCATAGTGTATACAGCTTGCTCTCATGCCGCTGTTGATGCACTATGTGAG
AAGGCATTAAAATATTTGCCTATAGATAAATGTAGTAGAATTATACCTGCACGTGCTC
GTGTAGAGTGTTTTGATAAATTCAAAGTGAATTCAACATTAGAACAGTATGTCTTTTGT
ACTGTAAATGCATTGCCTGAGACGACAGCAGATATAGTTGTCTTTGATGAAATTTCAA
TGGCCACAAATTATGATTTGAGTGTTGTCAATGCCAGATTACGTGCTAAGCACTATGT
GTACATTGGCGACCCTGCTCAATTACCTGCACCACGCACATTGCTAACTAAGGGCAC
ACTAGAACCAGAATATTTCAATTCAGTGTGTAGACTTATGAAAACTATAGGTCCAGAC
ATGTTCCTCGGAACTTGTCGGCGTTGTCCTGCTGAAATTGTTGACACTGTGAGTGCT
 
TTGGTTTATGATAATAAGCTTAAAGCACATAAAGACAAATCAGCTCAATGCTTTAAAAT
GTTTTATAAGGGTGTTATCACGCATGATGTTTCATCTGCAATTAACAGGCCACAAATA
GGCGTGGTAAGAGAATTCCTTACACGTAACCCTGCTTGGAGAAAAGCTGTCTTTATTT
CACCTTATAATTCACAGAATGCTGTAGCCTCAAAGATTTTGGGACTACCAACTCAAAC
TGTTGATTCATCACAGGGCTCAGAATATGACTATGTCATATTCACTCAAACCACTGAA
ACAGCTCACTCTTGTAATGTAAACAGATTTAATGTTGCTATTACCAGAGCAAAAGTAG
GCATACTTTGCATAATGTCTGATAGAGACCTTTATGACAAGTTGCAATTTACAAGTCTT
GAAATTCCACGTAGGAATGTGGCAACTTTACAAGCTGAAAATGTAACAGGACTCTTTA
AAGATTGTAGTAAGGTAATCACTGGGTTACATCCTACACAGGCACCTACACACCTCA
GTGTTGACACTAAATTCAAAACTGAAGGTTTATGTGTTGACATACCTGGCATACCTAA
GGACATGACCTATAGAAGACTCATCTCTATGATGGGTTTTAAAATGAATTATCAAGTT
AATGGTTACCCTAACATGTTTATCACCCGCGAAGAAGCTATAAGACATGTACGTGCAT
GGATTGGCTTCGATGTCGAGGGGTGTCATGCTACTAGAGAAGCTGTTGGTACCAATT
TACCTTTACAGCTAGGTTTTTCTACAGGTGTTAACCTAGTTGCTGTACCTACAGGTTA
TGTTGATACACCTAATAATACAGATTTTTCCAGAGTTAGTGCTAAACCACCGCCTGGA
GATCAATTTAAACACCTCATACCACTTATGTACAAAGGACTTCCTTGGAATGTAGTGC
GTATAAAGATTGTACAAATGTTAAGTGACACACTTAAAAATCTCTCTGACAGAGTCGT
ATTTGTCTTATGGGCACATGGCTTTGAGTTGACATCTATGAAGTATTTTGTGAAAATA
GGACCTGAGCGCACCTGTTGTCTATGTGATAGACGTGCCACATGCTTTTCCACTGCT
TCAGACACTTATGCCTGTTGGCATCATTCTATTGGATTTGATTACGTCTATAATCCGTT
TATGATTGATGTTCAACAATGGGGTTTTACAGGTAACCTACAAAGCAACCATGATCTG
TATTGTCAAGTCCATGGTAATGCACATGTAGCTAGTTGTGATGCAATCATGACTAGGT
GTCTAGCTGTCCACGAGTGCTTTGTTAAGCGTGTTGACTGGACTATTGAATATCCTAT
AATTGGTGATGAACTGAAGATTAATGCGGCTTGTAGAAAGGTTCAACACATGGTTGTT
AAAGCTGCATTATTAGCAGACAAATTCCCAGTTCTTCACGACATTGGTAACCCTAAAG
CTATTAAGTGTGTACCTCAAGCTGATGTAGAATGGAAGTTCTATGATGCACAGCCTTG
TAGTGACAAAGCTTATAAAATAGAAGAATTATTCTATTCTTATGCCACACATTCTGACA
AATTCACAGATGGTGTATGCCTATTTTGGAATTGCAATGTCGATAGATATCCTGCTAA
TTCCATTGTTTGTAGATTTGACACTAGAGTGCTATCTAACCTTAACTTGCCTGGTTGT
GATGGTGGCAGTTTGTATGTAAATAAACATGCATTCCACACACCAGCTTTTGATAAAA
GTGCTTTTGTTAATTTAAAACAATTACCATTTTTCTATTACTCTGACAGTCCATGTGAG
TCTCATGGAAAACAAGTAGTGTCAGATATAGATTATGTACCACTAAAGTCTGCTACGT
GTATAACACGTTGCAATTTAGGTGGTGCTGTCTGTAGACATCATGCTAATGAGTACAG
ATTGTATCTCGATGCTTATAACATGATGATCTCAGCTGGCTTTAGCTTGTGGGTTTAC
AAACAATTTGATACTTATAACCTCTGGAACACTTTTACAAGACTTCAGAGTTTAGAAAA
TGTGGCTTTTAATGTTGTAAATAAGGGACACTTTGATGGACAACAGGGTGAAGTACCA
GTTTCTATCATTAATAACACTGTTTACACAAAAGTTGATGGTGTTGATGTAGAATTGTT
 
TGAAAATAAAACAACATTACCTGTTAATGTAGCATTTGAGCTTTGGGCTAAGCGCAAC
ATTAAACCAGTACCAGAGGTGAAAATACTCAATAATTTGGGTGTGGACATTGCTGCTA
ATACTGTGATCTGGGACTACAAAAGAGATGCTCCAGCACATATATCTACTATTGGTGT
TTGTTCTATGACTGACATAGCCAAGAAACCAACTGAAACGATTTGTGCACCACTCACT
GTCTTTTTTGATGGTAGAGTTGATGGTCAAGTAGACTTATTTAGAAATGCCCGTAATG
GTGTTCTTATTACAGAAGGTAGTGTTAAAGGTTTACAACCATCTGTAGGTCCCAAACA
AGCTAGTCTTAATGGAGTCACATTAATTGGAGAAGCCGTAAAAACACAGTTCAATTAT
TATAAGAAAGTTGATGGTGTTGTCCAACAATTACCTGAAACTTACTTTACTCAGAGTA
GAAATTTACAAGAATTTAAACCCAGGAGTCAAATGGAAATTGATTTCTTAGAATTAGCT
ATGGATGAATTCATTGAACGGTATAAATTAGAAGGCTATGCCTTCGAACATATCGTTT
ATGGAGATTTTAGTCATAGTCAGTTAGGTGGTTTACATCTACTGATTGGACTAGCTAA
ACGTTTTAAGGAATCACCTTTTGAATTAGAAGATTTTATTCCTATGGACAGTACAGTTA
AAAACTATTTCATAACAGATGCGCAAACAGGTTCATCTAAGTGTGTGTGTTCTGTTATT
GATTTATTACTTGATGATTTTGTTGAAATAATAAAATCCCAAGATTTATCTGTAGTTTCT
AAGGTTGTCAAAGTGACTATTGACTATACAGAAATTTCATTTATGCTTTGGTGTAAAGA
TGGCCATGTAGAAACATTTTACCCAAAATTACAATCTAGTCAAGCGTGGCAACCGGG
TGTTGCTATGCCTAATCTTTACAAAATGCAAAGAATGCTATTAGAAAAGTGTGACCTT
CAAAATTATGGTGATAGTGCAACATTACCTAAAGGCATAATGATGAATGTCGCAAAAT
ATACTCAACTGTGTCAATATTTAAACACATTAACATTAGCTGTACCCTATAATATGAGA
GTTATACATTTTGGTGCTGGTTCTGATAAAGGAGTTGCACCAGGTACAGCTGTTTTAA
GACAGTGGTTGCCTACGGGTACGCTGCTTGTCGATTCAGATCTTAATGACTTTGTCT
CTGATGCAGATTCAACTTTGATTGGTGATTGTGCAACTGTACATACAGCTAATAAATG
GGATCTCATTATTAGTGATATGTACGACCCTAAGACTAAAAATGTTACAAAAGAAAAT
GACTCTAAAGAGGGTTTTTTCACTTACATTTGTGGGTTTATACAACAAAAGCTAGCTC
TTGGAGGTTCCGTGGCTATAAAGATAACAGAACATTCTTGGAATGCTGATCTTTATAA
GCTCATGGGACACTTCGCATGGTGGACAGCCTTTGTTACTAATGTGAATGCGTCATC
ATCTGAAGCATTTTTAATTGGATGTAATTATCTTGGCAAACCACGCGAACAAATAGAT
GGTTATGTCATGCATGCAAATTACATATTTTGGAGGAATACAAATCCAATTCAGTTGT
CTTCCTATTCTTTATTTGACATGAGTAAATTTCCCCTTAAATTAAGGGGTACTGCTGTT
ATGTCTTTAAAAGAAGGTCAAATCAATGATATGATTTTATCTCTTCTTAGTAAAGGTAG
ACTTATAATTAGAGAAAACAACAGAGTTGTTATTTCTAGTGATGTTCTTGTTAACAACT
AA
Sequence #36:
   > **RNA P&P SARS-CoV-2 *lacZ-1*** 
GGCGCCGUCUUGUUGACCAACAGUUUGUUGACUAAAUAACGGUUAACAAGCUGUU
UCAGAGAGGACGCAAAUAAAGUCCUCUUUGGAGUAGUUUGUUGAUUUACUUUAAG
GAGGGCAAAAUGGGAAGCUUCGAUCCCGCCGUCGUUUUACAACG
Sequence #37:
   > **RNA P&P SARS-CoV-2 *lacZ-2*** 
GGCGCCGUCUUGUUGACCAACAGUUUGUUGACUAAAUAACGGUUAACAAGCUGUU
UCAGAGAGGACGCAAAUAAAGUCCUCUUUGGAGUAGUUUGUUGAUUUACUUUAAG
GAGGGCAAAAUGGGAAGCUUCGAUCCCGCCGUCGUUUUACAACG
Sequence #38:
   > **Synthetic RNA target of SARS-CoV-2** (*orf1ab* gene, nt position 3215-3244)
   AGUCAACAAACUGUUGGUCAACAAGACGGC
Sequence #39:
   **> 5' primer of RNA P&P SARS-CoV-2 /*acZ-1***
   T7 promoter (in *italics)* 
TCATAAGCTTTAATACGACTCACTATAGGCGCCGTCTTGTTGACCAACAGTTTGTTGA
CTAAATAACGGTTAACAAGCTGTTTCAGAGAGGACGCAAATAAAGTCCTCTTTGGAGT
AGTTTGTTGATTTACTTTAAGGAGGGCAAAATGGGAAGCTTCGATCCCGCCGTCGTT
TTACAACG
Sequence #40:
   > **5' primer of RNA P&P SARS-CoV-2 *lacZ-2***
   T7 promoter (in *italics)* 
TCATAAGCTTTAATACGACTCACTATAGGCGCCGTCTTGTTGACCAACAGTTTGTTGA
CTAAATAACGGTTAACAAGCTGTTTCAAAGAGGACGCAAATAAAGTCCTCTTTGGAGT
AGTTTGTTGATTTACTTTAAGGAGGGCAAAATGGGAAGCTTCGATCCCGCCGTCGTT
TTACAACG
Sequence #41:
   > **PCR product of RNA P&P *lacZ-1***
   T7 promoter (in *italics),* YEp358 vector underlined, *lacZ* CDS in grey 
TCATAAGCTTTAATACGACTCACTATAGGCGCCGTCTTGTTGACCAACAGTTTGTTGA
CTAAATAACGGTTAACAAGCTGTTTCAGAGAGGACGCAAATAAAGTCCTCTTTGGAGT
 
AGTTTGTTGATTTACTTTAAGGAGGGCAAAATGGGAAGCTTCGATCCCGCCGTCGTT
TTACAACGTCGTGACTGGGAAAACCCTGGCGTTACCCAACTTAATCGCCTTGCAGCA
CATCCCCCTTTCGCCAGCTGGCGTAATAGCGAAGAGGCCCGCACCGATCGCCCTTC
CCAACAGTTGCGCAGCCTGAATGGCGAATGGCGCTTTGCCTGGTTTCCGGCACCAG
AAGCGGTGCCGGAAAGCTGGCTGGAGTGCGATCTTCCTGAGGCCGATACTGTCGTC
GTCCCCTCAAACTGGCAGATGCACGGTTACGATGCGCCCATCTACACCAACGTGAC
CTATCCCATTACGGTCAATCCGCCGTTTGTTCCCACGGAGAATCCGACGGGTTGTTA
CTCGCTCACATTTAATGTTGATGAAAGCTGGCTACAGGAAGGCCAGACGCGAATTAT
TTTTGATGGCGTTAACTCGGCGTTTCATCTGTGGTGCAACGGGCGCTGGGTCGGTTA
CGGCCAGGACAGTCGTTTGCCGTCTGAATTTGACCTGAGCGCATTTTTACGCGCCG
GAGAAAACCGCCTCGCGGTGATGGTGCTGCGCTGGAGTGACGGCAGTTATCTGGAA
GATCAGGATATGTGGCGGATGAGCGGCATTTTCCGTGACGTCTCGTTGCTGCATAAA
CCGACTACACAAATCAGCGATTTCCATGTTGCCACTCGCTTTAATGATGATTTCAGCC
GCGCTGTACTGGAGGCTGAAGTTCAGATGTGCGGCGAGTTGCGTGACTACCTACGG
GTAACAGTTTCTTTATGGCAGGGTGAAACGCAGGTCGCCAGCGGCACCGCGCCTTT
CGGCGGTGAAATTATCGATGAGCGTGGTGGTTATGCCGATCGCGTCACACTACGTCT
GAACGTCGAAAACCCGAAACTGTGGAGCGCCGAAATCCCGAATCTCTATCGTGCGG
TGGTTGAACTGCACACCGCCGACGGCACGCTGATTGAAGCAGAAGCCTGCGATGTC
GGTTTCCGCGAGGTGCGGATTGAAAATGGTCTGCTGCTGCTGAACGGCAAGCCGTT
GCTGATTCGAGGCGTTAACCGTCACGAGCATCATCCTCTGCATGGTCAGGTCATGGA
TGAGCAGACGATGGTGCAGGATATCCTGCTGATGAAGCAGAACAACTTTAACGCCGT
GCGCTGTTCGCATTATCCGAACCATCCGCTGTGGTACACGCTGTGCGACCGCTACG
GCCTGTATGTGGTGGATGAAGCCAATATTGAAACCCACGGCATGGTGCCAATGAATC
GTCTGACCGATGATCCGCGCTGGCTACCGGCGATGAGCGAACGCGTAACGCGAATG
GTGCAGCGCGATCGTAATCACCCGAGTGTGATCATCTGGTCGCTGGGGAATGAATC
AGGCCACGGCGCTAATCACGACGCGCTGTATCGCTGGATCAAATCTGTCGATCCTTC
CCGCCCGGTGCAGTATGAAGGCGGCGGAGCCGACACCACGGCCACCGATATTATTT
GCCCGATGTACGCGCGCGTGGATGAAGACCAGCCCTTCCCGGCTGTGCCGAAATG
GTCCATCAAAAAATGGCTTTCGCTACCTGGAGAGACGCGCCCGCTGATCCTTTGCGA
ATACGCCCACGCGATGGGTAACAGTCTTGGCGGTTTCGCTAAATACTGGCAGGCGTT
TCGTCAGTATCCCCGTTTACAGGGCGGCTTCGTCTGGGACTGGGTGGATCAGTCGC
TGATTAAATATGATGAAAACGGCAACCCGTGGTCGGCTTACGGCGGTGATTTTGGCG
ATACGCCGAACGATCGCCAGTTCTGTATGAACGGTCTGGTCTTTGCCGACCGCACG
CCGCATCCAGCGCTGACGGAAGCAAAACACCAGCAGCAGTTTTTCCAGTTCCGTTTA
TCCGGGCAAACCATCGAAGTGACCAGCGAATACCTGTTCCGTCATAGCGATAACGAG
CTCCTGCACTGGATGGTGGCGCTGGATGGTAAGCCGCTGGCAAGCGGTGAAGTGC
CTCTGGATGTCGCTCCACAAGGTAAACAGTTGATTGAACTGCCTGAACTACCGCAGC
 
CGGAGAGCGCCGGGCAACTCTGGCTCACAGTACGCGTAGTGCAACCGAACGCGAC
CGCATGGTCAGAAGCCGGGCACATCAGCGCCTGGCAGCAGTGGCGTCTGGCGGAA
AACCTCAGTGTGACGCTCCCCGCCGCGTCCCACGCCATCCCGCATCTGACCACCAG
CGAAATGGATTTTTGCATCGAGCTGGGTAATAAGCGTTGGCAATTTAACCGCCAGTC
AGGCTTTCTTTCACAGATGTGGATTGGCGATAAAAAACAACTGCTGACGCCGCTGCG
CGATCAGTTCACCCGTGCACCGCTGGATAACGACATTGGCGTAAGTGAAGCGACCC
GCATTGACCCTAACGCCTGGGTCGAACGCTGGAAGGCGGCGGGCCATTACCAGGC
CGAAGCAGCGTTGTTGCAGTGCACGGCAGATACACTTGCTGATGCGGTGCTGATTA
CGACCGCTCACGCGTGGCAGCATCAGGGGAAAACCTTATTTATCAGCCGGAAAACC
TACCGGATTGATGGTAGTGGTCAAATGGCGATTACCGTTGATGTTGAAGTGGCGAGC
GATACACCGCATCCGGCGCGGATTGGCCTGAACTGCCAGCTGGCGCAGGTAGCAG
AGCGGGTAAACTGGCTCGGATTAGGGCCGCAAGAAAACTATCCCGACCGCCTTACT
GCCGCCTGTTTTGACCGCTGGGATCTGCCATTGTCAGACATGTATACCCCGTACGTC
TTCCCGAGCGAAAACGGTCTGCGCTGCGGGACGCGCGAATTGAATTATGGCCCACA
CCAGTGGCGCGGCGACTTCCAGTTCAACATCAGCCGCTACAGTCAACAGCAACTGA
TGGAAACCAGCCATCGCCATCTGCTGCACGCGGAAGAAGGCACATGGCTGAATATC
GACGGTTTCCATATGGGGATTGGTGGCGACGACTCCTGGAGCCCGTCAGTATCGGC
GGAATTCCAGCTGAGCGCCGGTCGCTACCATTACCAGTTGGTCTGGTGTCAAAAATA
ACTAGCATAACCCCTCTCTAAACGGAGGGGTTT
Sequence #42:
   > **PCR product of RNA P&P *lacZ-2***
   T7 promoter (in *italics),* YEp358 vector underlined, *lacZ* CDS in grey 
TCATAAGCTTTAATACGACTCACTATAGGCGCCGTCTTGTTGACCAACAGTTTGTTGA
CTAAATAACGGTTAACAAGCTGTTTCAAAGAGGACGCAAATAAAGTCCTCTTTGGAGT
AGTTTGTTGATTTACTTTAAGGAGGGCAAAATGGGAAGCTTCGATCCCGCCGTCGTT
TTACAACGTCGTGACTGGGAAAACCCTGGCGTTACCCAACTTAATCGCCTTGCAGCA
CATCCCCCTTTCGCCAGCTGGCGTAATAGCGAAGAGGCCCGCACCGATCGCCCTTC
CCAACAGTTGCGCAGCCTGAATGGCGAATGGCGCTTTGCCTGGTTTCCGGCACCAG
AAGCGGTGCCGGAAAGCTGGCTGGAGTGCGATCTTCCTGAGGCCGATACTGTCGTC
GTCCCCTCAAACTGGCAGATGCACGGTTACGATGCGCCCATCTACACCAACGTGAC
CTATCCCATTACGGTCAATCCGCCGTTTGTTCCCACGGAGAATCCGACGGGTTGTTA
CTCGCTCACATTTAATGTTGATGAAAGCTGGCTACAGGAAGGCCAGACGCGAATTAT
TTTTGATGGCGTTAACTCGGCGTTTCATCTGTGGTGCAACGGGCGCTGGGTCGGTTA
CGGCCAGGACAGTCGTTTGCCGTCTGAATTTGACCTGAGCGCATTTTTACGCGCCG
GAGAAAACCGCCTCGCGGTGATGGTGCTGCGCTGGAGTGACGGCAGTTATCTGGAA
GATCAGGATATGTGGCGGATGAGCGGCATTTTCCGTGACGTCTCGTTGCTGCATAAA
 
CCGACTACACAAATCAGCGATTTCCATGTTGCCACTCGCTTTAATGATGATTTCAGCC
GCGCTGTACTGGAGGCTGAAGTTCAGATGTGCGGCGAGTTGCGTGACTACCTACGG
GTAACAGTTTCTTTATGGCAGGGTGAAACGCAGGTCGCCAGCGGCACCGCGCCTTT
CGGCGGTGAAATTATCGATGAGCGTGGTGGTTATGCCGATCGCGTCACACTACGTCT
GAACGTCGAAAACCCGAAACTGTGGAGCGCCGAAATCCCGAATCTCTATCGTGCGG
TGGTTGAACTGCACACCGCCGACGGCACGCTGATTGAAGCAGAAGCCTGCGATGTC
GGTTTCCGCGAGGTGCGGATTGAAAATGGTCTGCTGCTGCTGAACGGCAAGCCGTT
GCTGATTCGAGGCGTTAACCGTCACGAGCATCATCCTCTGCATGGTCAGGTCATGGA
TGAGCAGACGATGGTGCAGGATATCCTGCTGATGAAGCAGAACAACTTTAACGCCGT
GCGCTGTTCGCATTATCCGAACCATCCGCTGTGGTACACGCTGTGCGACCGCTACG
GCCTGTATGTGGTGGATGAAGCCAATATTGAAACCCACGGCATGGTGCCAATGAATC
GTCTGACCGATGATCCGCGCTGGCTACCGGCGATGAGCGAACGCGTAACGCGAATG
GTGCAGCGCGATCGTAATCACCCGAGTGTGATCATCTGGTCGCTGGGGAATGAATC
AGGCCACGGCGCTAATCACGACGCGCTGTATCGCTGGATCAAATCTGTCGATCCTTC
CCGCCCGGTGCAGTATGAAGGCGGCGGAGCCGACACCACGGCCACCGATATTATTT
GCCCGATGTACGCGCGCGTGGATGAAGACCAGCCCTTCCCGGCTGTGCCGAAATG
GTCCATCAAAAAATGGCTTTCGCTACCTGGAGAGACGCGCCCGCTGATCCTTTGCGA
ATACGCCCACGCGATGGGTAACAGTCTTGGCGGTTTCGCTAAATACTGGCAGGCGTT
TCGTCAGTATCCCCGTTTACAGGGCGGCTTCGTCTGGGACTGGGTGGATCAGTCGC
TGATTAAATATGATGAAAACGGCAACCCGTGGTCGGCTTACGGCGGTGATTTTGGCG
ATACGCCGAACGATCGCCAGTTCTGTATGAACGGTCTGGTCTTTGCCGACCGCACG
CCGCATCCAGCGCTGACGGAAGCAAAACACCAGCAGCAGTTTTTCCAGTTCCGTTTA
TCCGGGCAAACCATCGAAGTGACCAGCGAATACCTGTTCCGTCATAGCGATAACGAG
CTCCTGCACTGGATGGTGGCGCTGGATGGTAAGCCGCTGGCAAGCGGTGAAGTGC
CTCTGGATGTCGCTCCACAAGGTAAACAGTTGATTGAACTGCCTGAACTACCGCAGC
CGGAGAGCGCCGGGCAACTCTGGCTCACAGTACGCGTAGTGCAACCGAACGCGAC
CGCATGGTCAGAAGCCGGGCACATCAGCGCCTGGCAGCAGTGGCGTCTGGCGGAA
AACCTCAGTGTGACGCTCCCCGCCGCGTCCCACGCCATCCCGCATCTGACCACCAG
CGAAATGGATTTTTGCATCGAGCTGGGTAATAAGCGTTGGCAATTTAACCGCCAGTC
AGGCTTTCTTTCACAGATGTGGATTGGCGATAAAAAACAACTGCTGACGCCGCTGCG
CGATCAGTTCACCCGTGCACCGCTGGATAACGACATTGGCGTAAGTGAAGCGACCC
GCATTGACCCTAACGCCTGGGTCGAACGCTGGAAGGCGGCGGGCCATTACCAGGC
CGAAGCAGCGTTGTTGCAGTGCACGGCAGATACACTTGCTGATGCGGTGCTGATTA
CGACCGCTCACGCGTGGCAGCATCAGGGGAAAACCTTATTTATCAGCCGGAAAACC
TACCGGATTGATGGTAGTGGTCAAATGGCGATTACCGTTGATGTTGAAGTGGCGAGC
GATACACCGCATCCGGCGCGGATTGGCCTGAACTGCCAGCTGGCGCAGGTAGCAG
AGCGGGTAAACTGGCTCGGATTAGGGCCGCAAGAAAACTATCCCGACCGCCTTACT
 
GCCGCCTGTTTTGACCGCTGGGATCTGCCATTGTCAGACATGTATACCCCGTACGTC
TTCCCGAGCGAAAACGGTCTGCGCTGCGGGACGCGCGAATTGAATTATGGCCCACA
CCAGTGGCGCGGCGACTTCCAGTTCAACATCAGCCGCTACAGTCAACAGCAACTGA
TGGAAACCAGCCATCGCCATCTGCTGCACGCGGAAGAAGGCACATGGCTGAATATC
GACGGTTTCCATATGGGGATTGGTGGCGACGACTCCTGGAGCCCGTCAGTATCGGC
GGAATTCCAGCTGAGCGCCGGTCGCTACCATTACCAGTTGGTCTGGTGTCAAAAATA
ACTAGCATAACCCCTCTCTAAACGGAGGGGTTT
Sequence #43:
   *>* ***orf8* gene** (CDS); MN908947.3:27894-28259 Severe acute respiratory syndrome coronavirus 2 isolate Wuhan-Hu-1 (SARS-CoV-2), complete genome 
ATGAAATTTCTTGTTTTCTTAGGAATCATCACAACTGTAGCTGCATTTCACCAAGAATG
TAGTTTACAGTCATGTACTCAACATCAACCATATGTAGTTGATGACCCGTGTCCTATT
CACTTCTATTCTAAATGGTATATTAGAGTAGGAGCTAGAAAATCAGCACCTTTAATTGA
ATTGTGCGTGGATGAGGCTGGTTCTAAATCACCCATTCAGTACATCGATATCGGTAAT
TATACAGTTTCCTGTTTACCTTTTACAATTAATTGCCAGGAACCTAAATTGGGTAGTCT
TGTAGTGCGTTGTTCGTTCTATGAAGACTTTTTAGAGTATCATGACGTTCGTGTTGTTT
TAGATTTCATCTAA
Sequence #44:
   > **RNA P&P SARS-CoV-2 GFP-1** 
GGCGGUUAAUGGGUUGUUGGUUAGCGAGGCGGCAUAAAUCGCCGUCUUGCUGGC
CUAGGGAGGCGGCCAGGUACAUCGCCUCCUUAGGUUGGUAAGACGGUAACUUAA
GGAGGCGAAAAUGCAGAACCUGGCGGCAGCGCAAAAGAUGCGUAAA
Sequence #45:
   > **RNA P&P SARS-CoV-2 GFP-2** 
GGCCCUAUUCACUUUAUUCUAAAUGGUAUAUUUAAGACUGGUAUACCGUUUAGAU
AAGGAGGGCACCGGGGUAUUGUCCUCCUUGUUUGGACGGUAUAUCCGAAUAAGG
AGGACAAGAUGCAAAACCUGGCGGCAGCGCAAAAGAUGCGUAAA
Sequence #46:
   > **Synthetic RNA target of SARS-CoV-2 GFP-1** (*orf1ab* gene, nt position 3215-3244, reverse complement)
   GCCGUCUUGUUGACCAACAGUUUGUUGACU
Sequence #47:
   > **Synthetic RNA target of SARS-CoV-2 GFP-2** (*orf8* gene, nt position 28005-28034, reverse complement)
   AAUAUACCAUUUAGAAUAGAAGUGAAUAGG
Sequence #48:
   > **5' primer of RNA P&P SARS-CoV-2 GFP-1**
   T7 promoter in *italics*) 
TCATAAGCTTTAATACGACTCACTATAGGCGGTTAATGGGTTGTTGGTTAGCGAGGC
GGCATAAATCGCCGTCTTGCTGGCCTAGGGAGGCGGCCAGGTACATCGCCTCCTTA
GGTTGGTAAGACGGTAACTTAAGGAGGCGAAAATGCAGAACCTGGCGGCAGCGCAA
AAGATGCGTAAA
Sequence #49:
   > **5' primer of RNA P&P SARS-CoV-2 GFP-2**
   T7 promoter (in *italics)* 
TCATAAGCTTTAATACGACTCACTATAGGCCCTATTCACTTCTATTCTAAATGGTATAT
TTAAGACTGGTATACCGTTTAGATAAGGAGGGCACCGGGGTATTGTCCTCCTTGTTT
GGACGGTATATCCGAATAAGGAGGACAAGATGCAAAACCTGGCGGCAGCGCAAAAG
ATGCGTAAA
Sequence #50:
   > **PCR product of RNA P&P SARS-CoV-2 GFP-1**
   T7 promoter (in *italics), gfpmut3b-ASV* CDS in grey 
TCATAAGCTTTAATACGACTCACTATAGGCGGTTAATGGGTTGTTGGTTAGCGAGGC
GGCATAAATCGCCGTCTTGCTGGCCTAGGGAGGCGGCCAGGTACATCGCCTCCTTA
GGTTGGTAAGACGGTAACTTAAGGAGGCGAAAATGCAGAACCTGGCGGCAGCGCAA
AAGATGCGTAAAGGAGAAGAACTTTTCACTGGAGTTGTCCCAATTCTTGTTGAATTAG
ATGGTGATGTTAATGGGCACAAATTTTCTGTCAGTGGAGAGGGTGAAGGTGATGCAA
CATACGGAAAACTTACCCTTAAATTTATTTGCACTACTGGAAAACTACCTGTTCCGTG
GCCAACACTTGTCACTACTTTCGGTTATGGTGTTCAATGCTTTGCGAGATACCCAGAT
CACATGAAACAGCATGACTTTTTCAAGAGTGCCATGCCCGAAGGTTACGTACAGGAA
AGAACTATATTTTTCAAAGATGACGGGAACTACAAGACACGTGCTGAAGTCAAGTTTG
AAGGTGATACCCTTGTTAATAGAATCGAGTTAAAAGGTATTGATTTTAAAGAAGATGG
AAACATTCTTGGACACAAATTGGAATACAACTATAACTCACACAATGTATACATCATG
GCAGACAAACAAAAGAATGGAATCAAAGTTAACTTCAAAATTAGACACAACATTGAAG
ATGGAAGCGTTCAACTAGCAGACCATTATCAACAAAATACTCCGATTGGCGATGGCC
CTGTCCTTTTACCAGACAACCATTACCTGTCCACACAATCTGCCCTTTCGAAAGATCC
 
CAACGAAAAGAGAGACCACATGGTCCTTCTTGAGTTTGTAACCGCTGCTGGGATTAC
ACATGGCATGGATGAACTATACAAAAGGCCTGCAGCAAACGACGAAAACTACGCTGC
ATCAGTTTAATAATAAGATAAACCAGAGCGGCACGGCAAGCAGAGTATACGAGATTC
GGTAGCCACCGCTGAGCAATAACTAGCATAACCCCTTGGGGCCTCTAAACGGGTCTT
GAGGGGTTTTTTGCATATGCAACGG
Sequence #51:
   > **PCR product of RNA P&P SARS-CoV-2 GFP-2**
   T7 promoter (in *italics), gfpmut3b-ASV* CDS in grey 
TCATAAGCTTTAATACGACTCACTATAGGCAGTTAAGTTATTTATGGCTGAGAGTGGA
CTGTAAATAAGTCCGCTCTCAGTCGAAGGAGGCGTCTTTTGCAAACGCCTCCTTTGG
TTGAGAGCGGATTGACTAAAGGAGGCGTATATGTCGAACCTGGCGGCAGCGCAAAA
GATGCGTAAAGGAGAAGAACTTTTCACTGGAGTTGTCCCAATTCTTGTTGAATTAGAT
GGTGATGTTAATGGGCACAAATTTTCTGTCAGTGGAGAGGGTGAAGGTGATGCAACA
TACGGAAAACTTACCCTTAAATTTATTTGCACTACTGGAAAACTACCTGTTCCGTGGC
CAACACTTGTCACTACTTTCGGTTATGGTGTTCAATGCTTTGCGAGATACCCAGATCA
CATGAAACAGCATGACTTTTTCAAGAGTGCCATGCCCGAAGGTTACGTACAGGAAAG
AACTATATTTTTCAAAGATGACGGGAACTACAAGACACGTGCTGAAGTCAAGTTTGAA
GGTGATACCCTTGTTAATAGAATCGAGTTAAAAGGTATTGATTTTAAAGAAGATGGAA
ACATTCTTGGACACAAATTGGAATACAACTATAACTCACACAATGTATACATCATGGC
AGACAAACAAAAGAATGGAATCAAAGTTAACTTCAAAATTAGACACAACATTGAAGAT
GGAAGCGTTCAACTAGCAGACCATTATCAACAAAATACTCCGATTGGCGATGGCCCT
GTCCTTTTACCAGACAACCATTACCTGTCCACACAATCTGCCCTTTCGAAAGATCCCA
ACGAAAAGAGAGACCACATGGTCCTTCTTGAGTTTGTAACCGCTGCTGGGATTACAC
ATGGCATGGATGAACTATACAAAAGGCCTGCAGCAAACGACGAAAACTACGCTGCAT
CAGTTTAATAATAAGATAAACCAGAGCGGCACGGCAAGCAGAGTATACGAGATTCGG
TAGCCACCGCTGAGCAATAACTAGCATAACCCCTTGGGGCCTCTAAACGGGTCTTGA
GGGGTTTTTTGCATATGCAACGG
Sequence #52:
   **> MK176509.1 Synthetic construct near-infrared fluorescent protein *miRFP670nano* gene,** CDS 
ATGGCAAACCTGGACAAGATGCTGAATACCACAGTAACAGAGGTGCGGCAGTTCCT
GCAGGTGGACAGAGTGTGCGTGTTCCAGTTTGAGGAGGATTATAGCGGAGTGGTGG
TGGTGGAGGCCGTGGACGATAGGTGGATCTCCATCCTGAAGACCCAGGTGCGGGAT
AGATACTTCATGGAGACAAGGGGCGAGGAGTATTCTCACGGCCGCTACCAGGCCAT
CGCCGACATCTACACCGCAAACCTGACAGAGTGCTACAGGGATCTGCTGACACAGTT
TCAGGTGAGAGCAATCCTGGCCGTGCCCATCCTGCAGGGCAAGAAGCTGTGGGGC
 
CTGTTGGTGGCACACCAGCTGGCGGCCCCTAGACAGTGGCAGACCTGGGAGATCG
ACTTTCTGAAGCAGCAGGCCGTGGTGGTGGGCATCGCCATCCAGCAGAGCTAA
Sequence #53:
   > RNA P&P SARS-CoV-2 miRFP/GFP-1
   *miRFP670nano* gene CDS underlined; 
GGCGGUUGAUGGGUUGUUGGUCAACAGGGCGGCGAUAAUCGCCGUUCUGUUGGC
UAGCAAGGAGGAAAAUAUGGCAAACCUGGACAAGAUGCUGAAUACCACAGUAACAG
AGGUGCGGCAGUUCCUGCAGGUGGACAGAGUGUGCGUGUUCCAGUUUGAGGAGG
AUUAUAGCGGAGUGGUGGUGGUGGAGGCCGUGGACGAUAGGUGGAUCUCCAUCC
UGAAGACCCAGGUGCGGGAUAGAUACUUCAUGGAGACAAGGGGCGAGGAGUAUU
CUCACGGCCGCUACCAGGCCAUCGCCGACAUCUACACCGCAAACCUGACAGAGUG
CUACAGGGAUCUGCUGACACAGUUUCAGGUGAGAGCAAUCCUGGCCGUGCCCAUC
CUGCAGGGCAAGAAGCUGUGGGGCCUGUUGGUGGCACACCAGCUGGCGGCCCCU
AGACAGUGGCAGACCUGGGAGAUCGACUUUCUGAAGCAGCAGGCCGUGGUGGUG
GGCAUCGCCAUCCAGCAGAGCUAAUUUCCUCCUUGUUAGCCAAUAGAAUGGUCUA
ACAAGGAGGAAACAAUGUAUACAACAAACCUGGCGGCAGCGCAAAAGAUG
Sequence #54:
   > RNA P&P SARS-CoV-2 miRFP/GFP-2
   *miRFP670nano* gene CDS underlined; 
GGCGGUUGAUGGGUUGUUGGUCAACAGGGCGGCGAUAAUUGCCGUUCUGUUGGC
UAGCAAGGAGGAAAAUAUGGCAAACCUGGACAAGAUGCUGAAUACCACAGUAACAG
AGGUGCGGCAGUUCCUGCAGGUGGACAGAGUGUGCGUGUUCCAGUUUGAGGAGG
AUUAUAGCGGAGUGGUGGUGGUGGAGGCCGUGGACGAUAGGUGGAUCUCCAUCC
UGAAGACCCAGGUGCGGGAUAGAUACUUCAUGGAGACAAGGGGCGAGGAGUAUU
CUCACGGCCGCUACCAGGCCAUCGCCGACAUCUACACCGCAAACCUGACAGAGUG
CUACAGGGAUCUGCUGACACAGUUUCAGGUGAGAGCAAUCCUGGCCGUGCCCAUC
CUGCAGGGCAAGAAGCUGUGGGGCCUGUUGGUGGCACACCAGCUGGCGGCCCCU
AGACAGUGGCAGACCUGGGAGAUCGACUUUCUGAAGCAGCAGGCCGUGGUGGUG
GGCAUCGCCAUCCAGCAGAGCUAAUUUCCUCCUUGUUAGCCAAUAGAAUGGUCUA
ACAAGGAGGAAACAAUGUAUACAACAAACCUGGCGGCAGCGCAAAAGAUG
Sequence #55:
   > **5' primer #1 for RNA P&P SARS-CoV-2 miRFP/GFP-1** (PCR#1)
   T7 promoter (in *italics)* 
TCATAAGCTTTAATACGACTCACTATAGGCGGTTGATGGGTTGTTGGTCAACAGGGC
GGCGATAATCGCCGTTCTGTTGGCTAGCAAGGAGGAAAATATGGCAAACCTGGACAA
G
Sequence #56:
   > **5' primer #1 for RNA P&P SARS-CoV-2 miRFP/GFP-2** (PCR#1)
   T7 promoter (in *italics)* 
TCATAAGCTTTAATACGACTCACTATAGGCGGTTGATGGGTTGTTGGTCAACAGGGC
GGCGATAATTGCCGTTCTGTTGGCTAGCAAGGAGGAAAATATGGCAAACCTGGACAA
G
Sequence #57:
   > **3' primer** (reverse) **for RNA P&P SARS-CoV-2 and *Ecoli* miRFP/GFP-1/-2** (PCR#1) PCR#1/2 32-nt overlapping region in **bold**
   **CAGCTGGTGTGCCACCAACAGGCCCCACAGCT**TCTTGCCCTGCAGGATGGGC
Sequence #58:
   **> PCR product #1 of RNA P&P SARS-CoV-2 miRFP/GFP-1**
   T7 promoter (in *italics);* part of *miRFP670nano* CDS underlined; 32-nt PCR#1/2 overlapping region in **bold** 
TCATAAGCTTTAATACGACTCACTATAGGCGGTTGATGGGTTGTTGGTCAACAGGGC
GGCGATAATCGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTG
Sequence #59:
   > **PCR product #1 of RNA P&P SARS-CoV-2 miRFP/GFP-2**
   T7 promoter (in *italics);* part of *miRFP670nano* CDS underlined; 32-nt PCR#1/2 overlapping region in **bold** 
TCATAAGCTTTAATACGACTCACTATAGGCGGTTGATGGGTTGTTGGTCAACAGGGC
GGCGATAATTGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTG
Sequence #60:
   **> 5' primer #2 for RNA P&P SARS-CoV-2 miRFP/GFP-1/-2** (PCR#2) 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGTGGGCATCGCCATCCAGC
AGAGCTAATTTCCTCCTTGTTAGCCAATAGAATGGTCTAACAAGGAGGAAACAATGTA
TACAACAAACCTGGCGGCAGCGCAAAAGATG
Sequence #61:
   > **PCR product#2 for RNA P&P SARS-CoV-2 miRFP/GFP-1**
   End of *miRFP670nano* CDS underlined; 32-nt overlapping region in **bold,** *gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGGCATCGCCATCCAGC
AGAGCTAATTTCCTCCTTGTTAGCCAATAGAATGGTCTAACAAGGAGGAAACAATGTA
TACAACAAACCTGGCGGCAGCGCAAAAGATGCGTAAAGGAGAAGAACTTTTCACTGG
AGTTGTCCCAATTCTTGTTGAATTAGATGGTGATGTTAATGGGCACAAATTTTCTGTC
AGTGGAGAGGGTGAAGGTGATGCAACATACGGAAAACTTACCCTTAAATTTATTTGC
ACTACTGGAAAACTACCTGTTCCGTGGCCAACACTTGTCACTACTTTCGGTTATGGTG
TTCAATGCTTTGCGAGATACCCAGATCACATGAAACAGCATGACTTTTTCAAGAGTGC
CATGCCCGAAGGTTACGTACAGGAAAGAACTATATTTTTCAAAGATGACGGGAACTA
CAAGACACGTGCTGAAGTCAAGTTTGAAGGTGATACCCTTGTTAATAGAATCGAGTTA
AAAGGTATTGATTTTAAAGAAGATGGAAACATTCTTGGACACAAATTGGAATACAACT
ATAACTCACACAATGTATACATCATGGCAGACAAACAAAAGAATGGAATCAAAGTTAA
CTTCAAAATTAGACACAACATTGAAGATGGAAGCGTTCAACTAGCAGACCATTATCAA
CAAAATACTCCGATTGGCGATGGCCCTGTCCTTTTACCAGACAACCATTACCTGTCCA
CACAATCTGCCCTTTCGAAAGATCCCAACGAAAAGAGAGACCACATGGTCCTTCTTG
AGTTTGTAACCGCTGCTGGGATTACACATGGCATGGATGAACTATACAAAAGGCCTG
CAGCAAACGACGAAAACTACGCTGCATCAGTTTAATAATAAGATAAACCAGAGCGGC
ACGGCAAGCAGAGTATACGAGATTCGGTAGCCACCGCTGAGCAATAACTAGCATAAC
CCCTTGGGGCCTCTAAACGGGTCTTGAGGGGTTTTTTGCATATGCAACGG
Sequence #62:
   **> PCR product#2 for RNA P&P SARS-CoV-2 miRFP/GFP-2**
   End of *miRFP670nano* CDS underlined; 32-nt overlapping region in **bold;** *gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGTGGGCATCGCCATCCAGC
AGAGCTAATTTCCTCCTTGTTAGCCAATAGAATGGTCTAACAAGGAGGAAACAATGTA
TACAACAAACCTGGCGGCAGCGCAAAAGATGCGTAAAGGAGAAGAACTTTTCACTGG
AGTTGTCCCAATTCTTGTTGAATTAGATGGTGATGTTAATGGGCACAAATTTTCTGTC
AGTGGAGAGGGTGAAGGTGATGCAACATACGGAAAACTTACCCTTAAATTTATTTGC
ACTACTGGAAAACTACCTGTTCCGTGGCCAACACTTGTCACTACTTTCGGTTATGGTG
TTCAATGCTTTGCGAGATACCCAGATCACATGAAACAGCATGACTTTTTCAAGAGTGC
CATGCCCGAAGGTTACGTACAGGAAAGAACTATATTTTTCAAAGATGACGGGAACTA
 
CAAGACACGTGCTGAAGTCAAGTTTGAAGGTGATACCCTTGTTAATAGAATCGAGTTA
AAAGGTATTGATTTTAAAGAAGATGGAAACATTCTTGGACACAAATTGGAATACAACT
ATAACTCACACAATGTATACATCATGGCAGACAAACAAAAGAATGGAATCAAAGTTAA
CTTCAAAATTAGACACAACATTGAAGATGGAAGCGTTCAACTAGCAGACCATTATCAA
CAAAATACTCCGATTGGCGATGGCCCTGTCCTTTTACCAGACAACCATTACCTGTCCA
CACAATCTGCCCTTTCGAAAGATCCCAACGAAAAGAGAGACCACATGGTCCTTCTTG
AGTTTGTAACCGCTGCTGGGATTACACATGGCATGGATGAACTATACAAAAGGCCTG
CAGCAAACGACGAAAACTACGCTGCATCAGTTTAATAATAAGATAAACCAGAGCGGC
ACGGCAAGCAGAGTATACGAGATTCGGTAGCCACCGCTGAGCAATAACTAGCATAAC
CCCTTGGGGCCTCTAAACGGGTCTTGAGGGGTTTTTTGCATATGCAACGG
Sequence #63:
   > **5' primer T7** (forward)
   TCATAAGCTTTAATACGACTC
Sequence #64:
   > **3' primer GFP term** (reverse)
   CCGTTGCATATGCAAAAAACC
Sequence #65:
   > **PCR final product of RNA P&P SARS-CoV-2 miRFP/GFP-1**
   T7 promoter (in *italics), gfpmut3b-ASV* CDS in grey 
TCATAAGCTTTAATACGACTCACTATAGGCGGTTGATGGGTTGTTGGTCAACAGGGC
GGCGATAATCGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTGGCGGCCCCTAGACAGTGGCAGACCTGGGAGATCGACTTTCTGAAGCAGCAGGC
CGTGGTGGTGGGCATCGCCATCCAGCAGAGCTAATTTCCTCCTTGTTAGCCAATAGA
ATGGTCTAACAAGGAGGAAACAATGTATACAACAAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
 
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #66:
   > **PCR product of RNA P&P SARS-CoV-2 miRFP/GFP-1**
   T7 promoter (in *italics); miRFP670nano* underlined; *gfpmut3b-ASV* CDS in grey 
TCATAAGCTTTAATACGACTCACTATAGGCGGTTGATGGGTTGTTGGTCAACAGGGC
GGCGATAATTGCCGTTCTGTTGGCTAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTGGCGGCCCCTAGACAGTGGCAGACCTGGGAGATCGACTTTCTGAAGCAGCAGGC
CGTGGTGGTGGGCATCGCCATCCAGCAGAGCTAATTTCCTCCTTGTTAGCCAATAGA
ATGGTCTAACAAGGAGGAAACAATGTATACAACAAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #67:
   **> RNA P&P *Ecoli* miRFP/GFP-1**
   *miRFP670nano* gene CDS underlined; 
GGCAAGUUUGAUGUGGCUGUGCUGCGUGUAUGGGGAUAACUUGUAUGUGUGGCG
CAGCAAGGAGGAAAAUAUGGCAAACCUGGACAAGAUGCUGAAUACCACAGUAACAG
AGGUGCGGCAGUUCCUGCAGGUGGACAGAGUGUGCGUGUUCCAGUUUGAGGAGG
AUUAUAGCGGAGUGGUGGUGGUGGAGGCCGUGGACGAUAGGUGGAUCUCCAUCC
 
UGAAGACCCAGGUGCGGGAUAGAUACUUCAUGGAGACAAGGGGCGAGGAGUAUU
CUCACGGCCGCUACCAGGCCAUCGCCGACAUCUACACCGCAAACCUGACAGAGUG
CUACAGGGAUCUGCUGACACAGUUUCAGGUGAGAGCAAUCCUGGCCGUGCCCAUC
CUGCAGGGCAAGAAGCUGUGGGGCCUGUUGGUGGCACACCAGCUGGCGGCCCCU
AGACAGUGGCAGACCUGGGAGAUCGACUUUCUGAAGCAGCAGGCCGUGGUGGUG
GGCAUCGCCAUCCAGCAGAGCUAAUUUCCUCCUUGUUGCGCUGUAUGUACGGGC
AAUAAGGAGGAAAUAAUGUCAACCACCAACCUGGCGGCAGCGCAAAAGAUG
Sequence #68:
   **> RNA P&P *E.coli* miRFP/GFP-2**
   *miRFP670nano* gene CDS underlined; 
GGCUAGUUGUGUUGUGUGUAUGAAGAAGGCCUUGAAUAACGGGGUCUUCUUUGU
GGUUAAGGAGGAAAAUAUGGCAAACCUGGACAAGAUGCUGAAUACCACAGUAACA
GAGGUGCGGCAGUUCCUGCAGGUGGACAGAGUGUGCGUGUUCCAGUUUGAGGAG
GAUUAUAGCGGAGUGGUGGUGGUGGAGGCCGUGGACGAUAGGUGGAUCUCCAUC
CUGAAGACCCAGGUGCGGGAUAGAUACUUCAUGGAGACAAGGGGCGAGGAGUAU
UCUCACGGCCGCUACCAGGCCAUCGCCGACAUCUACACCGCAAACCUGACAGAGU
GCUACAGGGAUCUGCUGACACAGUUUCAGGUGAGAGCAAUCCUGGCCGUGCCCA
UCCUGCAGGGCAAGAAGCUGUGGGGCCUGUUGGUGGCACACCAGCUGGCGGCCC
CUAGACAGUGGCAGACCUGGGAGAUCGACUUUCUGAAGCAGCAGGCCGUGGUGG
UGGGCAUCGCCAUCCAGCAGAGCUAAUUUCCUCCUUAGUCACGAAGAAGACUUCU
GACUAAGGAGGGAAUAAUGUCGAAGACGAACCUGGCGGCAGCGCAAAAGAUG
Sequence #69:
   > **RNA P&P *E.coli* miRFP/GFP-3**
   *miRFP670nano* gene CDS underlined; 
GGCUAGUUGUGUUGUGUGUAUGAAGAAGGCCUUGAAUAACGGGGUCUUCUUCGU
GGUUAAGGAGGAAAAUAUGGCAAACCUGGACAAGAUGCUGAAUACCACAGUAACA
GAGGUGCGGCAGUUCCUGCAGGUGGACAGAGUGUGCGUGUUCCAGUUUGAGGAG
GAUUAUAGCGGAGUGGUGGUGGUGGAGGCCGUGGACGAUAGGUGGAUCUCCAUC
CUGAAGACCCAGGUGCGGGAUAGAUACUUCAUGGAGACAAGGGGCGAGGAGUAU
UCUCACGGCCGCUACCAGGCCAUCGCCGACAUCUACACCGCAAACCUGACAGAGU
GCUACAGGGAUCUGCUGACACAGUUUCAGGUGAGAGCAAUCCUGGCCGUGCCCA
UCCUGCAGGGCAAGAAGCUGUGGGGCCUGUUGGUGGCACACCAGCUGGCGGCCC
CUAGACAGUGGCAGACCUGGGAGAUCGACUUUCUGAAGCAGCAGGCCGUGGUGG
UGGGCAUCGCCAUCCAGCAGAGCUAAUUUCCUCCUUAGUCACGAAGAAGACUUCU
GACUAAGGAGGGAAUAAUGUCGAAGACGAACCUGGCGGCAGCGCAAAAGAUG
Sequence #70:
   > **RNA P&P *E.coli* miRFP/GFP-4**
   *miRFP670nano* gene CDS underlined; 
GGCUAGUUGUGUUGUGUGUAUGAAGAGGGCCUUGAAUAACGGGGUCUUCUUCGU
GGUUAAGGAGGAAAAUAUGGCAAACCUGGACAAGAUGCUGAAUACCACAGUAACA
GAGGUGCGGCAGUUCCUGCAGGUGGACAGAGUGUGCGUGUUCCAGUUUGAGGAG
GAUUAUAGCGGAGUGGUGGUGGAGGCCGUGGACGAUAGGUGGAUCUCCAUC
CUGAAGACCCAGGUGCGGGAUAGAUACUUCAUGGAGACAAGGGGCGAGGAGUAU
UCUCACGGCCGCUACCAGGCCAUCGCCGACAUCUACACCGCAAACCUGACAGAGU
GCUACAGGGAUCUGCUGACACAGUUUCAGGUGAGAGCAAUCCUGGCCGUGCCCA
UCCUGCAGGGCAAGAAGCUGUGGGGCCUGUUGGUGGCACACCAGCUGGCGGCCC
CUAGACAGUGGCAGACCUGGGAGAUCGACUUUCUGAAGCAGCAGGCCGUGGUGG
UGGGCAUCGCCAUCCAGCAGAGCUAAUUUCCUCCUUAGUCACGAAGAAGACUUCU
GACUAAGGAGGGAAUAAUGUCGAAGACGAACCUGGCGGCAGCGCAAAAGAUG
Sequence #71:
   **> 5' primer #1 for RNA P&P *E.coli* miRFP/GFP-1** (PCR#1)
   T7 promoter (in *italics)* 
TCATAAGCTTTAATACGACTCACTATAGGCAAGTTTGATGTGGCTGTGCTGCGTGTAT
GGGGATAACTTGTATGTGTGGCGCAGCAAGGAGGAAAATATGGCAAACCTGGACAA
G
Sequence #72:
   **> 5' primer #1 for RNA P&P *E.coli* miRFP/GFP-2** (PCR#1)
   T7 promoter (in *italics)* 
TCATAAGCTTTAATACGACTCACTATAGGCTAGTTGTGTTGTGTGTATGAAGAAGGCC
TTGAATAACGGGGTCTTCTTTGTGGTTAAGGAGGAAAATATGGCAAACCTGGACAAG
Sequence #73:
   > **5' primer #1 for RNA P&P *E.coli* miRFP/GFP-3** (PCR#1)
   T7 promoter in *italics*) 
TCATAAGCTTTAATACGACTCACTATAGGCTAGTTGTGTTGTGTGTATGAAGAAGGCC
TTGAATAACGGGGTCTTCTTCGTGGTTAAGGAGGAAAATATGGCAAACCTGGACAAG
Sequence #74:
   > **5' primer #1 for RNA P&P *E.coli* miRFP/GFP-4** (PCR#1)
   T7 promoter (in *italics)* 
TCATAAGCTTTAATACGACTCACTATAGGCTAGTTGTGTTGTGTGTATGAAGAGGGCC
TTGAATAACGGGGTCTTCTTCGTGGTTAAGGAGGAAAATATGGCAAACCTGGACAAG
Sequence #75:
   > **PCR product #1 of RNA P&P *E.coli* miRFP/GFP-1**
   T7 promoter (in *italics);* part of *miRFP670nano* CDS underlined; 32-nt PCR#1/2 overlapping region in **bold** 
TCATAAGCTTTAATACGACTCACTATAGGCAAGTTTGATGTGGCTGTGCTGCGTGTAT
GGGGATAACTTGTATGTGTGGCGCAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTG
Sequence #76:
   > **PCR product #1 of RNA P&P *E.coli* miRFP/GFP-2**
   T7 promoter (in *italics);* part of *miRFP670nano* CDS underlined; 32-nt PCR#1/2 overlapping region in **bold** 
TCATAAGCTTTAATACGACTCACTATAGGCTAGTTGTGTTGTGTGTATGAAGAAGGCC
TTGAATAACGGGGTCTTCTTTGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TG
Sequence #77:
   > **PCR product #1 of RNA P&P *E.coli* miRFP/GFP-3**
   T7 promoter (in *italics);* part of *miRFP670nano* CDS underlined; 32-nt PCR#1/2 overlapping region in **bold** 
TCATAAGCTTTAATACGACTCACTATAGGCTAGTTGTGTTGTGTGTATGAAGAAGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TG
Sequence #78:
   > **PCR product #1 of RNA P&P *E.coli* miRFP/GFP-4**
   T7 promoter (in *italics);* part of *miRFP670nano* CDS underlined; 32-nt PCR#1/2 overlapping region in **bold** 
TCATAAGCTTTAATACGACTCACTATAGGCTAGTTGTGTTGTGTGTATGAAGAGGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TG
Sequence #79:
   **> 5' primer #2 for RNA P&P *E.coli* miRFP/GFP-1** (PCR#2) 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGTGGGCATCGCCATCCAGC
AGAGCTAATTTCCTCCTTGTTGCGCTGTATGTACGGGCAATAAGGAGGAAATAATGT
CAACCACCAACCTGGCGGCAGCGCAAAAGATG
Sequence #80:
   > **5' primer #2 for RNA P&P *E.coli* miRFP/GFP-2/-3/-4** (PCR#2) 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGTGGGCATCGCCATCCAGC
AGAGCTAATTTCCTCCTTAGTCACGAAGAAGACTTCTGACTAAGGAGGGAATAATGTC
GAAGACGAACCTGGCGGCAGCGCAAAAGATG
Sequence #81:
   **> PCR product#2 for RNA P&P *E.coli* miRFP/GFP-1**
   End of *miRFP670nano* CDS underlined; 32-nt overlapping region in **bold*****;** gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGGCATCGCCATCCAGC
AGAGCTAATTTCCTCCTTGTTGCGCTGTATGTACGGGCAATAAGGAGGAAATAATGT
CAACCACCAACCTGGCGGCAGCGCAAAAGATGCGTAAAGGAGAAGAACTTTTCACT
GGAGTTGTCCCAATTCTTGTTGAATTAGATGGTGATGTTAATGGGCACAAATTTTCTG
TCAGTGGAGAGGGTGAAGGTGATGCAACATACGGAAAACTTACCCTTAAATTTATTTG
CACTACTGGAAAACTACCTGTTCCGTGGCCAACACTTGTCACTACTTTCGGTTATGGT
GTTCAATGCTTTGCGAGATACCCAGATCACATGAAACAGCATGACTTTTTCAAGAGTG
CCATGCCCGAAGGTTACGTACAGGAAAGAACTATATTTTTCAAAGATGACGGGAACT
ACAAGACACGTGCTGAAGTCAAGTTTGAAGGTGATACCCTTGTTAATAGAATCGAGTT
AAAAGGTATTGATTTTAAAGAAGATGGAAACATTCTTGGACACAAATTGGAATACAAC
 
TATAACTCACACAATGTATACATCATGGCAGACAAACAAAAGAATGGAATCAAAGTTA
ACTTCAAAATTAGACACAACATTGAAGATGGAAGCGTTCAACTAGCAGACCATTATCA
ACAAAATACTCCGATTGGCGATGGCCCTGTCCTTTTACCAGACAACCATTACCTGTCC
ACACAATCTGCCCTTTCGAAAGATCCCAACGAAAAGAGAGACCACATGGTCCTTCTT
GAGTTTGTAACCGCTGCTGGGATTACACATGGCATGGATGAACTATACAAAAGGCCT
GCAGCAAACGACGAAAACTACGCTGCATCAGTTTAATAATAAGATAAACCAGAGCGG
CACGGCAAGCAGAGTATACGAGATTCGGTAGCCACCGCTGAGCAATAACTAGCATAA
CCCCTTGGGGCCTCTAAACGGGTCTTGAGGGGTTTTTTGCATATGCAACGG
Sequence #82:
   > **PCR product#2 for RNA P&P *E.coli* miRFP/GFP-2/-3/-4**
   End of *miRFP670nano* CDS underlined; 32-nt overlapping region in **bold,** *gfpmut3b-ASV* CDS in grey 
AGCTGTGGGGCCTGTTGGTGGCACACCAGCTGGCGGCCCCTAGACAGTGGCAGAC
CTGGGAGATCGACTTTCTGAAGCAGCAGGCCGTGGTGGGCATCGCCATCCAGC
AGAGCTAATTTCCTCCTTAGTCACGAAGAAGACTTCTGACTAAGGAGGGAATAATGTC
GAAGACGAACCTGGCGGCAGCGCAAAAGATGCGTAAAGGAGAAGAACTTTTCACTG
GAGTTGTCCCAATTCTTGTTGAATTAGATGGTGATGTTAATGGGCACAAATTTTCTGT
CAGTGGAGAGGGTGAAGGTGATGCAACATACGGAAAACTTACCCTTAAATTTATTTG
CACTACTGGAAAACTACCTGTTCCGTGGCCAACACTTGTCACTACTTTCGGTTATGGT
GTTCAATGCTTTGCGAGATACCCAGATCACATGAAACAGCATGACTTTTTCAAGAGTG
CCATGCCCGAAGGTTACGTACAGGAAAGAACTATATTTTTCAAAGATGACGGGAACT
ACAAGACACGTGCTGAAGTCAAGTTTGAAGGTGATACCCTTGTTAATAGAATCGAGTT
AAAAGGTATTGATTTTAAAGAAGATGGAAACATTCTTGGACACAAATTGGAATACAAC
TATAACTCACACAATGTATACATCATGGCAGACAAACAAAAGAATGGAATCAAAGTTA
ACTTCAAAATTAGACACAACATTGAAGATGGAAGCGTTCAACTAGCAGACCATTATCA
ACAAAATACTCCGATTGGCGATGGCCCTGTCCTTTTACCAGACAACCATTACCTGTCC
ACACAATCTGCCCTTTCGAAAGATCCCAACGAAAAGAGAGACCACATGGTCCTTCTT
GAGTTTGTAACCGCTGCTGGGATTACACATGGCATGGATGAACTATACAAAAGGCCT
GCAGCAAACGACGAAAACTACGCTGCATCAGTTTAATAATAAGATAAACCAGAGCGG
CACGGCAAGCAGAGTATACGAGATTCGGTAGCCACCGCTGAGCAATAACTAGCATAA
CCCCTTGGGGCCTCTAAACGGGTCTTGAGGGGTTTTTTGCATATGCAACGG
Sequence #83:
   **> PCR final product of RNA P&P *E.coli* miRFP/GFP-1**
   T7 promoter (in *italics), gfpmut3b-ASV* CDS in grey 
TCATAAGCTTTAATACGACTCACTATAGGCAAGTTTGATGTGGCTGTGCTGCGTGTAT
GGGGATAACTTGTATGTGTGGCGCAGCAAGGAGGAAAATATGGCAAACCTGGACAA
GATGCTGAATACCACAGTAACAGAGGTGCGGCAGTTCCTGCAGGTGGACAGAGTGT
GCGTGTTCCAGTTTGAGGAGGATTATAGCGGAGTGGTGGTGGAGGCCGTGGAC
GATAGGTGGATCTCCATCCTGAAGACCCAGGTGCGGGATAGATACTTCATGGAGACA
AGGGGCGAGGAGTATTCTCACGGCCGCTACCAGGCCATCGCCGACATCTACACCGC
AAACCTGACAGAGTGCTACAGGGATCTGCTGACACAGTTTCAGGTGAGAGCAATCCT
GGCCGTGCCCATCCTGCAGGGCAAGAAGCTGTGGGGCCTGTTGGTGGCACACCAG
CTGGCGGCCCCTAGACAGTGGCAGACCTGGGAGATCGACTTTCTGAAGCAGCAGGC
CGTGGTGGTGGGCATCGCCATCCAGCAGAGCTAATTTCCTCCTTGTTGCGCTGTATG
TACGGGCAATAAGGAGGAAATAATGTCAACCACCAACCTGGCGGCAGCGCAAAAGA
 
TGCGTAAAGGAGAAGAACTTTTCACTGGAGTTGTCCCAATTCTTGTTGAATTAGATGG
TGATGTTAATGGGCACAAATTTTCTGTCAGTGGAGAGGGTGAAGGTGATGCAACATA
CGGAAAACTTACCCTTAAATTTATTTGCACTACTGGAAAACTACCTGTTCCGTGGCCA
ACACTTGTCACTACTTTCGGTTATGGTGTTCAATGCTTTGCGAGATACCCAGATCACA
TGAAACAGCATGACTTTTTCAAGAGTGCCATGCCCGAAGGTTACGTACAGGAAAGAA
CTATATTTTTCAAAGATGACGGGAACTACAAGACACGTGCTGAAGTCAAGTTTGAAGG
TGATACCCTTGTTAATAGAATCGAGTTAAAAGGTATTGATTTTAAAGAAGATGGAAAC
ATTCTTGGACACAAATTGGAATACAACTATAACTCACACAATGTATACATCATGGCAG
ACAAACAAAAGAATGGAATCAAAGTTAACTTCAAAATTAGACACAACATTGAAGATGG
AAGCGTTCAACTAGCAGACCATTATCAACAAAATACTCCGATTGGCGATGGCCCTGT
CCTTTTACCAGACAACCATTACCTGTCCACACAATCTGCCCTTTCGAAAGATCCCAAC
GAAAAGAGAGACCACATGGTCCTTCTTGAGTTTGTAACCGCTGCTGGGATTACACAT
GGCATGGATGAACTATACAAAAGGCCTGCAGCAAACGACGAAAACTACGCTGCATCA
GTTTAATAATAAGATAAACCAGAGCGGCACGGCAAGCAGAGTATACGAGATTCGGTA
GCCACCGCTGAGCAATAACTAGCATAACCCCTTGGGGCCTCTAAACGGGTCTTGAG
GGGTTTTTTGCATATGCAACGG
Sequence #84:
   > **PCR final product of RNA P&P *E.coli* miRFP/GFP-2**
   T7 promoter (in *italics), gfpmut3b-ASV* CDS in grey 
TCATAAGCTTTAATACGACTCACTATAGGCTAGTTGTGTTGTGTGTATGAAGAAGGCC
TTGAATAACGGGGTCTTCTTTGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TGGCGGCCCCTAGACAGTGGCAGACCTGGGAGATCGACTTTCTGAAGCAGCAGGCC
GTGGTGGTGGGCATCGCCATCCAGCAGAGCTAATTTCCTCCTTAGTCACGAAGAAGA
CTTCTGACTAAGGAGGGAATAATGTCGAAGACGAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #85:
   > **PCR final product of RNA P&P *E.coli* miRFP/GFP-3**
   T7 promoter (in *italics), gfpmut3b-ASV* CDS in grey 
TCATAAGCTTTAATACGACTCACTATAGGCTAGTTGTGTTGTGTGTATGAAGAAGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TGGCGGCCCCTAGACAGTGGCAGACCTGGGAGATCGACTTTCTGAAGCAGCAGGCC
GTGGTGGTGGGCATCGCCATCCAGCAGAGCTAATTTCCTCCTTAGTCACGAAGAAGA
CTTCTGACTAAGGAGGGAATAATGTCGAAGACGAACCTGGCGGCAGCGCAAAAGAT
GCGTAAAGGAGAAGAACTTTTCACTGGAGTTGTCCCAATTCTTGTTGAATTAGATGGT
GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG
Sequence #86:
   **> PCR final product of RNA P&P *E.coli* miRFP/GFP-4**
   T7 promoter (in *italics), gfpmut3b-ASV* CDS in grey

TCATAAGCTTTAATACGACTCACTATAGGCTAGTTGTGTTGTGTGTATGAAGAGGGCC
TTGAATAACGGGGTCTTCTTCGTGGTTAAGGAGGAAAATATGGCAAACCTGGACAAG
ATGCTGAATACCACAGTAACAGAGGTGCGGCAGTTCCTGCAGGTGGACAGAGTGTG
CGTGTTCCAGTTTGAGGAGGATTATAGCGGAGTGGTGGTGGAGGCCGTGGACG
ATAGGTGGATCTCCATCCTGAAGACCCAGGTGCGGGATAGATACTTCATGGAGACAA
GGGGCGAGGAGTATTCTCACGGCCGCTACCAGGCCATCGCCGACATCTACACCGCA
AACCTGACAGAGTGCTACAGGGATCTGCTGACACAGTTTCAGGTGAGAGCAATCCTG
GCCGTGCCCATCCTGCAGGGCAAGAAGCTGTGGGGCCTGTTGGTGGCACACCAGC
TGGCGGCCCCTAGACAGTGGCAGACCTGGGAGATCGACTTTCTGAAGCAGCAGGCC
GTGGTGGTGGGCATCGCCATCCAGCAGAGCTAATTTCCTCCTTAGTCACGAAGAAGA
CTTCTGACTAAGGAGGGAATAATGTCGAAGACGAACCTGGCGGCAGCGCAAAAGAT
GCGTAAAGGAGAAGAACTTTTCACTGGAGTTGTCCCAATTCTTGTTGAATTAGATGGT

GATGTTAATGGGCACAAATTTTCTGTCAGTGGAGAGGGTGAAGGTGATGCAACATAC
GGAAAACTTACCCTTAAATTTATTTGCACTACTGGAAAACTACCTGTTCCGTGGCCAA
CACTTGTCACTACTTTCGGTTATGGTGTTCAATGCTTTGCGAGATACCCAGATCACAT
GAAACAGCATGACTTTTTCAAGAGTGCCATGCCCGAAGGTTACGTACAGGAAAGAAC
TATATTTTTCAAAGATGACGGGAACTACAAGACACGTGCTGAAGTCAAGTTTGAAGGT
GATACCCTTGTTAATAGAATCGAGTTAAAAGGTATTGATTTTAAAGAAGATGGAAACA
TTCTTGGACACAAATTGGAATACAACTATAACTCACACAATGTATACATCATGGCAGA
CAAACAAAAGAATGGAATCAAAGTTAACTTCAAAATTAGACACAACATTGAAGATGGA
AGCGTTCAACTAGCAGACCATTATCAACAAAATACTCCGATTGGCGATGGCCCTGTC
CTTTTACCAGACAACCATTACCTGTCCACACAATCTGCCCTTTCGAAAGATCCCAACG
AAAAGAGAGACCACATGGTCCTTCTTGAGTTTGTAACCGCTGCTGGGATTACACATG
GCATGGATGAACTATACAAAAGGCCTGCAGCAAACGACGAAAACTACGCTGCATCAG
TTTAATAATAAGATAAACCAGAGCGGCACGGCAAGCAGAGTATACGAGATTCGGTAG
CCACCGCTGAGCAATAACTAGCATAACCCCTTGGGGCCTCTAAACGGGTCTTGAGG
GGTTTTTTGCATATGCAACGG

## Claims

1. An RNA molecule comprising a sensor part and a trigger part, wherein the trigger part is an mRNA coding for a protein and the sensor part comprises 2 stem loops divided in the following regions in order from 5' to 3': T (T1 ,T2), A, B, C (C1,C2), D (D1,D2), E, F (F1,F2) and G, wherein
- region T1 is unpaired;
- T2 forms a stem with B where A is the loop region;
- C can be paired or unpaired;
- D forms a stem with F, where E is the loop region;
- F2 must contain an RBS;
- stem loop D1 basepairs with F2 and D2 with F1;
- G contains an ATG and a linker and is immediately followed by the trigger part;
- further with the following structural limitations:
a) T has a length of >= 30 nucleotides;
b) T has the same number of nucleotides as B;
c) D1 has the same number of nucleotides as F2 and D2 has the same number of nucleotides as F1:
d) RBS in F1 is at least 3 nucleotides away from its start;
e) (B + C1) has the same number of nucleotides as (D + E);
f) D2 + E has the same number of nucleotides as T;
g) C1 has the same number of nucleotides as D1;
h) RBS in F2 must be at least 3 nucleotides away from F2 3' end;
further wherein in the absence of a target RNA, the RBS in region F2 is sequestered in the DEF stem loop and thus, translation of the trigger is not possible;
further wherein in the presence of a target RNA, the sensor part hybridizes the target RNA and a conformational change is produced, in which region T is sequestered by target RNA, B and C1 form a stem loop with D and E, where C2 is the loop region, F and G are partially or totally unpaired and thus the RBS in F2 is not sequestered and translation of the trigger is possible; and wherein RBS do not form stems with regions that also hybridize to the target RNA or form a stem with regions that hybridize with the regions which hybridize the target RNA.

2. An RNA molecule, according to claim 1, wherein in the sensor part:
- C contains an RBS in C1 and a sensor reporter gene different from the trigger part,
- RBS in C1 must be at least 3 nucleotides away from C1 each end;
- ATG of the reporter gene can be either on C1 or C2 as long as the distance from the RBS is from 3 to 10, preferably 5-6 nucleotides;
- D+E must be proportional to the length of C2; and
wherein in the absence of RNA target, RBS in C1 is partially or totally unpaired, and
wherein in the presence of RNA target, C1-D1 are part of the stem BC1-DE.

3. The RNA molecule according to claim 1, wherein the trigger part is an mRNA coding for a bioluminescence protein, a fluorescent protein or a colorimetric reporter protein.

4. The RNA molecule according to claim 2, wherein both the sensor and the trigger reporter genes are mRNAs coding for a bioluminescence protein, a fluorescent protein or a colorimetric reporter protein, as long as both reporter genes are different.

5. The RNA molecule, according to claim 3 or 4, wherein said bioluminescence protein is selected from luciferase and aequorin, said fluorescent protein is selected from GFP, YFP and RFP and said colorimetric reporter protein is LacZ.

6. The RNA molecule of claim 1,3,5 where there is no sensor reporter gene and the trigger is a LacZ mRNA and the sensor part corresponds to the following artificial RNA sequences selected from SEQ ID NO:, 4, 5 and 6 , where their respective artificial RNA targets are the following: SEQ ID NO: 1 for SEQ ID NO: 4 and 5; and SEQ ID NO: 2 for SEQ ID NO: 6.

7. The RNA molecule according to any of claims 1 to 6, wherein said artificial RNA target is a biomarker characterizing a disease selected from a viral infection, a bacterial infection, cancer or a genetic disease.

8. The RNA molecule according to claim 7, being dependent of claim 1, 3, 5 and 6, wherein said biomarker is a region of the e.coli rRNA16s mRNA reverse complemented, the trigger is a LacZ mRNA and wherein the sensor part corresponds to the following artificial RNA sequences: SEQ ID NO: 16 and 17 or alternatively, where the trigger is a GFP mRNA and wherein the sensor part corresponds to the following artificial RNA sequences: SEQ ID NO: 24, 25 and 26, or alternatively, being dependent of claim 2, 4, 5, wherein the sensor reporter gene is RFP and the trigger is a GFP mRNA corresponding to sequences SEQ ID NOs: 67, 68, 69 and 70.

9. The RNA molecule according to claim 7, being dependent of claim 1, 3, 5 and 6, wherein said biomarker is a region of the SARS-CoV-2 viral genome (SEQ ID NO: 38), the trigger is a LacZ mRNA and wherein the sensor part corresponds to the following artificial RNA sequences: SEQ ID NO: 36 and SEQ ID NO: 37 or alternatively, wherein said biomarker are regions of the SARS-CoV-2 viral genome reverse complemented (SEQ ID NO: 46, SEQ ID NO: 47), the trigger is a GFP mRNA and wherein the sensor part corresponds to the following artificial RNA sequences: SEQ ID NO: 44 and 45, or alternatively, being dependent of claim 2, 4, 5, wherein said biomarker are regions of the SARS-CoV-2 viral genome reverse complemented , the sensor reporter gene is RFP and the trigger is a GFP mRNA corresponding to sequences SEQ ID NOs:53 and 54.

10. Use of the RNA molecule according to claims 1 to 9 in a colorimetric or fluorometric assay upon RNA target binding of said RNA molecule, wherein said RNA molecule is contained with a cell extract in the recipient to be used in an in-vitro diagnostic of a certain disease.

11. A process for detecting in vitro a disease comprising the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule according to any claims 1, 3, 5, 6, 7, 8, 9; and
f) identifying a positive result of the disease by confirming the expression of the trigger mRNA.

12. A process for detecting in vitro a disease comprising the steps of:
a) obtaining an isolated sample of blood or saliva or swabs of body fluids from an individual;
c) extracting and purifying the RNA;
d) amplifying the RNA obtained in step c) through isothermal amplification;
e) detecting the RNA from cells and/or microorganisms causing the disease through an RNA molecule according to any claims 2, 4, 5, 7, 8, 9; and
f) identifying a positive result of the disease by confirming the expression of the trigger mRNA.
g) identifying a negative result of the disease by confirming the expression of the sensor mRNA.
h) Null if no expression detected.

13. The process, according to claims 11 or 12, wherein
1) the isothermal amplification is selected from NASBA, TMA and SMART technologies, and/or
2) the RNA molecule according to claims 1 to 9 is lyophilized, along with a cell extract, in a microfluidics device, and/or
3) the identification of a positive result in step f) is confirmed through a colorimetric or fluorometric assay wherein the trigger is a LacZ mRNA or GFP.
4) the identification of a negative result in step f) is confirmed through a fluorimetric assay wherein the sensor reporter is RFP.

14. The process, according to claim any of claims 11 to 13, wherein the process further comprises between steps a) and c):
b) treating said isolated sample or swab with at least a lysate buffer and/or a heating step.

15. A kit for detecting in vitro a disease comprising:
a) at least one receiving means for receiving said sample or swab isolated from the individual;
b) a lysate buffer for treating the sample or swab;
c) heating capacity.
d) at least the RNA molecule according to any of claims 1 to 9 for the detection of a disease in the individual;
e) instructions for using the kit.

16. The process, according to any of claims 11 to 14, the kit, according to claim 15, or the use, according to claim 10, wherein said disease is a bloodstream infection caused by pathogenic bacteria or COVID-19.

## Patentansprüche

1. Ein RNA-Molekül, umfassend ein Sensorteil und ein Triggerteil, wobei das Triggerteil eine für ein Protein kodierende mRNA ist und das Sensorteil 2 Stammschleifen umfasst, die in den folgenden Bereichen in der Reihenfolge von 5 'bis 3 ': T (T1,T2), a, B, C (C1,C2), D (D1,D2), E, F (F1,F2) und G unterteilt sind, wobei
- Bereich T1 ungeordnet ist;
- T2 einen Stamm mit B bildet, wobei a der Schlaufenbereich ist;
- C kann gepaart oder ungeordnet sein;
- D einen Stamm mit F bildet, wobei E der Schlaufenbereich ist;
- F2 muss eine RBS enthalten;
- Stammschleife D1 Basenpaare mit F2 und D2 mit F1;
- G enthält ein ATG und einen Linker und wird unmittelbar gefolgt von dem Triggerteil;
- ferner mit den folgenden strukturellen Einschränkungen :
a) T eine Länge von > = 30 Nukleotiden aufweist;
B) T hat die gleiche Anzahl von Nukleotiden wie B;
c) D1 hat die gleiche Anzahl von Nukleotiden wie F2 und D2 hat die gleiche Anzahl von Nukleotiden wie F1 :
d) RBS in F1 ist mindestens 3 Nukleotide von ihrem Anfang entfernt;
E) (B + C1) die gleiche Anzahl an Nukleotiden wie (D + E) aufweist);
f) D2 + E hat dieselbe Anzahl von Nukleotiden wie T;
g) C1 hat die gleiche Anzahl von Nukleotiden wie D1;
h) RBS in F2 muss mindestens 3 Nukleotide von F2 3 'entfernt sein;
Wobei in Abwesenheit einer Ziel-RNA die RBS im Bereich F2 in der DEF-Stammschleife sequestriert wird und somit eine Translation des Auslösers nicht möglich ist;
Wobei das Sensorteil in Gegenwart einer Ziel-RNA mit der Ziel-RNA hybridisiert und eine Konformationsänderung erzeugt wird, wobei die Region T durch Target-RNA sequestriert wird, B und C1 eine Stammschleife mit D und E bilden, wobei C2 der Schleifenbereich ist, F und G teilweise oder vollständig ungeordnet sind und somit die RBS in F2 nicht sequestriert wird und die Translation des Auslösers möglich ist; und wobei RBS keine Stämme mit Regionen bilden, die auch mit der Ziel-RNA hybridisieren oder einen Stamm mit Regionen bilden, die mit den Regionen hybridisieren, die Ziel-RNA hybridisieren.

2. Ein RNA-Molekül nach Anspruch 1, wobei in dem Sensorteil :
- C eine RBS in C1 und ein vom Triggerteil verschiedenes Sensorreportergen enthält,
- RBS in C1 muss mindestens 3 Nukleotide von jedem Ende weg sein;
- ATG des Reportergens kann entweder auf C1 oder C2 liegen, solange der Abstand von der RBS 3 bis 10, vorzugsweise 5 -6 Nukleotide beträgt;
- D + E proportional zur Länge von C2 sein muss; und
Wobei in Abwesenheit von RNA-Target RBS in C1 teilweise oder vollständig ungeordnet ist, und wobei in Gegenwart von RNA-Target C1 -D1 Teil des Stamms BC1 -DE sind.

3. Das RNA-Molekül nach Anspruch 1, wobei das Triggerteil eine mRNA ist, die für ein Biolumineszenzprotein, ein fluoreszierendes Protein oder ein kolorimetrisches Reporterprotein kodiert.

4. Das RNA-Molekül nach Anspruch 2, wobei sowohl der Sensor als auch die Trigger-Reportergene mRNAs sind, die für ein Biolumineszenzprotein, ein fluoreszierendes Protein oder ein kolorimetrisches Reporterprotein kodieren, solange beide Reportergene unterschiedlich sind.

5. Das RNA-Molekül nach Anspruch 3 oder 4, wobei das Biolumineszenz-Protein ausgewählt ist aus Luciferase und Aequorin, wobei das fluoreszierende Protein aus GFP, YFP und RFP ausgewählt ist und das kolorimetrische Reporterprotein LACZ ist.

6. Das RNA-Molekül nach Anspruch 1,3,5, wobei es kein Sensorreportergen gibt und der Trigger eine LACZ-mRNA ist und das Sensorteil den folgenden künstlichen RNA-Sequenzen entspricht, die aus SEQ ID NO: 4, 5 und 6 ausgewählt sind, wobei ihre jeweiligen künstlichen RNA-Ziele die folgenden sind: SEQ ID NO: 1 für SEQ ID NO: 4 und 5; und SEQ ID NO: 2 für SEQ ID NO: 6.

7. Das RNA-Molekül nach einem der Ansprüche 1 bis 6, wobei das künstliche RNA-Target ein Biomarker ist, der eine Erkrankung charakterisiert, die aus einer viralen Infektion, einer bakteriellen Infektion, Krebs oder einer genetischen Erkrankung ausgewählt ist.

8. Das RNA-Molekül nach Anspruch 7, das von Anspruch 1, 3, 5 und 6 abhängig ist, wobei es sich bei dem Biomarker um einen Bereich der mRNA-Reverse-Komplementierung von E. coli handelt, der Auslöser eine LACZ-mRNA ist und wobei das Sensorteil den folgenden künstlichen RNA-Sequenzen entspricht : SEQ ID NO: 16 und 17 oder alternativ, wobei der Auslöser eine GFP-mRNA ist und wobei das Sensorteil den folgenden künstlichen RNA-Sequenzen entspricht: SEQ ID NO: 24, 25 und 26 oder alternativ abhängig von Anspruch 2, 4, 5, wobei das Sensor-Reportergen RFP ist und der Auslöser eine GFP-mRNA entsprechend den Sequenzen SEQ ID NO: 67, 68, 69 und 70 ist.

9. Das RNA-Molekül nach Anspruch 7, das von Anspruch 1, 3, 5 und 6 abhängig ist, wobei der Biomarker ein Bereich des SARS-CoV-2 - viralen Genoms (SEQ ID NO : 38), wobei der Auslöser eine LACZ-mRNA ist und wobei das Sensorteil den folgenden künstlichen RNA-Sequenzen entspricht : SEQ ID NO : 36 und SEQ ID NO : 37 oder alternativ, wobei der Biomarker Bereiche des SARS-CoV-2 viralen Genoms revers komplementiert (SEQ ID NO : 46, SEQ ID NO: 47), wobei der Auslöser eine GFP-mRNA ist und wobei das Sensorteil den folgenden künstlichen RNA-Sequenzen entspricht: SEQ ID NO: 44 und 45 oder alternativ abhängig von Anspruch 2, 4, 5, wobei der Biomarker Regionen des SARS-CoV-2 - viralen Genoms rückwärts komplementiert sind, das Sensor-Reportergen RFP ist und der Auslöser eine GFP-mRNA ist, die den Sequenzen SEQ ID NO: 53 und 54 entspricht.

10. Verwendung des RNA-Moleküls nach den Ansprüchen 1 bis 9 in einem kolorimetrischen oder fluorometrischen Assay auf RNA-Zielbindung des RNA-Moleküls, wobei das RNA-Molekül mit einem Zellextrakt in dem Empfänger enthalten ist, um in einer in-vitro-Diagnose einer bestimmten Erkrankung verwendet zu werden.

11. Verfahren zur In-vitro-Detektion einer Krankheit, umfassend die Schritte:
a) Erhalten einer isolierten Probe von Blut oder Speichel oder Schlupfen von Körperflüssigkeiten aus einem Individuum;
c) Extrahieren und Reinigen der RNA;
d) Amplifizieren der in Schritt c) erhaltenen RNA durch isotherme Amplifikation;
e) Detektieren der RNA aus Zellen und/oder Mikroorganismen, die Erkrankung durch ein RNA-Molekül nach einem der Ansprüche 1, 3, 5, 6, 7, 8, 9 verursachen; und
f) Identifizieren eines positiven Ergebnisses der Erkrankung durch Bestätigen der Expression der Trigger-mRNA.

12. Verfahren zur In-vitro-Detektion einer Krankheit, umfassend die Schritte:
a) Erhalten einer isolierten Probe von Blut oder Speichel oder Schlupfen von Körperflüssigkeiten aus einem Individuum;
c) Extrahieren und Reinigen der RNA;
d) Amplifizieren der in Schritt c) erhaltenen RNA durch isotherme Amplifikation;
e) Detektieren der RNA aus Zellen und/oder Mikroorganismen, die Erkrankung durch ein RNA-Molekül nach einem der Ansprüche 2, 4, 5, 7, 8, 9 verursachen; und
f) Identifizieren eines positiven Ergebnisses der Erkrankung durch Bestätigen der Expression der Trigger-mRNA.
g) Identifizieren eines negativen Ergebnisses der Erkrankung durch Bestätigen der Expression der Sensor-mRNA.
h) Null, wenn keine Expression nachgewiesen wird.

13. Verfahren nach Anspruch 11 oder 12, wobei
1) die isotherme Amplifikation ausgewählt ist aus NASBA, TMA und SMART Technologies, und/oder
2) das RNA-Molekül nach den Ansprüchen 1 bis 9 wird zusammen mit einem Zellextrakt in einer Mikrofluidik-Vorrichtung lyophilisiert und/oder
3) Die Identifizierung eines positiven Ergebnisses in Schritt f) wird durch einen kolorimetrischen oder fluorometrischen Assay bestätigt, wobei der Trigger eine LACZ-mRNA oder GFP ist.
4) die Identifizierung eines negativen Ergebnisses in Schritt f) durch einen fluorimetrischen Assay bestätigt wird, wobei der Sensorreporter RFP ist.

14. Verfahren nach einem der Ansprüche 11 bis 13, wobei das Verfahren ferner zwischen den Schritten a) und c) umfasst ):
b) Behandeln der isolierten Probe oder Tupfer mit mindestens einem Lysatpuffer und/oder einem Erwärmungsschritt.

15. Kit zum In-vitro-Nachweis einer Krankheit, umfassend :
a) mindestens ein Aufnahmemittel zur Aufnahme der aus dem Individuum isolierten Probe oder Tupfers;
b) einen Lysatpuffer zur Behandlung der Probe oder des Tupfers;
c) Heizkapazität.
d) mindestens das RNA-Molekül nach einem der Ansprüche 1 bis 9 zur Detektion einer Erkrankung im Individuum;
e) Anweisungen zur Verwendung des Kits.

16. Verfahren nach einem der Ansprüche 11 bis 14, Kit nach Anspruch 15 oder Verwendung nach Anspruch 10, wobei die Krankheit eine Blutstrominfektion ist, die durch pathogene Bakterien oder COVID-19 verursacht wird.

## Revendications

1. Une molécule d'ARN comprend une partie de capteur et une partie de déclenchement, la partie de déclenchement étant un ARNm codant pour une protéine et la partie de capteur comprenant 2 boucles de tige divisées dans les régions suivantes dans l'ordre de 5 'à 3 ' : T (T1, T2), a, B, C (C1, C2), D (D1,D2), E, F (F1,F2) et G,
- la région T1 n'est pas appariée;
- T2 forme une tige avec B où a est la région de boucle;
C peut être apparié ou non apparié;
- D forme une tige avec F, E étant la région de boucle;
- F2 doit contenir une RBS;
- boucle de tige D1 en paires avec F2 et D2 avec F1;
- G contient un ATG et un lieur et est immédiatement suivie par la partie de déclenchement;
- en outre avec les limitations structurales suivantes :
a) T a une longueur > = 30 nucléotides;
B) T a le même nombre de nucléotides que B;
c) D1 a le même nombre de nucléotides que F2 et D2 a le même nombre de nucléotides que F1 :
d) RBS dans F1 est au moins 3 nucléotides à distance de son début;
E) (B + C1) a le même nombre de nucléotides que (D + E );
f) D2 + E a le même nombre de nucléotides que T;
g) C1 a le même nombre de nucléotides que D1;
h) RBS dans F2 doit être au moins 3 nucléotides à l'écart de l'extrémité F2 3 ';
En outre, en l'absence d'un ARN cible, la RBS dans la région F2 est séquestrée dans la boucle de tige DEF et ainsi, la translation du déclencheur n'est pas possible;
En outre, en présence d'un ARN cible, la partie capteur hybride l'ARN cible et un changement conformationnel est produit, dans laquelle la région T est séquestrée par l'ARN cible, B et C1 forment une boucle de tige avec D et E, où C2 est la région de boucle, F et G sont partiellement ou totalement non appariés et ainsi la RBS dans F2 n'est pas séquestrée et la translation du déclencheur est possible; et RBS ne formant pas de tiges avec des régions qui s'hybrident également à l'ARN cible ou forment une tige avec des régions qui s'hybrident avec les régions qui hybrident l'ARN cible.

2. Une molécule d'ARN selon la revendication 1, dans laquelle dans la partie capteur :
- C contient une RBS en C1 et un gène rapporteur de capteur différent de la partie de déclenchement,
- RBS dans C1 doit être d'au moins 3 nucléotides à distance de C1 chaque extrémité;
- ATG du gène rapporteur peut être soit en C1 soit en C2 tant que la distance à partir de la RBS est de 3 à 10, de préférence de 5 à 6 nucléotides;
- D + E doit être proportionnel à la longueur de C2; et
dans laquelle en l'absence de cible d'ARN, la RBS dans C1 est partiellement ou totalement non appariée, et en présence d'une cible d'ARN, C1 -D1 font partie de la tige BC1 -DE.

3. La molécule d'ARN selon la revendication 1, dans laquelle la partie de déclenchement est un ARNm codant pour une protéine de bioluminescence, une protéine fluorescente ou une protéine rapporteur colorimétrique.

4. La molécule d'ARN selon la revendication 2, dans laquelle le capteur et les gènes rapporteurs de déclenchement sont tous deux des ARNm codant pour une protéine de bioluminescence, une protéine fluorescente ou une protéine rapporteur colorimétrique, tant que les deux gènes rapporteurs sont différents.

5. La molécule d'ARN, selon la revendication 3 ou 4, dans laquelle ladite protéine de bioluminescence est choisie parmi la luciférase et l'aéquorine, ladite protéine fluorescente est choisie parmi GFP, YFP et RFP et ladite protéine rapporteur colorimétrique est LacZ.

6. La molécule d'ARN selon la revendication 1,3,5, dans laquelle il n'y a pas de gène rapporteur de capteur et le déclencheur est un ARNm de LacZ et la partie de capteur correspond aux séquences d'ARN artificiel suivantes choisies parmi SEQ ID NO :, 4, 5 et 6, leurs cibles d'ARN artificiel respectives étant les suivantes : SEQ ID NO : 1 pour SEQ ID NO : 4 et 5; et SEQ ID NO : 2 pour SEQ ID NO : 6.

7. La molécule d'ARN selon l'une quelconque des revendications 1 à 6, dans laquelle ladite cible d'ARN artificiel est un biomarqueur caractérisant une maladie choisie parmi une infection virale, une infection bactérienne, un cancer ou une maladie génétique.

8. La molécule d'ARN selon la revendication 7, dépendant des revendications 1, 3, 5 et 6, dans laquelle ledit biomarqueur est une région de l'ARNm de rRNA16s E.coli inversé, le déclencheur est un ARNm de LacZ et la partie de capteur correspond aux séquences d'ARN artificielles suivantes : SEQ ID NO : 16 et 17 ou en variante, le déclencheur étant un ARNm GFP et la partie capteur correspondant aux séquences d'ARN artificielles suivantes : SEQ ID NO : 24, 25 et 26, ou en variante, dépendant de la revendication 2, 4, 5, le gène rapporteur de capteur étant RFP et le déclencheur étant un ARNm GFP correspondant aux séquences SEQ ID NO : 67, 68, 69 et 70.

9. La molécule d'ARN selon la revendication 7, dépendant des revendications 1, 3, 5 et 6, ledit biomarqueur étant une région du génome viral du SARS-CoV-2 (SEQ ID NO : 38), le déclencheur étant un ARNm de LacZ et la partie de capteur correspondant aux séquences d'ARN artificielles suivantes : SEQ ID NO : 36 et SEQ ID NO : 37 ou en variante, ledit biomarqueur étant des régions du génome viral du SARS-CoV-2 inversé (SEQ ID NO : 46, SEQ ID NO : 47), le déclencheur étant un ARNm GFP et la partie capteur correspondant aux séquences d'ARN artificielles suivantes : SEQ ID NO : 44 et 45, ou en variante, dépendant de la revendication 2, 4, 5, ledit biomarqueur étant des régions du génome viral du SARS-CoV-2 complémentées en sens inverse, le gène rapporteur de capteur étant RFP et le déclencheur étant un ARNm GFP correspondant aux séquences SEQ ID NO : 53 et 54.

10. L'utilisation de la molécule d'ARN selon les revendications 1 à 9 dans un dosage colorimétrique ou fluorométrique lors de la liaison à une cible d'ARN de ladite molécule d'ARN, ladite molécule d'ARN étant contenue avec un extrait cellulaire dans le receveur à utiliser dans un diagnostic in vitro d'une certaine maladie.

11. Un procédé de détection in vitro d'une maladie comprenant les étapes consistant à :
a) l'obtention d'un échantillon isolé de sang ou de salive ou d'écouvillons de fluides corporels provenant d'un individu;
c) extraction et purification de l'ARN;
d) amplification de l'ARN obtenu à l'étape c) par amplification isotherme;
e) la détection de l'ARN à partir de cellules et/ou de micro-organismes provoquant la maladie par l'intermédiaire d'une molécule d'ARN selon l'une quelconque des revendications 1, 3, 5, 6, 7, 8, 9; et
f) identifier un résultat positif de la maladie en confirmant l'expression de l'ARNm déclencheur.

12. Un procédé de détection in vitro d'une maladie comprenant les étapes consistant à :
a) l'obtention d'un échantillon isolé de sang ou de salive ou d'écouvillons de fluides corporels provenant d'un individu;
c) extraction et purification de l'ARN;
d) amplification de l'ARN obtenu à l'étape c) par amplification isotherme;
e) la détection de l'ARN à partir de cellules et/ou de micro-organismes provoquant la maladie par l'intermédiaire d'une molécule d'ARN selon l'une quelconque des revendications 2, 4, 5, 7, 8, 9; et
f) identifier un résultat positif de la maladie en confirmant l'expression de l'ARNm déclencheur.
g) l'identification d'un résultat négatif de la maladie par confirmation de l'expression de l'ARNm du capteur.
h) Null si aucune expression n'est détectée.

13. Procédé selon les revendications 11 ou 12,
1) l'amplification isotherme est choisie parmi les technologies NASBA, TMA et SMART, et/ou
2) la molécule d'ARN selon les revendications 1 à 9 est lyophilisée, conjointement avec un extrait cellulaire, dans un dispositif microfluidique, et/ou
3) l'identification d'un résultat positif dans l'étape f) est confirmée par un dosage colorimétrique ou fluorométrique, le déclencheur étant un ARNm de LacZ ou une GFP.
4) l'identification d'un résultat négatif dans l'étape f) est confirmée par l'intermédiaire d'un dosage fluorimétrique dans lequel le rapporteur de capteur est RFP.

14. Procédé selon l'une quelconque des revendications 11 à 13, le procédé comprenant en outre entre les étapes a) et c ):
b) traiter ledit échantillon ou écouvillon isolé avec au moins un tampon de lysat et/ou une étape de chauffage.

15. Un kit de détection in vitro d'une maladie comprenant :
a) au moins un moyen de réception pour recevoir ledit échantillon ou écouvillon isolé de l'individu;
b) un tampon de lysat pour traiter l'échantillon ou l'écouvillon;
c) capacité de chauffage.
d) au moins la molécule d'ARN selon l'une quelconque des revendications 1 à 9 pour la détection d'une maladie chez l'individu;
e) des instructions d'utilisation du kit.

16. Procédé selon l'une quelconque des revendications 11 à 14, kit, selon la revendication 15, ou utilisation, selon la revendication 10, dans lequel ladite maladie est une infection sanguine provoquée par des bactéries pathogènes ou la COVID-19.
